(19) **Europäisches Patentamt / European Patent Office / Office européen des brevets**

(11) **EP 3 835 313 A1**

(12) **EUROPEAN PATENT APPLICATION**
published in accordance with Art. 153(4) EPC

(43) Date of publication:
**16.06.2021  Bulletin 2021/24**

(51) Int Cl.:
**C07K 14/575** (2006.01)  **C07K 1/12** (2006.01)
**C07K 1/18** (2006.01)  **C07K 1/22** (2006.01)
**C07K 1/34** (2006.01)  **C07K 1/36** (2006.01)
**C12P 21/00** (2006.01)

(21) Application number: **19847866.1**

(22) Date of filing: **25.07.2019**

(86) International application number:
**PCT/KR2019/009265**

(87) International publication number:
**WO 2020/032444 (13.02.2020 Gazette 2020/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority:  **07.08.2018  KR 20180091887**

(71) Applicant: **Huons Co., Ltd.**
**Gyeonggi-do 13486 (KR)**

(72) Inventors:
• **KIM, Yeong-Mok**
**Seoul 04732 (KR)**

• **JANG, Do Soo**
**Yongin-Si, Gyeonggi-do 16943 (KR)**
• **KANG, Seok-Chan**
**Bucheon-Si, Gyeonggi-do 14596 (KR)**
• **KIM, Wanseop**
**Yongin-Si, Gyeonggi-do 16822 (KR)**
• **UM, Key-An**
**Suwon-Si, Gyeonggi-do 16295 (KR)**

(74) Representative: **Dehns**
**St. Bride's House**
**10 Salisbury Square**
**London EC4Y 8JD (GB)**

(54)  **METHOD FOR PREPARING GLY-T?4**

(57)  The present invention relates to a method for preparing glycine-thymosin β4 (Gly-Tβ4). The present invention has an excellent effect in that, according to the present invention, a large amount of high-purity Gly-Tβ4 can be obtained with high productivity through chromatography, enzyme treatment, and filtration of a sample containing GST fusion thymosin β4 (GST-Tβ4).

FIG. 50

EP 3 835 313 A1

**Description**

[Technical Field]

The present invention relates to a method of preparing Gly-thymosin β4 (Gly-Tβ4), and more particularly, to a method of preparing Gly-Tβ4, which includes an expression process for expressing GST fusion recombinant human thymosin β4 (hereinafter, "GST-Tβ4"), which is a precursor of Gly-Tβ4, and a Gly-Tβ4 purification process.

[Background Art]

Large-scale protein purification is an issue that has become more and more important in the biotechnological industry. Generally, proteins are produced by cell culture using a mammalian or bacterial cell line engineered to produce a target protein by inserting a recombinant plasmid containing the gene of the target protein. Since cell lines used herein are living organisms, composite culture media containing saccharides, amino acids and growth factors, which are generally provided from animal serum preparations, will be provided to the cells. It is very difficult to separate a desired protein from the mixture of the compounds provided to the cells and by-products produced from the cells in sufficient purity for human treatment.

However, in the case of biologics such as proteins used as drugs, the removal of impurities as well as a product yield is very important. There may be a difference between process-dependent impurities and product-dependent impurities. The process-dependent impurities contain host cell components such as a protein and a nucleic acid, and are derived from a cell culture (e.g., medium ingredient), or a posttreatment process (e.g., a salt or separated chromatography ligand). The product-dependent impurities are molecular variants of products with different properties. For example, such molecular variants include modified forms produced by deamination, incorrect glycosylation or mismatched disulfide bridges as well as shortened forms such as a precursor and hydrolytic degradation products. The product-dependent variants also include a polymer and an aggregate. The term "contaminant" is used to present any material having a chemical, biochemical or microbiological characteristic, which is not directly involved in a preparation process. The contaminant includes viruses that may undesirably appear in a cell culture.

Impurities and contaminants cause safety issues in the case of biologics. It happens very often in the case of biologics, and for example, it could be serious if a therapeutic protein is administered directly into the bloodstream by injection or infusion. Accordingly, host cell ingredients may cause an allergic response or an immunopathological effect. In addition, impurities may lead to undesirable immunogenicity of the administered protein, which triggers an undesirable immune response of a patient to the therapeutic, for example, to the point of lethal anaphylactic shock. Therefore, there is a need for a suitable purification method that can remove all undesirable materials to an insignificant level.

Meanwhile, in the case of biologics, economical aspects cannot be ignored. Accordingly, the production and purification methods used should not threaten the economic feasibility of the biologics produced. In addition, the measure of time over which a novel purification method is able to be established plays an important role. That is, in addition to a cost effect, process development needs to be coordinated with preclinical and clinical development of the drug. Therefore, for instance, some preclinical trials and all clinical trials can only be initiated when biologics of sufficient purity are available at sufficient amounts.

A method of purifying a protein from cell debris initially depends on the protein's expression site. One protein may be directly secreted from cells to a surrounding proliferation medium, and another protein may be made in cells. In the case of the latter protein, the first step of the purification method includes a cell lysis step that can be performed by various methods including mechanical shearing, osmotic shock or enzymatic treatment. Due to such cell lysis, the entire cell contents are released into a cell suspension, and intracellular fragments that are difficult to remove due to their small size are produced. These intracellular fragments are generally removed by fractional centrifugation or filtration. To a lesser extent than in the above-described case, this problem also appears in proteins directly secreted due to the release of host cell proteins in cells during natural cell death and protein production.

When a purified solution containing a target protein is obtained, separation of the target protein from another protein produced by cells is generally performed by a combination of different chromatography techniques. The key to this chromatography technique is that, as proteins may move down a long column at different speeds, the proteins may be more and more physically separated, or selectively adhered to separation media, thereby being differentially eluted by different solvents. In some cases, when impurities are specifically attached to a column, and the target protein is not attached thereto, that is, present in flow-through, the target protein is separated from the impurities, or when the target protein is specifically attached to the column, the attached target protein may be separated from the impurities through elution. Numerous kinds of the column chromatography method and materials that can be used in this step are known. However, as the number of alternative methods increases, a much larger number of preliminary trials need to be carried out to determine optimal material and method in terms of purification effect, yield, biological activity, time and cost.

In addition, in the establishment and optimization of a purification method, it is clear that the method has to be adjusted individually according to the biochemical and biophysical properties of a specific molecule to be purified (target molecule or target protein) and a biological starting material. The biological starting material from which the target material will be separated is generally formed of a mixture of very complicated materials. To separate and concentrate the target molecule, specific characteristics such as a shape, a size, solubility, a surface charge, surface hydrophobicity and bio-specific affinity to a binding partner are used. For a new target molecule, that is, even for the same target molecule changed only in one of the previous steps (e.g., a change in composition of a fermentation culture medium), the purification process has to be newly adjusted because the best possible result may not be achieved from the above-mentioned aspects under the new conditions.

At the same time, the number of alternative processes that can be theoretically estimated depends on the number of parameters listed above. In column chromatography, for example, in the entire process, a series of chromatography steps, a column material, a pH, a salt content and the properties of various elution buffers used, a protein concentration when charged in the column, and may other aspects need to be optimized. This makes it impossible to develop an optimal column chromatography process within appropriate cost and time ranges on an industrial scale. Meanwhile, economic feasibility and device-related limitations (e.g., the use of as few kinds of buffers as possible, the necessity of minimum amounts or volumes of the buffers and chromatography materials, the necessity of maintaining a product-containing fraction volume as small as possible, and the necessity of minimum process time and wastewater volume) also need to be optimized in each step of the process.

Therefore, the column chromatography purification method of a biomolecule may be established and optimized rapidly and at low cost, and there is still an urgent need for a process of obtaining satisfactory results under large-scale production and purification conditions.

However, Gly-thymosin β4 (Gly-Tβ4) is a peptide represented by a sequence of 44 amino acids, and has glycine (Gly) added to the N-terminus of conventional thymosin β4 (Tβ4). Gly-Tβ4 was found through the known quaternary structure analysis of Tβ4 and the genetic engineering modification of the N-terminus of the protein, and has different activity from original Tβ4. The structure and function of Tβ4 are relatively defined, and Tβ4 is one of the known major thymus factors. It is known that Tβ4 is the first separated polypeptide from thymosin fraction 5 (TF5) extracted from the bovine mammary gland, and has 43 amino acids and a molecular weight of 4963KD without bisulfide bonds and glycosylation (The Journal of Biological Chemistry, Vol 257, No2, pp1000-1006, 1982, Chemical Characterization of Thymosin beta).

Gly-Tβ4 is obtained by expression and purification of Gly-Tβ4 through fermentation in bacterial cells manipulated to express conventional Gly-Tβ4. However, it has been pointed out that a considerable amount of proteins is lost in purification, and Gly-Tβ4 obtained through the above-mentioned process ultimately has low productivity. In addition, when GST-Tβ4 is fermented according to a conventional 30 L culture batch record (hereinafter, "NR culture process") manufactured by Northland Biotech, Beijing, China, disadvantages such as the formation of a medium precipitate and acetate accumulation as well as low productivity have been pointed out. In addition, when purification is carried out according to the purification process of Northland Biotech, due to a low proliferation yield, a high unit cost, and the low purity of the final product, there were many problems for use as a medicine.

[Disclosure]

[Technical Problem]

The present invention is directed to providing a method of stably obtaining a high-purity Gly-Tβ4 on a large scale. More specifically, the present invention provides a method of preparing glycine-thymosin β4 (Gly-Tβ4), which includes the following steps: (S1) loading a sample containing GST fusion thymosin β4 (GST-Tβ4) in a primary anion exchange chromatography column equilibrated with an equilibration buffer and eluting a fraction attached to the column with the elution buffer; (S2) performing affinity chromatography on an eluate; (S3) enzymatically cleaving the eluate by thrombin treatment; (S4) loading the cleaved product in a secondary anion exchange chromatography column equilibrated with an equilibration buffer, and eluting a fraction attached to the column with an elution buffer at a dynamic binding capacity (DBC) of 1.8 mg/mL or less; (S5) loading the eluate in a cation exchange chromatography column equilibrated with an equilibration buffer, washing the column with a wash buffer, and eluting a fraction attached to the column with an elution buffer at a DBC of 5 mg/mL or less; and (S6) filtrating the eluate.

However, technical problems to be solved in the present invention are not limited to the above-described problems, and other problems which are not described herein will be fully understood by those of ordinary skill in the art from the following descriptions.

[Technical Solution]

To solve the above-described problem, the present invention provides a method of preparing glycine-thymosin β4 (Gly-Tβ4).

More specifically, the method of preparing Gly-Tβ4 according to the present invention includes the following steps:

(S1) loading a sample in a primary anion exchange chromatography column equilibrated with an equilibration buffer, washing the column with a wash buffer, and eluting a fraction attached to the column with the elution buffer;
(S2) performing affinity chromatography on an eluate;
(S3) enzymatically cleaving the eluate by thrombin treatment;
(S4) loading the cleaved product in a secondary anion exchange chromatography column equilibrated with an equilibration buffer, washing the column with a washing buffer, and eluting a fraction attached to the column with an elution buffer at a dynamic binding capacity (DBC) of 3 mg/mL or less;
(S5) loading the eluate in a cation exchange chromatography column equilibrated with an equilibration buffer, washing the column with a wash buffer, and eluting a fraction attached to the column with an elution buffer at a DBC of 3 to 8 mg/mL; and
(S6) filtrating the eluate.

In Step (S4), the cleaved product may be loaded in the column at pH 8 to 9 and a conductivity of 4 mS/cm or less.

The fraction attached to the column in the anion exchange chromatography in Step (S4) is eluted with a buffer of pH 7 to 9, which contains 10 to 30 mM Tris-HCl and 30 to 130 mM NaCl, at a flow rate of 100 to 300 cm/hr.

In the cation exchange chromatography in Step (S5), the cleaved product is loaded in the column at pH 4 to 7 and a conductivity of 4 mS/cm or less.

In the cation exchange chromatography in Step (S5), the fraction attached to the column is eluted with a buffer of pH 4 to 5, which contains 10 to 30 mM acetate and 150 to 210 mM NaCl, at a flow rate of 100 to 300 cm/hr.

In Step (S3), the ratio of the target protein to thrombin may be 5 to 0.5:1 (unit:mg).

The reaction temperature in Step (S3) may be 13 to 27 °C.

The purity of the final purified extract according to the method may be 97% or more.

In Step (S1), the sample may be obtained by the following steps:
culturing host cells expressing the target protein; lysing the host cells; and washing and filtering the lysate.

The main culture of the host cells may be fed-batch culture at $OD_{600}$ of 40 to 65 and an induction temperature of 25 to 35°C in the initiation of induction.

Cell productivity according to the method may be 180 g pellet/L or more, and the productivity of the target protein may be 54 mg/g pellet or more.

Hereinafter, the present invention will be described in detail.

The present invention relates to a method of preparing glycine-thymosin β4 (Gly-Tβ4), which includes the following steps:

(S1) loading a sample in a primary anion exchange chromatography column equilibrated with an equilibration buffer, washing the column with a wash buffer, and eluting a fraction attached to the column with an elution buffer;
(S2) performing affinity chromatography on an eluate;
(S3) enzymatically cleaving the eluate by thrombin treatment;
(S4) loading the cleaved product in a secondary anion exchange chromatography column equilibrated with an equilibration buffer, washing the column with a wash buffer, and eluting the fraction attached to the column with an elution buffer at a dynamic binding capacity (DBC) of 1.8 mg/mL or less;
(S5) loading the eluate in a cation exchange chromatography equilibrated with an equilibration buffer, washing the column with a wash buffer, and eluting a fraction attached to the column with an elution buffer at a dynamic binding capacity (DBC) of 5 mg/mL or less; and
(S6) filtering the eluate.

The term "glycine-thymosin β4 (Gly-Tβ4)" used herein is preferably mammalian, and most preferably human Gly-Tβ4 protein or a polypeptide thereof. The amino acid sequence of the human Gly-Tβ4 protein is disclosed in GenBank No. NP_066932.1, and the genetic sequence of the human Gly-Tβ4 protein is disclosed in GenBank No. NM_021109.

The term "sample" used herein refers to a mixture of a target protein and one or more types of contaminants, and the "target protein" includes Gly-Tβ4, a polypeptide thereof or a precursor thereof, and preferably, the precursor of the Gly-Tβ4 refers to GST fusion thymosin β4 (GST-Tβ4). According to one embodiment of the present invention, the sample contains GST fusion thymosin β4 (GST-Tβ4).

In the present invention, at least 4 cycles of chromatography are performed. Preferably, 4 cycles of chromatography are performed. Most preferably, 2 cycles of anion chromatography, one cycle of affinity chromatography and one cycle of cation chromatography are performed. In addition, before or after Step S1), before or after Step S2), before or after Step S3), before or after Step S4), before or after Step S5), or before or after Step S6), any one or more chromatography techniques selected from the group consisting of affinity chromatography, ion exchange chromatography, hydrophobic interaction chromatography and mixed mode chromatography may be further performed.

In the present invention, all types of chromatography are preferably performed according to a binding/elution (B/E) mode. The term "B/E mode" refers to a working method of chromatography in which a target protein binds to a chromatography material by applying a solution containing the target protein to be purified (e.g., sample) to the chromatography material. Accordingly, the target protein is maintained on the chromatography material, whereas a material that does not correspond to the target protein is removed along with a flow-through or supernatant. The target protein is then recovered from the chromatography material by an elution buffer. In the present invention, according to the B/E mode, it is possible to obtain a high-purity target protein with high efficiency.

The term "application" refers to a partial step of a purification method in which a solution containing a target protein is in contact with a chromatography material. This means that (a) a solution is added to a chromatography apparatus containing a chromatography material, or (b) a chromatography material is added to a solution. In (a), the solution enables an interaction between the chromatography material and a material contained in the solution by passing through the apparatus. For example, according to conditions such as pH, conductivity, a salt concentration, a temperature and/or a flow rate, some materials in the solution may bind to the chromatography material, and other materials may be recovered from the chromatography material. Materials remaining in the solution or recovered from the chromatography material may be found in the flow-through. The term "flow-through" refers to a solution obtained after passing through the apparatus, which may be a buffer (or solution) applied to wash a column or cause the elution of a material binding to the chromatography material. The apparatus is a column or cassette. In (b), the chromatography material is, for example, a solid, and allows the interaction between the chromatography material and a material in the solution by being added to a solution containing a material to be purified. After the interaction, the chromatography material may be removed by filtration, and a material binding to the chromatography material is also removed from the solution, whereas a material that does not bind to the chromatography material remains in the solution.

In the chromatography step of the present invention, equilibration, loading, washing and elution processes are included, and in each process, a buffer may be used.

The term "buffer" used herein is a solution resistant to a pH change by the action of an acid-base complex ingredient. For example, various buffers that can be used according to desired pH of the buffer are disclosed in the document [Buffers. A Guide for the Preparation and Use of Buffers in Biological System, Gueffroy, D., Ed. Calbiochem Corporation (1975)]. Generally, a pharmaceutically acceptable buffer is used. In one aspect, the buffer is selected from a phosphate buffer consisting of phosphoric acid and/or a salt thereof, an acetate buffer consisting of acetic acid and a salt thereof, a citrate buffer consisting of citric acid and/or a salt thereof, a morpholine buffer, a 2-(N-morpholino)ethanesulfonic acid buffer, a histidine buffer, a glycine buffer and a tris(hydroxymethyl)aminomethane(tris) buffer. In another aspect, the buffer is selected from a Tris buffer, a Tris-hydrochloric acid buffer, a citrate buffer, and a histidine buffer. The buffer may contain, for example, an inorganic salt such as sodium chloride, sodium sulfate, calcium chloride, calcium sulfate, ammonium chloride or ammonium sulfate.

In the present invention, the term "equilibration buffer" is used before being loaded in a column, and the equilibration buffer may be, for example, any one selected from the group consisting of sodium citrate, sodium acetate, sodium chloride, sodium phosphate, sodium sulfate, calcium chloride, potassium sulfate, potassium phosphate, Tris, Tris-hydrochloric acid (HCl), 2-(N-morpholino)ethanesulfonic acid (MES), 4-(2-hydroxyethyl)-1-piperazineethanesulfonic acid (HEPES) and 3-[(3-cholamidopropyl)-dimethylammonio]-1-propanesulfonate (CHAPS), but the present invention is not limited thereto.

The term "wash buffer" used herein refers to a buffer used to wash or re-equilibrate an ion exchange resin before elution of a target protein. The "washing" of an ion exchange resin means penetrating or passing a suitable (wash) buffer into or by an ion exchange resin. For example, the wash buffer may be any one selected from the group consisting of sodium citrate, sodium acetate, sodium chloride, sodium phosphate, sodium sulfate, potassium chloride, potassium sulfate, potassium phosphate, Tris, Tris-hydrochloric acid (HCl), 2-(N-morpholino)ethanesulfonic acid (MES), 4-(2-hydroxyethyl)-1-piperazineethanesulfonic acid (HEPES) and 3-[(3-cholamidopropyl)-dimethylammonio]-1-propanesulfonate (CHAPS), but the present invention is not limited thereto.

The term "elution" used herein is for elution of a molecule (e.g., the target protein or contaminant) from an ion exchange resin (or column), and the removal of a molecule from an ion exchange resin (or column) by changing the ion concentration of a buffer surrounding the ion exchange resin (or column) so that the buffer competes with the molecules for a charged part of the ion exchange resin. The elution is performed with a sufficient amount of buffer to cause efficient elution of the target protein so that the elution buffer containing the target protein is recovered. The "efficient elution" refers to elution of 75% or more, or 80% or more, for example, 85% or more of the solution containing the target protein loaded in the resin from the resin. In the present invention, the term "elution buffer" is used to elute the target protein from the ion exchange resin (or column). The elution buffer may be, for example, any one or more selected from the group consisting of sodium citrate, sodium acetate, sodium chloride, sodium phosphate, sodium sulfate, calcium chloride, potassium sulfate, potassium phosphate, Tris, Tris-hydrochloric acid (HCl), 2-(N-morpholino)ethanesulfonic acid (MES), 4-(2-hydroxyethyl)-1-piperazineethanesulfonic acid (HEPES) and 3-[(3-cholamidopropyl)-dimethylammonio]-1-propanesulfonate (CHAPS), but the present invention is not limited thereto. The conductivity and/or pH of the elution buffer are/is the conductivity and/or pH at which the target protein is eluted from a resin (column). In the present invention, the conductivity and/or pH is a degree of elution of virtually all contaminants and target proteins using a resin.

The term "conductivity" used herein refers to an ability of an aqueous solution allowing current flow between two electrodes. In the solution, current flows by ion transport. Accordingly, when an ion amount present in the aqueous solution is increased, the solution may have a higher conductivity. The measurement unit of conductivity is mmhos (mS/cm), and the conductivity may be measured using a commercially available conductivity meter, for example, Orion. The conductivity of the solution may be changed by changing the ion concentration of the solution. For example, to obtain a desired conductivity, the concentration of a buffer material in the solution and/or a salt (e.g., sodium citrate, sodium acetate, sodium chloride, sodium phosphate, sodium sulfate, calcium chloride, potassium sulfate, potassium phosphate, Tris, Tris-hydrochloric acid (HCl), 2-(N-morpholino)ethanesulfonic acid (MES), 4-(2-hydroxyethyl)-1-piperazineethanesulfonic acid (HEPES) and 3-[(3-cholamidopropyl)-dimethylammonio]-1-propanesulfonate (CHAPS)) may be changed. Preferably, a desired conductivity may be obtained by changing salt concentrations of various types of buffers.

The term "dynamic binding capacity (DBC)" used herein refers to the capacity (Q) of a target molecule binding to a certain amount of resin under conditions for chromatography. Since the binding ability between materials causes changes in equilibrium and dissociation constants (K) according to a temperature, pH or a flow rate, the binding ability of a target molecule in a resin should be known to calculate a required amount of resin or a load amount of a specimen per column. DBC may be used as reference data to determine how much material to be separated or purified. DBC measures the point at which elution starts after all target molecules are bound to a column, and generally, the time point at which the concentration of 10% of the loaded target molecules is reached is set as a starting point ($Q_{B,\ 10\%}$: Binding Capacity at 10% Breakthrough point). According to the characteristics of the target molecule, a method for an identity test and measurement may be, for example, an analysis method such as ELISA, SEC or UV absorbance, and after setting the 10% breakthrough point, the total volume from the moment of loading to the corresponding starting point is calculated to calculate the total amount and load amount ($V_{10\%} * C_0$) of the target material, and then divided by a resin volume (Vc): $Q_{B,\ 10\%} = V_{10\%} * C_0/ Vc$.

The sample of the target protein obtained according to the method of the present invention may be subjected to an additional purification step if necessary.

The "purification" used herein refers to an increase in purity of the target protein by completely or partially removing one or more types of contaminants from the sample in which the target protein is mixed with one or more types of contaminants.

According to the present invention, the purity of the final purified extract is 97% or more.

Hereinafter, the present invention will be described by step. However, in order to avoid excessive repetition, overlapping description in each step is provided only once in the first mentioned part and omitted.

The method includes (S1) loading a sample containing GST fusion thymosin β4 (GST-Tβ4) in a primary anion exchange chromatography column equilibrated with an equilibration buffer, washing the column with a wash buffer, and eluting a fraction attached to the column with the elution buffer.

In the present invention, an anion exchange resin in the anion exchange chromatography step may be one or more positively-charged ligands attached to a solid phase, which are substituted with, for example, diethylamino ethyl (DEAE) or quaternary ammonium groups, but the present invention is not limited thereto. Preferably, any one of anion exchange resins having a group having a strongly basic quaternary ammonium group and a weakly basic DEAE group may be selected and used. For example, as a strongly basic anion exchange resin, Q Sepharose Fast Flow, Q Sepharose High Performance, Resource Q, Source 15Q, Source 30Q, Mono Q, Mini Q, Capto Q, Capto Q ImpRes, Q HyperCel, Q Cermic HyperD F, Nuvia Q, UNOsphere Q, Macro-Prep High Q, Macro-Prep 25 Q, Fractogel EMD TMAE(S), Fractogel EMD TMAE Hicap (M), Fractogel EMD TMAE (M), Eshmono Q, Toyopearl QAE-550C, Toyopearl SuperQ-650C, Toyopearl GigaCap Q-650M, Toyopearl Q-600C AR, Toyopearl SuperQ-650M, Toyopearl SuperQ-650S, TSKgel SuperQ-5PW (30), TSKgel SuperQ-5PW (20), or TSKgel SuperQ-5PW may be used, but the present invention is not limited thereto, and an anion exchange resin known in the art may be used. In the present invention, as a primary anion exchange resin, Fractogel EMD TMAE (M) is used, but the present invention is not limited thereto.

A suitable volume of a resin used in the anion exchange chromatography step is reflected by column dimensions, that is, the diameter of the column and the height of the resin, and for example, varies according to an amount of the target protein in the applied solution and binding performance of the used resin. Before the anion exchange chromatography, the anion exchange resin is preferably equilibrated with a buffer to allow the resin to bind with a counter ion thereof.

According to an embodiment of the present invention, in the anion exchange chromatography in step (S1), the sample may be loaded in the column at pH 8 to 9 and a conductivity of 4 mS/cm or less. More preferably, the sample may be loaded in the column at pH 8 and a conductivity of 2 mS/cm or less.

In the present invention, the anion exchange chromatography in step (S1) is performed using a buffer having pH 8 to 9.

According to an embodiment of the present invention, the column for the anion exchange chromatography in step (S1) of the present invention may be equilibrated with 10 to 30 mM and preferably, 20 mM Tris-HCl to be pH 7 to 9, and preferably, pH 8.

In addition, according to an embodiment of the present invention, the ion exchange resin of the anion exchange chromatography in step (S1) of the present invention is washed with a buffer having pH 7 to 9, and preferably, pH 8, which contains 10 to 30 mM and preferably, 20 mM Tris-HCl.

According to an embodiment of the present invention, a fraction attached to the column in the anion exchange chromatography in step (S1) of the present invention is eluted with a buffer of pH 7 to 9, and preferably, pH 8, which contains 10 to 30 mM and preferably, 20 mM Tris-HCl and 200 to 400 mM and preferably, 300 mM NaCl, at a flow rate of 100 to 300 cm/hr, and preferably, 200 cm/hr.

According to an embodiment of the present invention, in the anion exchange chromatography of step (S1), a DBC is 5 to 9, and preferably, 7.5 mg GST-Tβ4/mL of resin.

The method includes (S2) performing affinity chromatography on an eluate.

The term "affinity chromatography" used herein refers to a protein separation technique in which a target protein specifically binds to a ligand specific therefor. The ligand is generally called a bio-specific ligand, and specifically interacts with the target protein (e.g., enzyme-substrate, enzyme-inhibitor, or antigen-antibody). In some embodiments, bio-specific ligands are attached to a chromatography resin by covalent bonds, and as a solution contacts the chromatography resin, the ligands have access to the target protein in the solution. The target protein generally possesses specific binding affinity to the bio-specific ligand during chromatography, but other solutes and/or proteins in the mixture do not clearly or specifically bind to the ligand. Binding of the target protein to an immobilized ligand allows a contaminant protein or protein impurities to pass through the chromatography resin, but holds the target protein specifically binding to the immobilized ligand on a solid-phase material. The specifically-binding target protein is then removed in an active form from the immobilized ligand under a suitable condition (e.g., low pH, high pH, a high salt, or a competing ligand), and passes through a chromatography column with an elution buffer, without contaminant proteins or protein impurities that are previously allowed to pass through the column.

The affinity resin in the affinity chromatography step may be one or more types of ligands attached to a solid phase, for example, Blue Trisacryl M, Protein A Ceramic HyperD, MEP HyperCel, Lysine HyperD, Heparin HyperD M, HA Ultrogel, or Glutathione Sepharose 4 Fast Flow, but the present invention is not limited thereto, and may also be an affinity resin known in the art. In the present invention, as the affinity resin, Glutathione Sepharose 4 Fast Flow was used, but the present invention is not limited thereto.

According to an embodiment of the present invention, the column for the affinity chromatography in step (S2) of the present invention is equilibrated with 10 to 30 mM and preferably 20 mM Tris-HCl and 100 to 200 mM and preferably 150 mM NaCl to have a pH of 7 to 9 and preferably pH 8, the resin of the affinity chromatography in step (S2) is washed with a buffer containing 20 mM Tris-HCl, and 100 to 200 mM and preferably 150 mM NaCl and having pH 7 to 9 and preferably pH 8, and a fraction attached to the column in the affinity chromatography is eluted with a buffer containing 40 to 60 mM and preferably 50 mM Tris-HCl and 5 to 15 mM and preferably 10 mM glutathione and having pH 7 to 9 and preferably pH 8 at a flow rate of 53 to 73 cm/hr and preferably 63 cm/hr. As an acidic buffer, a buffer containing 90 to 110 mM and preferably 100 mM sodium acetate, 400 to 600 mM and preferably 500 mM NaCl, and having pH 4 to 6 and preferably pH 4.5 may be used, and as a basic buffer, a buffer containing 50 to 150 mM and preferably 100 mM Tris-HCl and 400 to 600 mM and preferably 500 mM NaCl and having pH 7 to 9 and preferably pH 8 may be used, but the present invention is not limited thereto.

According to an embodiment of the present invention, the DBC of Glutathione Sepharose 4 Fast Flow with respect to GST-Tβ4 in the affinity chromatography of step (S2) is 9.3 to 9.5 mg GST-Tβ4/mL of resin.

The method includes (S3) enzymatically cleaving the eluate by thrombin treatment.

In this step, the eluate obtained through the affinity chromatography in step (S2) is treated with an enzyme (particularly, thrombin). Through this step, GST fusion thymosin β4 (GST-Tβ4), which is a precursor of Gly-Tβ4 contained in the eluate obtained through affinity chromatography in step (S2), becomes Gly-Tβ4 through enzymatic cleavage. Accordingly, the target protein to be purified in the chromatography before step (S3) is preferably GST-Tβ4, and the target protein to be purified in chromatography after step (S3) is preferably Gly-Tβ4.

Compared to an enzyme (e.g., thrombin), a volume ratio of the target protein (e.g., GST-Tβ4) is, but not limited to, preferably, 0.5 to 5:1 (unit:mg), more preferably 3 to 1:1 (unit:mg), and most preferably 2:1 (unit:mg). In the range of 0.5 to 5:1 (unit:mg), an enzyme reaction rate is particularly high. The time for enzyme (particularly, thrombin) treatment is, but not limited to, preferably 16 to 20 (h). A temperature for enzyme (particularly, thrombin) treatment is, but not limited to, preferably 13 to 27°C, and more preferably 20 to 24 °C. The above-described enzyme volume, and enzyme treatment time and temperature may be appropriately changed according to a condition by one of ordinary skill in the art, but within the preferable ranges, they exhibit most excellent effects.

The method includes (S4) loading the cleaved product in a secondary anion exchange chromatography column equilibrated with an equilibration buffer, and eluting a fraction attached to the column with an elution buffer at a dynamic binding capacity (DBC) of 3 mg/mL or less.

According to an embodiment of the present invention, as the secondary anion exchange resin, Fractogel EMD TMAE (M) was used, but the present invention is not limited thereto.

In the present invention, the anion exchange chromatography in step (S4) is performed with a buffer having pH 8 to 9.

According to an embodiment of the present invention, in the anion exchange chromatography in step (S4), the cleaved product in this step may be loaded in the column at pH 8 to 9 and a conductivity of 4 mS/cm or less. More preferably, the cleaved product is loaded in the column at pH 8 and a conductivity of 2 mS/cm or less.

According to an embodiment of the present invention, the column for the anion exchange chromatography in step (S4) of the present invention is equilibrated using an equilibration buffer containing 10 to 30 mM and preferably, 20 mM Tris-HCl to have pH of 7 to 9, and preferably pH 8.

In addition, according to an embodiment of the present invention, the ion exchange resin in the anion exchange chromatography in step (S4) of the present invention is washed with a buffer containing 10 to 30 mM and preferably, 20 mM Tris-HCl and having pH 7 to 9, and preferably, pH 8.

According to an embodiment of the present invention, a fraction attached to the column in the anion exchange chromatography in step (S4) of the present invention is eluted with a solution containing 10 to 30 mM and preferably 20 mM Tris-HCl and 30 to 130 mM and preferably 80 mM NaCl and having pH 7 to 9, and preferably pH 8 at a flow rate of 100 to 300 cm/hr, and preferably 200 cm/hr.

According to an embodiment of the present invention, a DBC in the anion exchange chromatography in step (S4) is 3 mg GST-Tβ4/mL of resin or less, and preferably 1.8 mg GST-Tβ4/mL of resin. Here, when the amount of the column of the present invention is multiplied by the DBC, the maximum load amount may be obtained, and for example, when a column volume (resin volume) is 24 mL and a DBC is 1.8 mg Gly-Tβ4/mL of resin, the maximum load amount of Gly-Tβ4 is 45.2 mg.

The method includes (S5) loading the eluate in a cation exchange chromatography column equilibrated with an equilibration buffer, washing the column with a wash buffer, and eluting a fraction attached to the column with an elution buffer at a DBC of 3 to 8 mg/mL or less.

The term "cation exchange chromatography" used herein depends on the charge-charge interaction between the target protein and a resin. In cation exchange chromatography, molecules to be bound are positively charged, and an immobilized ligand is negatively charged. Conventionally used cation exchange resins are an S-resin (sulfonate), an SP resin (sulfopropyl), an SE resin (sulfoethyl), and a CM resin (carboxymethyl). However, generally, the cation exchange chromatography step may be performed with any commercially available cation exchange resin. Typical commercially available products include, for example, Macro-Prep High S, Macro-Prep CM, Unosphere Rapid S, Unosphere Rapid S40, Nuvia S, and Nuvia HR-S (Bio-Rad, California, USA), Toyopearl CM, Toyopearl SP, and Toyopearl GigaCap S (Tosoh Bioscience, Germany), Millipore ProRes S, Fractogel EMD COO-, Fractogel EMD SO3-(Merck KGaA, Germany), Biosepra CM ceramic HyperD, Biosepra S ceramic HyperD, S HyperCel (Pall Corporation, New York, USA), Poros HS, Poros XS (Applied Biosystems, Germany), YMC BioPro 30S, YMC BioPro 70S(YMC Europe) CM-Sepharose FF, SP-Sepharose FF, S-Sepharose FF, SP-Sepharose HP, SP-Sepharose XL, SP-Sepharose Big Beads, CM-Sephadex, Capto S, Capto SP ImpRes, and Source S (all, GE Healthcare, Germany), but the present invention is not limited thereto. The "cation exchange resin" includes chromatography resins having a mixed function of an ion exchange function and a hydrophobic interaction function (e.g., Capto adhere, Capto MMC, MEP HyperCell, and Eshmuno HCX), and chromatography resins having a mixed function of an anion exchange function and a cation exchange function (e.g., hydroxyapatite and ceramic hydroxyapatite). The preferable cation exchange resin of the present invention is a strong cation exchange group using a sulfonate or sulfopropyl ligand. Most preferably, the preferable cation exchange resin is a sulfonate or sulfopropyl ligand binding to a rigid matrix, for example, a highly-crosslinked agarose such as Nuvia HR-S, or poly(styrenevinylbenzene) such as Poros 50 HS. According to an exemplary embodiment of the present invention, as a cation exchange resin, a sulfonate group and Nuvia HR-S such as a strong cation exchange group based on a highly-crosslinked agarose matrix were used, but the present invention is not limited thereto.

In the present invention, the cation exchange chromatography in step (S5) is performed using a buffer having pH 4 to 7.

According to an embodiment of the present invention, in the cation exchange chromatography of step (S5), the cleaved product may be loaded in the column at pH 4 to 7 and a conductivity of 4 mS/cm or less. More preferably, the cleaved product may be loaded in the column at pH 4.5 and a conductivity of 2 mS/cm or less.

According to an embodiment of the present invention, the column for the cation exchange chromatography of step (S5) of the present invention is equilibrated with 10 to 30 mM and preferably 20 mM acetate to have pH 4 to 5, and preferably pH 4.5.

In addition, according to an embodiment of the present invention, the ion exchange resin in the cation exchange chromatography of step (S5) of the present invention is washed with a buffer containing 10 to 30 mM and preferably 20 mM acetate and 50 to 150 mM and preferably 100 mM NaCl and having pH 4 to 5, and preferably, pH 4.5.

According to an embodiment of the present invention, a fraction attached to the column in the cation exchange chromatography of step (S5) of the present invention is eluted with a buffer containing 10 to 30mM, and preferably, 20 mM acetate and 150 to 210 mM and preferably 180 mM NaCl and having pH 4 to 5, and preferably pH 4.5 at a flow rate of 100 to 300 cm/hr, and preferably 200 cm/hr.

In the present invention, a DBC in the cation exchange chromatography of step (S5) is 8 mg or less Gly-Tβ4/mL of resin, 3 to 8 mg Gly-Tβ4/mL of resin, and preferably 5 mg Gly-Tβ4/mL of resin. Here, when the amount of the column of the present invention is multiplied by the DBC, the maximum load amount may be obtained, and for example, when a column volume (resin volume) is 12.6 mL and a DBC is 5 mg Gly-Tβ4/mL of resin, the maximum load amount of Gly-Tβ4 is 63 mg.

The method includes (S6) filtrating the eluate.

In the present invention, the filtration in step (S6) may be performed according to various methods known in the art. Preferably, the filtration may use a nanofiltration or ultrafiltration/diafiltration (UF/DF) system, and most preferably, ultrafiltration/diafiltration (UF/DF). The ultrafiltration/diafiltration (UF/DF) is a method of removing only some of solutes from a liquid containing a solvent and two or more types of solutes with different molecular sizes, and is effective in purification of a polymer material and has an advantage of reducing time and costs, compared to common dialysis. The ultrafiltration/diafiltration (UF/DF) is performed at an osmotic pressure of 330 mOsmol/kg or less, and then is characterized by adjusting a pH to 8.

In this step, the eluate may be dialyzed and concentrated.

In addition, in the present invention, the sample may be obtained by the following steps:
culturing host cells expressing a target protein; lysing the host cells; and washing and filtering the lysate.

The target protein contains Gly-Tβ4, a polypeptide thereof, and a precursor thereof, and the precursor of Gly-Tβ4 includes GST fusion thymosin β4.

The host cells used herein may be derived from various species known in the art, and according to an embodiment of the present invention, *E. coli* may be used, and the present invention is not limited thereto.

The term "culture" used herein includes seed culture for survival or proliferation of host cells and main culture for fermentation to express the target protein in host cells.

The host cells used in the present invention may be cultured in various media. Commercially available media, for example, Ham's F10 (Sigma), an LB (Lysogeny broth) medium, a minimal essential medium (MEM; Sigma), RPMI-1640 (Sigma) and a Dulbecco's Modified Eagle's Medium (DMEM, Sigma) are suitable for the culture of host cells. In addition, any medium disclosed in the document by Ham et al. [Meth. Enz. 58:44 (1979)], the document by Barns et al. [Anal. Biochem. 102:255 (1980)], U.S. Patent No. 4,767,704, 4,657,866, 4,927,762, 4,560,655 or 5,122,469, International Patent Application Publication No. 90/03430 or 87/00195, or U. S. Patent No. 30,985 may be used as a culture medium for host cells. As needed, the medium may be supplemented with hormones and/or other growth factors (e.g., insulin, transferrin or an epithelial growth factor), salts (e.g., sodium chloride, calcium, magnesium and a phosphate), buffers (e.g., HEPES), nucleotides (e.g., adenosine and thymidine), antibiotics (e.g., Gentamycin™ drug), trace elements (generally, defined as an inorganic compound present in a micromole range at the final concentration), and glucose or an equivalent energy source. Any other necessary supplements may be included in the medium at suitable concentrations known to those of ordinary skill in the art. Culture conditions for the host cells selected for expression, for example, a temperature and a pH are the same as used conventionally, and apparent to those of ordinary skill in the art.

The medium for the seed culture and the main culture of the present invention may be suitably selected from the above-described media and necessary supplements by those of ordinary skill in the art. Preferably, the seed culture may be performed in an LB medium at 35 to 40 °C, and preferably, 37 °C for 3 to 4 hours, and when $OD_{600}$ reaches 2 to 6, the culture may be terminated. In addition, the main culture may be performed with Feed A supplementing glucose and $MgSO_4.7H_2O$ and Feed B supplementing tryptone and an yeast extract. In addition, the main culture is performed at 35 to 40 °C, and preferably, $37\pm0.5$ °C for 17 hours at pH 7 to 8. The main culture is preferably initiated with Feed A, and when induction is initiated at $OD_{600}$ of 40 to 65, and preferably, 49 to 59, Feed B is applied with IPTG addition and temperature variations (25 to 35 °C, and preferably 30 °C), and when the induction is terminated, the supply of a Feed B medium may be interrupted. This may maximize cell growth and the productivity of the target protein.

In the present invention, the culture of the present invention may be followed by a fed-batch fermentation process. The fed-batch refers to a culture method in which a culture broth is not extracted from a fermenter until the end of the culture while a nutrient medium is slowly added to the fermenter at the same time as the progression of the culture. In the present invention, according to a fed-batch fermentation process, high productivity is exhibited, and there is an excellent effect for resolving a disadvantage of the formation of a medium precipitate and an acetate. According to the culture method of the present invention, cell productivity may be 180 g pellet/L or more, and preferably, 185 g pellet/L or more, and the productivity of the target protein (GST-T4) may be 54 mg/g pellet or more, and preferably, 56 mg/g pellet or more.

When a recombinant technique is preferably used to express the target protein, host cells may be transformed with an expression or cloning vector, and cultured in the above-described conventional medium modified to be suitable for inducing a promoter, selecting a transformant and amplifying genes encoding a desired sequence.

In the present invention, when a recombinant technique is used to express the target protein in the host cells, the target protein may be produced in cells, in the intermembrane space, or directly secreted into the medium. When the target protein is produced in cells, a step of lysing the host cells, and washing and filtering the lysate is included, as a first step, lysed microparticles (e.g., produced by homogenization) of the host or lysed cells are removed, for example, by centrifugation or ultrafiltration (UF). When the target protein is secreted into the medium, a supernatant of an expression system is first concentrated generally using a commercially available protein concentration filter, for example, an Amicon or Millipore Pellicon ultrafiltration (UF) device. According to an embodiment of the present invention, a step of lysing the host cells and a step of washing and filtering the lysate are preferably included.

[Advantageous Effects]

According to the present invention, high-purity Gly-Tβ4 can be stably prepared on a large scale, and thus Gly-Tβ4 having an excellent industrial utility value can be economically obtained with high efficiency.

[Description of Drawings]

FIG. 1 shows a 5 L verification run feeding strategy.
FIG. 2 shows a 50 L verification run feeding strategy.
FIG. 3 shows cell growth and glucose & acetate concentrations (verification run 5 L culture).
FIG. 4 shows a result of SDS-PAGE assay (5 L verification run).
FIG. 5 shows cell growth and glucose & acetate concentrations (verification run 50 L culture).
FIG. 6 shows a result of SDS-PAGE (verification run 50 L culture).
FIG. 7 shows flask culture cell growth (flask 1: w/YE, flask 2: w/o YE).
FIG. 8 shows cell growth and glucose & acetate concentrations (5 L fed-batch growth test).
FIG. 9 (FIGS. 9A to 9M) shows cell growth (DoE 13 runs) and glucose & acetate residual amounts in a culture broth.
FIG. 10 shows SDS-PAGE & protein quantification (Run data: DoE 12, 7, 8, 5, 10, 2, 13).
FIG. 11 shows SDS-PAGE & protein quantification (Run data: DoE 11, 3, 9, 1, 6, 4).
FIG. 12 shows a result of the goodness of fit for DoE variance analysis (residual plot).
FIG. 13 shows the optimization of Minitab response conditions.
FIG. 14 shows the contour plot for responses (GST-Tβ4 and cell productivity).
FIG. 15 shows cell growth and glucose & acetate concentrations (confirming DoE optimal condition reproducibility).
FIG. 16 shows SDS-PAGE & protein quantification analysis 1 (optimal condition reproducibility batch).
FIG. 17 shows SDS-PAGE & protein quantification analysis 2 (optimal condition reproducibility batch).
FIG. 18 shows cell growth and glucose & acetate concentrations (50 L optimal condition scale-up process).
FIG. 19 shows a 50L verification run downstream process flow chart.
FIG. 20 shows the SDS-PAGE analysis for a cell lysate by cell lysis buffer conditions.
FIG. 21 shows the SDS-PAGE analysis for an ammonium sulfate study in a cell lysate.

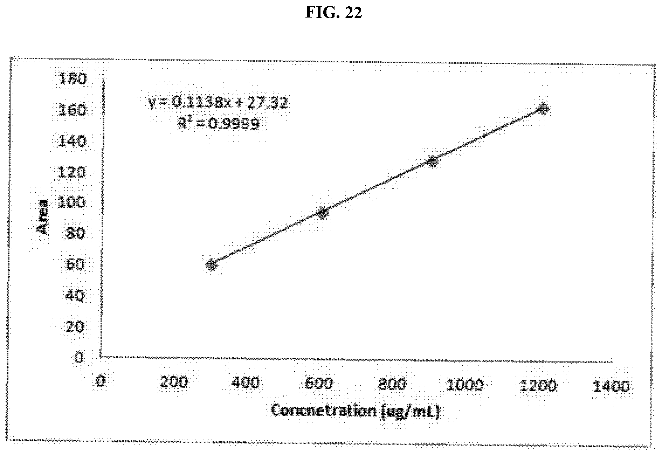

FIG. 22 shows a regression curve for GST-Tβ4 using an affinity column.
FIG. 23 shows an AEXI process chromatogram for a linear flow rate (61 cm/hr).
FIG. 24 shows an AEXI process chromatogram for a linear flow rate (200 cm/hr).
FIG. 25 (FIGS. 25A to 25B) show the SDS-PAGE analysis for a linear flow rate study (a: 61 cm/hr, b: 200 cm/hr).
FIG. 26 shows an AEXI process chromatogram for a 500 mM NaCl elution condition.
FIG. 27 shows an AEXI process chromatogram for a 200 mM NaCl elution condition.
FIG. 28 (FIGS. 28A and 28B) shows the SDS-PAGE analysis for an elution buffer study (a: 500 mM NaCl, b: 200 mM NaCl).
FIG. 29 shows an AEXI process chromatogram for a loading sample condition (Control).
FIG. 30 shows an AEXI process chromatogram for a loading sample condition (50 mM NaCl).
FIG. 31 shows an AEXI process chromatogram for a loading sample condition (80 mM NaCl).
FIG. 32 (FIG. 32A and 32B) shows the SDS-PAGE analysis for a loading sample & an EQ buffer study (a: 80 mM NaCl, b: 50 mM NaCl).
FIG. 33 (FIG. 33A, 33B and 33C) shows the SDS-PAGE analysis for an AEX DBC test (a: 10 mg GST-Tβ4/mL of resin, b: 8 mg GST-Tβ4/mL of resin, and c: 75 mg GST-Tβ4/mL of resin).
FIG. 34 (FIG. 34A and FIG. 34B) shows a hold time study with an AEXI eluted pool in two different conditions (a: storage temperature: 2 to 8 °C, b: storage temperature: 18 to 22 °C).

FIG. 35 shows an affinity chromatogram for a linear flow rate (90 cm/hr).

FIG. 36 shows an affinity chromatogram for a linear flow rate (180 cm/hr).

FIG. 37 (FIGS. 37A and 37B) show AEXI chromatograms for a scale up test from XK16(a) to XK50(b), respectively.

FIG. 38 (FIGS. 38A and 38B) shows affinity chromatograms for a feasibility test in LRC10 scale (a) and XK50 scale (b), respectively.

FIG. 39 (FIGS. 39A and 39B) shows a hold time study with an affinity eluted pool in two different conditions (A: storage temperature: 2 to 8 °C, B: storage temperature: 18 to 22 °C).

FIG. 40 shows an SEC-HPLC chromatogram of reaction mixtures after digestion at three different temperatures.

FIG. 41 shows a CEX process chromatogram in a pH 4.5 condition.

FIG. 42 shows an RPC-HPLC chromatogram for a CEX process in a pH 4.5 condition.

FIG. 43 (FIGS. 43A and 43B) shows a set of HIC chromatograms for butyl (a) and Phenyl (b).

FIG. 44 shows a Capto MMC process chromatogram.

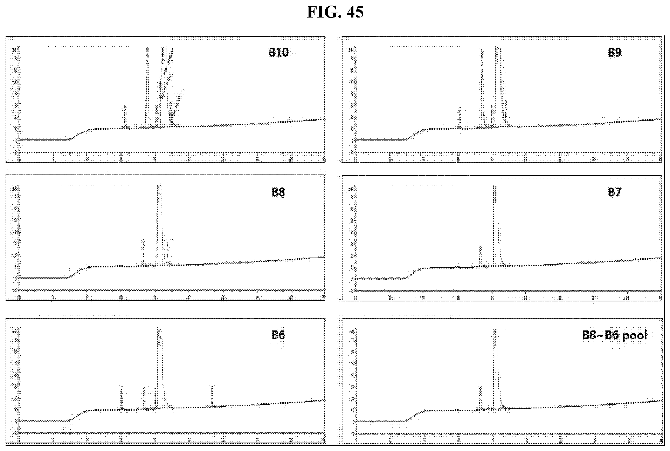

FIG. 45 shows the RP-HPLC analysis for MMC process eluted fractions.

FIG. 46 shows an MMC chromatogram by step elution after washing.

FIG. 47 shows an MMC chromatogram for a 100 mM NaCl washing step.

FIG. 48 shows an MMC chromatogram for a 120 mM NaCl washing step.

FIG. 49 shows an MMC chromatogram for a 140 mM NaCl washing step.

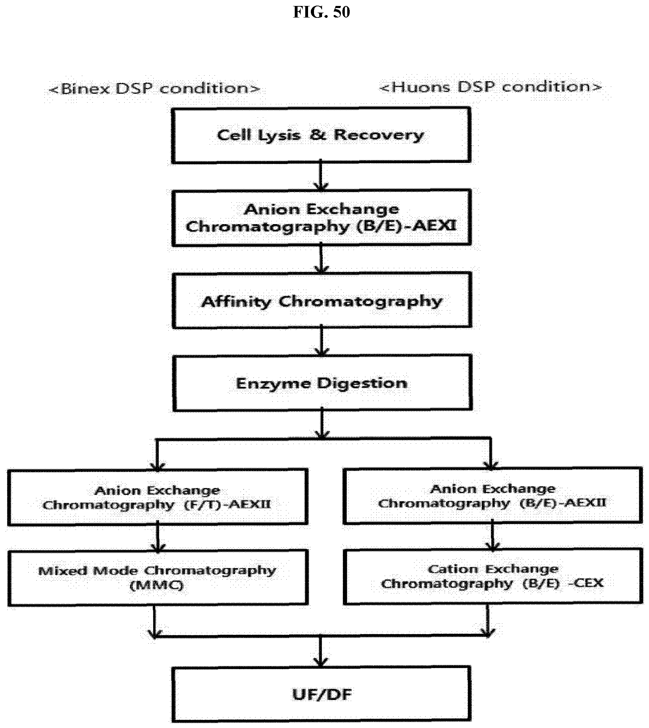

FIG. 50 shows a DSP flow chart.

FIG. 51 shows the SEC-HPLC analysis for an AEXII DBC elution sample.

FIG. 52 shows an AEXII process chromatogram for a load amount (1 mg Gly-Tβ4 per resin mL).

FIG. 53 shows an AEXII process chromatogram for a load amount (15 mg Gly-Tβ4 per resin mL).

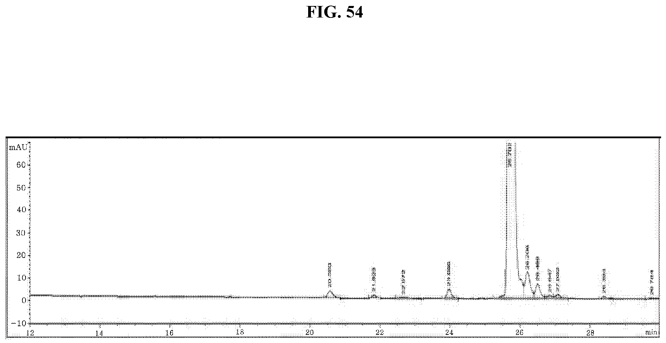

FIG. 54 shows the RP-HPLC analysis for an AEXII process elution sample.

FIG. 55 shows an AEXII process chromatogram with increased conductivity of a loading sample.

FIG. 56 shows an RP-HPLC chromatogram for an AEXII hold time study.

FIG. 57 shows a CEX process chromatogram (at Huons).

FIG. 58 shows a CEX process chromatogram (at BINEX).

FIG. 59 shows a CEX process chromatogram for a prepack column (height: 10 cm).

FIG. 60 shows a CEX process chromatogram for a XK16 column (height: 20 cm).

FIG. 61 shows a CEX process chromatogram with a column having a bed height of 10 cm.

FIG. 62 shows a RP-HPLC chromatogram for a CEX hold time study.

FIG. 63 shows an AEXI process chromatogram for a 5L scale process.

FIG. 64 shows an affinity process chromatogram for a 5L scale process.

FIG. 65 shows an AEXII process chromatogram for a 5L scale process.

FIG. 66 shows a CEX process chromatogram for a 5L scale process.

FIG. 67 (FIGS. 67A, 67B and 67C) shows AEXI process chromatograms (a: Cycle 1, b: Cycle 2, c: Cycle 3).

FIG. 68 (FIGS. 68A, 68B and 68C) shows affinity process chromatograms (a: Cycle 1, b: Cycle 2, c: Cycle 3).

FIG. 69 (FIGS. 69A and 69B) shows AEXII process chromatograms (a: Cycle 1, b: Cycle 2).

FIG. 70 (FIGS. 70A and 70B) show CEX process Chromatograms (a: Cycle 1, b: Cycle 2).

[Modes of the Invention]

Hereinafter, to help in understanding the present invention, exemplary examples will be suggested. However, the following examples are merely provided to more easily understand the present invention, and not to limit the present invention.

[1. Fermentation process]

Example (1) Strain information

A GST-fusion human thymosinβ4 (GST-Tβ4)-producing recombinant strain was prepared using an *Escherichia coli* BL21 strain, and an RCB was prepared to be used in the present invention (Table 1).

[Table 1]

| Items | Detail |
|---|---|
| Host | *Escherichia coli* BL21 |
| Product | GST-fusion Human Thymosinp4 |

(continued)

| Items | Detail |
|---|---|
| Promoter | Tac promoter |
| Inducer | IPTG or Lactose |
| Expression | Soluble expression in cytoplasm |

## Example (2) Confirmation of reproducibility of China Northland process (2-1) 5 L scale culture

Referring to the 30 L culture batch record (hereinafter, "NR process") of Northland Biotech, Beijing, China, conditions for a 5 L scale-down culture process were deduced (Tables 2, 3 and 4), and 1st to 4th cultures were carried out.

[Table 2]

| Process | Item | | 5L scale |
|---|---|---|---|
| N/A | Strain | | HU024 RCB |
| Seed culture (Baffled Flask) | Inoculation | | 1 mL (RCB) |
| | Medium | | LB media |
| | Culture volume | | 50 mL |
| | Flask volume | | 500 mL |
| | Kanamycin | | 50 $\mu$g/mL |
| | Shaking speed | | 200-230 rpm |
| | Temperature | | 37 °C |
| | Culture time | | 3~4 h |
| | Culture end | | $OD_{600}$ = 2~6 |
| Main culture (5L fermenter) | Incubation volume | | 50 mL |
| | Culture start volume | | 3L |
| | Incubator | | 5L fermenter |
| | Process type | | Fed-batch |
| | Kanamycin | | Not added |
| | Culture temperature | | 37 °C |
| | Incubation | Start condition | $OD_{600}$ 80±10 |
| | | Temperature | 32 °C |
| | | Inducer | IPTG 1 mM |
| | | Added amount | 3 mL (1M stock) |
| | | Time | 4h |
| | Agitation speed | | 300~1,000 rpm |
| | DO control | | 30% |
| | pH | | 7.0 ±0.2 |
| | Aeration | | 1 vvm (3 L/min) |
| | Culture time | | ≥ 17h |

Culture conditions for 5 L verification run

[Table 3]

| Preparation Item | Composition | Preparation criterion (L$^{-1}$) | |
|---|---|---|---|
| Seed culture | LB powder | 25 | g |
| Initial medium (3 L) | K$_2$HPO$_4$ | 13.3 | g |
| | (NH$_4$)$_2$HPO$_4$ | 4.0 | g |
| | Citric acid | 1.7 | g |
| | Soy peptone | 1.0 | g |
| | Yeast extract (Biospringer 0202) | 0.5 | g |
| | Yeast extract (Biospringer 0251) | 0.5 | g |
| | Sodium hydroxide | 2.9 | g |
| | Antifoam 204 | 0.2 | mL |
| | MgSO$_4$·7H$_2$O | 1.1 | g |
| | Glucose | 5.0 | g |
| | Microelement solution | 5.0 | mL |
| Feed A (1 L) | Glucose | 500.0 | g |
| | MgSO$_4$·7H$_2$O | 1.8 | g |
| Feed B (0.5 L) | Tryptone | 33.3 | g |
| | Yeast extract (Biospringer 0202) | 50.0 | g |
| | Yeast extract (Biospringer 0251) | 50.0 | g |

Medium composition for 5 L verification run

[Table 4]

| Composition | Preparation criterion (L$^{-1}$) | |
|---|---|---|
| Ferric chloride | 16.0 | g |
| Cobalt dichloride | 2.0 | g |
| Copper chloride | 1.0 | g |
| Zinc chloride | 2.0 | g |
| Sodium molybdate | 2.0 | g |
| Boric acid | 0.5 | g |
| Concentrated hydrochloric acid | 100.0 | mL |
| Calcium chloride | 15.0 | g |
| Vitamin B1 | 1.0 | g |

Composition of microelement solution (verification run)

## (2-2) Production of 50 L scale sample

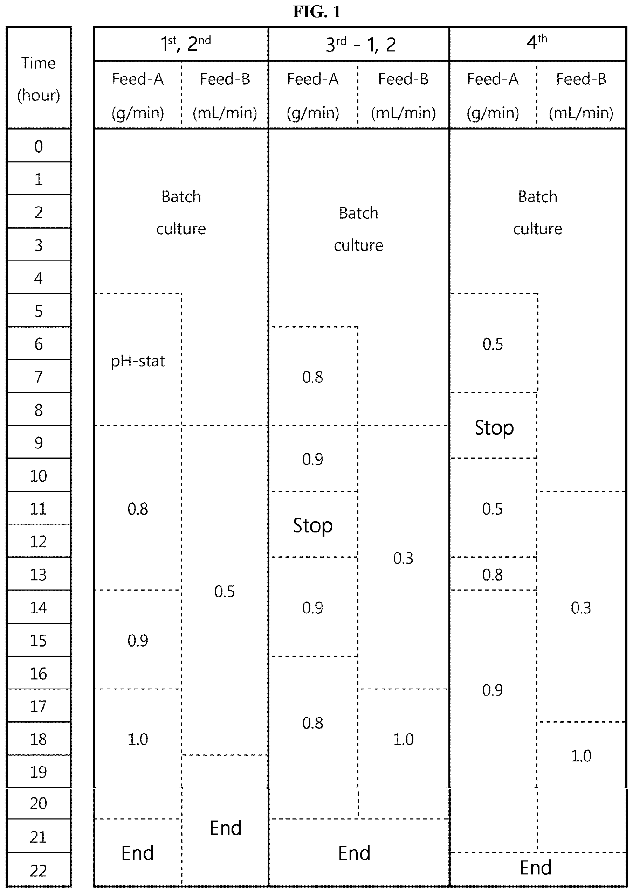

Based on the results of 5 L-scale culture and the results of partial condition changes, 50 L scale-up conditions for sample production were deduced (Tables 5 and 6). In addition, after establishing a 50 L feeding strategy, a process for 2-batch non-clinical sample production was carried out (FIG. 1).

[Table 5]

| Process | Item | | Condition |
|---|---|---|---|
| Seed culture 1 (500 mL Flask) | Inoculation volume | | RCB 1mL |
| | Medium | | LB medium |
| | Culture volume | | 100 mL x 1ea |
| | Flask volume | | 500 mL |
| | Kanamycin stock (50 mg/mL) | | 100 μL |
| | Shaking speed | | 200 rpm |
| | Temperature | | 37 °C |
| | Culture time | | 3~4 h |
| | Culture end | | 2~5 OD$_{600}$ |
| Seed culture 2 (1 L flask) | Inoculation volume | | 15 ML |
| | Medium | | LB medium |
| | Culture volume | | 300 mL x 3 ea |
| | Flask volume | | 1 L |
| | Kanamycin stock (50 mg/mL) | | 300 μL x 3 ea |
| | Shaking speed | | 200 rpm |
| | Temperature | | 37 °C |
| | Culture time | | 2.5 h |
| | Culture end | | 2~5 OD$_{600}$ |
| Main culture (75 L fermenter) | Incubation | | 0.9 L |
| | Culture start volume | | 30 L |
| | Incubator | | 75 L fermenter |
| | Culture type | | Fed-batch |
| | Culture temperature | | 37 ± 0.5 °C |
| | pH | | 7.0 ± 0.1 |
| | Incubation | Start | OD$_{600}$ = 80±10 |
| | | Temperature | 32 °C |
| | | Inducer | IPTG 1 mM |
| | | Added amount | 35 mL (1 M stock) |
| | | Time | 4 h |
| | Agitation speed | | 300 ~ 700 rpm |
| | Aeration | | 30 L./min |
| | DO control | | 30% |
| | Culture time | | ≥17 h |

Culture conditions for 50L verification run

[Table 6]

| Preparation Item | Volume | Composition | Preparation criterion (L$^{-1}$) | |
|---|---|---|---|---|
| Batch medium | 30 L | KH$_2$PO$_4$ | 13.3 | g |
| | | (NH$_4$)$_2$HPO$_4$ | 4.0 | g |
| | | Citric acid | 1.7 | g |
| | | Soytone | 1.0 | g |
| | | Y. extract (springer-0202) | 0.5 | g |
| | | Y. extract (springer-0251) | 0.5 | g |
| | | Sodium hyroxide | 2.9 | g |
| | | Antifoam204 (Sigma) | 0.3 | mL |
| | | MgSO$_4$·7H$_2$O | 1.1 | g |
| | | Glucose | 5.0 | g |
| | | Microelement solution | 5.0 | mL |
| Feed I (Carbon source) | 8L | Glucose | 500.0 | g |
| | | MgSO$_4$·7H$_2$O | 1.8 | g |
| Feed II (Nitrogen source) | 5 L | Soytone | 33.3 | g |
| | | Y. extract (Biospringer-0202) | 50.0 | g |
| | | Y. extract (Biospringer-0251) | 50.0 | g |
| IPTG stock (1 M, 1000 x) | 0.04 L | IPTG (238.3 g/mol) | 237.5 | g |
| Medium composition for 50 L verification run | | | | |

### Example (3) Development of fed-batch fermentation process for improving GST-TB4 productivity

It was confirmed that the above-described NR process has several problems such as low productivity, the formation of a medium precipitate and acetate accumulation. Accordingly, it was intended to develop a basic medium composition and process conditions that are able to solve the above-described problems.

### (3-1) Flask culture (batch-culture)

Flask culture experiments (flask 1, 2) were performed according to the presence or absence of a yeast extract (hereinafter, "YE") in a medium composition of a BNS-platform (Tables 7 and 8). A strain-specific growth parameter for the fed-batch process development was identified, and seed culture conditions for performing the main culture in a fermenter were confirmed.

[Table 7]

| Preparation Item | Composition | Criterion |
|---|---|---|

(continued)

| Preparation Item | | Composition | Criterion | |
|---|---|---|---|---|
| Flask 1 (w/ YE) | Flask 2 (w/o YE) | Tri-sodium citrate dihydrate | 2.0 | g |
| | | $KH_2PO_4$ | 4.0 | g |
| | | $Na_2HPO_4$ | 10.0 | g |
| | | $(NH_4)_2SO_4$ | 4.0 | g |
| | | Glucose | 5.0 | g |
| | | $MgSO_4 \cdot 7H_2O$ | 0.4 | g |
| | | Microelement solution | 6.0 | mL |
| | | Yeast extract | 1.0 | g |

Flask test medium composition

[Table 8]

| Item | Condition |
|---|---|
| Inoculation volume | RCB 1 mL |
| Medium | Main culture initial medium |
| Culture volume | 100 mL |
| Flask volume | 1 L (Baffled flask) |
| Kanamycin | 50 $\mu$g/mL |
| Shaking speed | 200 rpm |
| Temperature | 37 °C |
| Culture end | Stationary phase |

Flask test culture conditions

## (3-2) 5 L-scale fed-batch culture conditions

### (3-2-1) Feeding strategy

Fed-batch culture parameters (Table 9) were set to calculate the optimal feeding rate of a Feed-A medium. Feed-B was applied by calculating a rate of adding all preparation quantities by a constant feeding method during an induction section (the initiation of induction to the end of culture) according to a DoE experiment condition (Table 10).

[Table 9]

| Variables | Definition |
|---|---|
| $V_0$ | Initial fermentation volume (L) |
| $X_0$ | Initial strain concentration (OD) |
| $Y_{x/s}$ | Yield of strain based on substrate (OD/g/L) |
| dt | Feeding rate adjustment interval (h) |
| Si | Substrate concentration in feed medium (g/L) |
| $S_0$ | Initial substrate concentration (g/L) |
| $\mu$ | Cell specific growth rate (h$^{-1}$) |
| Gravity | Specific gravity of feed medium (g/mL) |

(continued)

| Variables | Definition |
|---|---|
| $F_0$ | Flow rate of feed medium (L/h) |

Exponential feeding parameters

[Table 10]

| Step | Feeding strategy | |
|---|---|---|
| | Feed-A (C-source) | Feed-B (N-source) |
| Batch growth | N/A | N/A |
| Fed-batch growth | Exponential feeding | |
| Induction | | Constant feeding |

DoE fed-batch culture feeding strategy

### (3-2-2) Medium preparation and process conditions

A DoE fed-batch culture process starting from a 2.5 L initial medium and terminated at a 4.2 L (0.7 L of feed-A, 1.0 L of feed-B) scale was established (Tables 11, 12 and 13), and the same conditions were applied to the DoE culture experiment.

[Table 11]

| Preparation item | Volume | Composition | Preparation criterion ($L^{-1}$) | |
|---|---|---|---|---|
| Initial medium | 2.5 L | Tri-sodium citrate dihydrate | 2.0 | g |
| | | $KH_2PO_4$ | 4.0 | g |
| | | $Na_2HPO_4$ | 10.0 | g |
| | | $(NH_4)_2SO_4$ | 4.0 | g |
| | | Y. extract (Biospringer-0202) | 1.0 | g |
| | | Antifoam204 (sigma) | 100.0 | μL |
| | | Glucose | 5.0 | g |
| | | $MgSO_4 \cdot 7H_2O$ | 0.4 | g |
| | | Microelement solution | 6.0 | mL |
| Feed-A (Carbon source) | 0.7 L | Glucose | 500.0 | g |
| | | $MgSO_4 \cdot 7H_2O$ | 10.0 | g |
| Feed-B (Nitrogen source) | 1 L | Y. extract (Biospringer-0202) | 150.0 | g |
| | | Antifoam (sigma 204) | 200.0 | μL |

Composition of DoE fed-batch culture medium (BNS-Platform)

[Table 12]

| Composition | Preparation criterion ($L^{-1}$) |
|---|---|
| Ferrous sulfate heptahydrate | 10.0 g |
| Zinc sulfate heptahydrate | 2.25 g |
| Cupric sulfate pentahydrate | 1.00 g |

(continued)

| Composition | Preparation criterion (L$^{-1}$) |
| --- | --- |
| Manganese sulfate monohydrate | 0.35 g |
| Sodium borate decahydrate | 0.23 g |
| Calcium chloride dehydrate | 2.00 g |
| Ammonium heptamolybdate tetrahydrate | 0.10 g |
| HCl 37% | 44.40 mL |

Composition of microelement solution (BNS-Platform)

[Table 13]

| Process | Item | Condition |
| --- | --- | --- |
| Seed culture (1 L baffled flask) | Inoculation | 1 mL |
| | Culture volume | 125 mL |
| | Flask volume | 1 L |
| | Kanamycin | 50 $\mu$g/mL |
| | Shaking speed | 200 rpm |
| | Temperature | 37 °C |
| | Culture time | $\geq$4 h |
| | Culture end | $\geq$2 OD$_{600}$ |
| Main culture (5 L fermenter) | Inoculation volume | 125 mL |
| | Culture volume | 2.5 L → 4.2 L |
| | Incubator | Sartorius B-plus |
| | Culture type | Fed-batch |

| | | Item | Condition |
| --- | --- | --- | --- |
| | Culture temperature | | 37 $\pm$ 0.5 °C |
| | pH | | 6.8 $\pm$ 0.1 |
| | Induction | Start condition | DoE condition |
| | | Temperature | DoE condition |
| | | Inducer | IPTG 1 mM |
| | | Added amount | 3 mL (1 M stock) |
| | | Time | Until the end of culture |
| | Agitation speed | | 300~1000 rpm |
| | Aeration | | 3 L/min |

Conditions for DoE fed-batch culture process

## (3-3) Deduction of optimal fed-batch culture conditions

To optimize an induction strategy, a response surface method (hereinafter, "RSM") test was designed. A 2-level full central composite experiment for two factors (induction starting point and induction temperature) was designed using Minitab software to carry out 13 runs of fed-batch culture experiments (Tables 14 to 16).

[Table 14]

| Factor | Experimental condition | | Center point condition |
|---|---|---|---|
| Induction cell density | High | $OD_{600} = 80 \pm 10$ | $OD_{600} = 55 \pm 10$ |
| | Low | $OD_{600} = 30 \pm 10$ | |
| Induction temperature | High | 34°C | 31°C |
| | Low | 28°C | |

DoE factors and condition ranges

[Table 15]

| Item | Condition | Details |
|---|---|---|
| DoE design method | Central synthesis method | -present the minimum experimental points to find the optimal response surface point for each factor<br>-forming experimental conditions with "vertex + axis point + central point" or the like. |
| Factor | 2 | Refer to Table 14 |
| Level | 2 | |
| Response | 2 | GST-Tβ4 productivity (mg/g), cell productivity (g/L) |
| Resolution | complete | -As the resolution of the DoE design is higher, it is recognized to be more statistically reliable.<br>-"complete" is the highest level of resolution, confirming the interaction between a factor and a main effect, and deducing the optimal condition |
| Repeated experiment | 1 | Number of repeated experiments: 1 cycle |
| Center point | 5 | -Experiment conditions for the intermediate value of each factor $OD_{600}$=55, temperature: 31 °C<br>-Designed so that the central point is close to the expected point in the optimal condition.<br>-When k (factor) = 2, statistically reliable no. of central points is 5 or more. |
| Axis point | 4 | Experimental point at the $\alpha$ value position from the center. Composed of 2k experimental points. |
| Vertex | 4 | Complete factor batch experiment condition: composed of 2k experimental points |
| $\alpha$ value | 1.41 | Distance from the center to the axis point, different according to the number of factors (k) |
| Run | 13 | No. of combinations of experiment conditions |

Conditions for DoE experiment design

[Table 16]

| Batch No. | DoE No. | Center point | Factor | |
|---|---|---|---|---|
| | | | Induction cell density ($OD_{600}$) | Induction temp. (°C) |
| 160928-B1 | 12 | 0 | 55.0 | 31.0 |
| 161005-B1 | 7 | -1 | 55.0 | 26.8 |
| 161005-B2 | 8 | -1 | 55.0 | 35.2 |

(continued)

| Batch No. | DoE No. | Center point | Factor | |
|---|---|---|---|---|
| | | | Induction cell density (OD$_{600}$) | Induction temp. (°C) |
| 161027-B1 | 5 | -1 | 19.6 | 31.0 |
| 161011-B2 | 10 | 0 | 55.0 | 31.0 |
| 161101-B1 | 2 | 1 | 80.0 | 28.0 |
| 161018-B2 | 13 | 0 | 55.0 | 31.0 |
| 161018-B1 | 11 | 0 | 55.0 | 31.0 |
| 161103-B1 | 3 | 1 | 30.0 | 34.0 |
| 161011-B1 | 9 | 0 | 55.0 | 31.0 |
| 161025-B1 | 1 | 1 | 30.0 | 28.0 |
| 161103-B2 | 6 | -1 | 90.4 | 31.0 |
| 161101-B2 | 4 | 1 | 80.0 | 34.0 |

Design of 2-level full central composite factor experiment

### (3-4) Synthesis of reproducibility of optimal culture process

Results of a RSM DoE culture experiment were statistically analyzed to deduce optimal conditions and the target standard of response values, and culture was carried out under optimal conditions to confirm the reproducibility of the cultures (Table 17).

[Table 17]

| Batch No. | Induction OD & Temp. | YE (Yeast extract) manufacturer | Antifoam manufacturer | Aeration |
|---|---|---|---|---|
| 161122-B1 | **DoE experiment optimal condition** | Biospringer 0202 | Sigma | 1 vvm |
| 161115-B1 | | BD | Sigma | 1 vvm |
| 161122-B2 | | BD | Sigma | 1 wm |
| 161124-B1 | | BD | Dow corning ("DC") | 0.5 vvm |

Confirmation of reproducibility of DoE optimal conditions

### Example (4) Optimal condition scale-up for 50 L-scale process (sample production)

### (4-1) Conditions for culture process

A 50L culture process for sample production was performed by scale-up of optimal conditions for developing a 5L-scale process (Tables 18 and 19).

[Table 18]

| Process | Item | | Condition |
|---|---|---|---|
| Seed culture 1 (500 mL Flask) | Inoculation volume | | RCB 1 mL |
| | Culture volume | | 100 mL x 1 ea |
| | Flask volume | | 500 mL baffled flask |
| | Shaking speed | | 200 rpm |
| | Temperature | | 37 °C |
| | Culture time | | 5~6 h |
| | Culture end | | $\geq$2 $OD_{600}$ |
| Seed culture 2 (1 L Flask) | Inoculation volume | | 15 mL |
| | Culture volume | | 315 mL x 4 ea |
| | Flask volume | | 1 L baffled flask |
| | Shaking speed | | 200 rpm |
| | Temperature | | 37 °C |
| | Culture time | | 3~4 h |
| | Culture end | | $\geq$2 $OD_{600}$ |
| Main culture (75 L fermenter) | Inoculation | | 1.25 L (5%, v/v) |
| | Culture start volume | | 25 L |
| | Incubator | | 75 L fermenter |
| | Culture type | | Fed-batch |
| | Culture temperature* | | 36.5 $\pm$ 0.5 °C |
| | pH* | | 6.8 $\pm$ 0.1 |
| | Induction | Start | $OD_{600}$ = 54$\pm$5 |
| | | Temperature | 30.0 $\pm$ 1.0 °C |
| | | Inducer | IPTG 1 mM |
| | | Added amount | 40 mL (1 M stock) |
| | | Time | $\geq$9 h |
| | Agitation speed** | | 300~700 rpm |
| | Aeration | | 15 L/min |
| | DO control | | 30% |
| | Culture time | | $\geq$2 h |

Conditions for optimal 50L scale-up culture * A culture temperature and pH are the same as BNS-platform conditions.

** The maximum agitation speed of 75 L equipment (700 rpm) is reflected.

[Table 19]

| Preparation item | Volume | Composition | Preparation criterion (L$^{-1}$) | |
|---|---|---|---|---|
| Batch medium | 25 L | Tri-sodium citrate dihydrate | 2.0 | g |
| | | KH$_2$PO$_4$ | 4.0 | g |
| | | Na$_2$HPO$_4$ | 10.0 | g |
| | | (NH$_4$)$_2$SO$_4$ | 4.0 | g |
| | | Y. extract (BD) | 1.0 | g |
| | | Antifoam (Dow corning) | 2.0 | mL |
| | | Glucose | 5.0 | g |
| | | MgSO$_4$·7H$_2$O | 0.4 | g |
| | | Microelement solution | 6.0 | mL |
| Feed-A (Carbon source) | 7 L | Glucose | 500.0 | g |
| | | MgSO$_4$·7H$_2$O | 10.0 | g |
| Feed-B (Nitrogen source) | 10 L | Yeast extract (BD) | 150.0 | g |
| | | Antifoam (Dow corning) | 5.0 | mL |
| IPTG stock (1 M, 1000 x) | 0.04 L | IPTG | 238.3 | g |
| Seed medium | 1.4 | Tri-sodium citrate dihydrate | 2.0 | g |
| | | KH$_2$PO$_4$ | 4.0 | g |
| | | Na$_2$HPO$_4$ | 10.0 | g |
| | | (NH$_4$)$_2$SO$_4$ | 4.0 | g |
| | | Y. extract (BD) | 1.0 | g |
| | | Glucose | 5.0 | g |
| | | MgSO$_4$·7H$_2$O | 0.4 | g |
| | | Microelement solution | 6.0 | mL |

Medium composition for 50 L optimal condition scale-up culture

**(4-2) Recovery conditions**

**(4-2-1) Cell slurry recovery**

(A) Recovery of culture broth

- Recovery time: overnight after cooling of culture broth (≤15.0 °C)
- Recovery volume: 40 to 42 L

(B) Recovery of cell slurry (continuous centrifugation)

① 1st recovery

- Solid content before recovery: ≥20%
- Bowl rotation speed: 9,500 rpm (11,119 x g)
- Inlet flow rate: 100 L/M
- Outlet pressure: 100 kPa
- Cell washing: 40L of deionized water (DIW)

② 2nd recovery

- Solid content before recovery: ≥ 10%
- Bowl rotation speed: 9,500 rpm
- Inlet flow rate: 100 L/m
- Outlet pressure: 100 kPa
- Cell washing: 40 L of DIW

③ 3rd recovery

- Solid content before recovery: ≥10%
- Bowl rotation speed: 9,500 rpm
- Inlet flow rate: 100 L/m
- Outlet pressure: 100 kPa

**(4-2-2) Cell lysis and supernatant recovery**

(A) Preparation of lysis buffer (20X)

- 400 mM Tris buffer, pH 8.0 ± 0.2 (suitable reagent: HCl), Conductivity 17 ± 2 mS/cm
- 400 mM Tris buffer composition: Tris 21.8 g/L, Tris-HCl 34.7 g/L

(B) Cell slurry preparation (15±1.5%, w/w)

① The volume was adjusted so that the solid content was 15±1.5% by adding 20X lysis buffer and DIW.

② Calculation formula

# Total weight = Cell cake weight / 0.15
# 20X Lysis Buffer weight = Total weight / 20
# Amount of added DIW = Total weight - slurry weight - 20X Lysis buffer weight

(C) CIP (Microfluidizer)

- 0.5 N NaOH: 11,000 psi condition, 300 mL once, 500 mL once
- DIW: 11,000 psi condition, 300 mL once, 500 mL once

(D) Cell lysis (Microfluidizer)

① EQ: 11,000 psi condition, * 300 mL 1X lysis buffer

② Cell lysis: 11,000 psi condition, 15±1.5% cell slurry lysis twice in a row

- Cell lysis criteria: OD ratio before and after lysis ≤ 30%
- Calculation of cell lysis rate: $(OD_{600}$ after lysis/ $OD_{600}$ before lysis$) \times 100$

③ Flushing: 11,000 psi condition, 1X lysis buffer 50 mL

(E) Recovery of supernatant of cell lysate (continuous centrifugation)

- Bowl rotation speed: 9,500 rpm

- Inlet flow rate: 100 L/M

- Outlet pressure: 250 kPa

**(4-2-3) Depth filtration**

(A) Filter information

    ① Depth filter:

-     Millistak+POD C0HC: 0.11 m$^2$ × 2ea

-     Millistak+POD F0HC: 0.11 m$^2$ × 2ea

    ② Membrane filter

-     Opticap XL10 (0.45μm) 0.69 m$^2$ × 1ea

(B) Filter washing

-     Depth filter: 100 L/m$^2$ of DIW, flow rate 300 LMH (1,100 mL/min)

-     Sterile filter: 10 L/m$^2$ of DIW, flow rate 1,000 mL/min

(C) Depth filtration

| Step | Input | Volum | Feed Flux |
|---|---|---|---|
| Clarification | Cell lysate | N/A | 75 LMH (275 mL/min) |
| Flushing | **DIW** | 11.6 L | 75 LMH (275 mL/min) |
| Air Flushing | N/A | N/A | 300 LM (1,100 mL/min) |

**Example (5) Culture broth analysis**

**(5-1) Measurement of cell density**

The cell concentration in the culture broth was obtained by measuring optical density at 600 nm. 1 mL of the culture broth was diluted with distilled water to have $OD_{600}$=0.2 to 0.5, and an optical density ($OD_{600}$) was measured, and then multiplied by a dilution factor, thereby obtaining a cell density. Sample dilution was performed by standardization in the manner as shown in Table 20.

[Table 20]

| OD600 | Dilution rate | Dilution volume |
|---|---|---|
| 1 ~ 3 | 10 x | 1 mL |
| 4 ~ 7 | 20 x | 1 mL |
| 8 ~ 10 | 30 x | 6 mL |
| 10 ~ 20 | 50 x | 10 mL |
| 20 ~ 40 | 100 x | 10 mL |
| 40 ~ 60 | 200 x | 20 mL |
| 60 ~ 100 | 300 x | 30 mL |
| 100 ≤ | 500 x | 50 mL |

Dilution conditions for OD value measurement

**(5-2) Cell metabolite measurement**

Glucose and acetate concentrations in the culture broth were measured from a supernatant obtained by centrifuging 1 mL of the culture broth at 13,000 rpm for 5 minutes using a Cedex Bio (Roche, Switzerland) analyzer (SOP No. FE02-921).

**(5-3) Measurement of cell productivity**

**(5-3-1) Measurement of wet cell weight (g/L)**

A wet cell weight was measured by the following method.

① Measure 500 mL tube + SUPPORT CUSHION weight (prepare two)

② Add aliquots of culture broth by 500 mL (use 500 mL measuring cylinder)

③ Centrifuge under conditions of 4500 rpm and 4 °C for 1 hour (CE-6140 equipment)

④ Discard supernatant and measure weight of pellet containing container (tube + cushion)

⑤ Calculate wet cell weight (g/L)

$$\text{Wet cell weight} =$$

$$(\text{pellet contained weight} - \text{container weight}) \text{ g} / 0.5 \text{ L (volume of culture broth)}$$

⑥ Determine cell productivity by the average of two measured wet cell weights.

**(5-3-2) Measurement of solid content (%)**

① Prepare two micro tubes

② Measure and record tube weights using precision balance

③ Measure and record weight of tube containing 1 mL culture broth

④ Centrifuge under conditions of 13,000 rpm and 4 °C for 5 minutes

⑤ Discard supernatant and measure weight of tube containing pellet ⑥ Calculate solid content (%):

$$\frac{pellet\ contained\ weight\ -\ tube\ weight}{weight\ including\ 1mL\ medium\ -\ tube\ weight} \times 100$$

⑦ Take mean value of two measured solid contents

**(5-4) Measurement of GST-Tβ4 productivity (SDS-PAGE & Protein quantification)**

① Add 1,000 μL of lysis buffer (50 mM Tris, 100 mM NaCl, 0.1 % 2ME, 5 mM EDTA, pH 7.0) to cell pellet sample of OD600=5 and perform vortexing
② Perform sonication for 10 seconds and leave on ice for 1 minute (repeated 7 times)
③ After centrifugation at 10,000 X g for 20 minutes, take 15 μL of supernatant
④ Prepare 15 μL of bovine serum albumin (BSA) protein standard reagent to be contained at 0.5, 1, 2 or 4 μg

⑤ Mix 15 μL sample (centrifugation supernatant and BSA standard) with 5 μL of the sample buffer mixture prepared in ④ (total volume: 20 μL)

⑥ React at 70 °C for 10 minutes

⑦ Load entire sample (20 μL) to each well of gel

⑧ Run under conditions of 200 V and 400 mA for 35 minutes

⑨ Perform Coomassie blue staining for 20 minutes or more

⑩ Destain for 2 hours or more

⑪ Analyze image of gel band density using densitometer

## Experimental Example (1) Confirmation of reproducibility of Chinese Northland process

### (1-1) 5 L scale culture

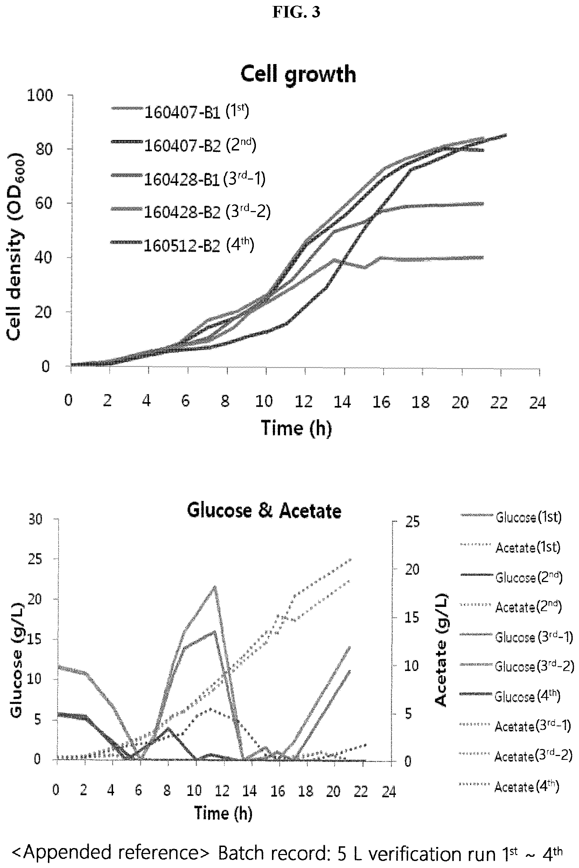

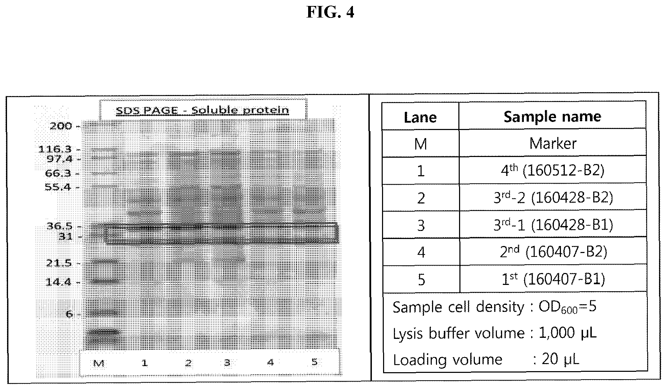

5 L scale verification run was carried out by scaling down an NR process, and the normal expression of GST-Tβ4 was confirmed through SDS-PAGE (FIG. 4). The final cell density was $OD_{600}$=85, 81, 61, 41, 86 (Table 21), and except two tertiary batches (Batch No. 160428-B1, B2), growth similar to the NR process was shown (FIG. 3). However, in the entire process, glucose in a culture broth was accumulated to control growth by interrupting and resuming feed-A supply, and while proceeding to the $4^{th}$ process, continuous improvement in C-source (feed-A medium) supply was attempted. However, except the $4^{th}$ process, acetate accumulation was observed.

Accordingly, it was necessary to more precisely improve a feeding rate of a feed-A medium. In addition, after the medium was prepared, a large amount of precipitates were formed, and the pH of the medium composition was not optimized, and therefore, the disadvantage of a conventional NR process, such as the requirement of a separate titration process before inoculation, was confirmed.

[Table 21]

| Batch No. | Batch name | Induction start ($OD_{600}$) | Induction temperature (°C) | Final $OD_{600}$ | Cell productivity (g/L) |
|---|---|---|---|---|---|
| 160407-B 1 | 5 L $1^{st}$ | 77 | 32 | 85 | 104 |
| 160407-B2 | 5 L $2^{nd}$ | 75 | 32 | 81 | 106 |
| 160428-B1 | 5 L $3^{rd}$-1 | 60 | 32 | 61 | 86 |
| 160428-B2 | 5 L $3^{rd}$-2 | 40 | 32 | 41 | 58 |
| 160512-B2 | 5 L $4^{th}$ | 75 | 32 | 86 | 125 |

Results for 5 L verification run culture

### (1-2) 50 L scale culture (sample production)

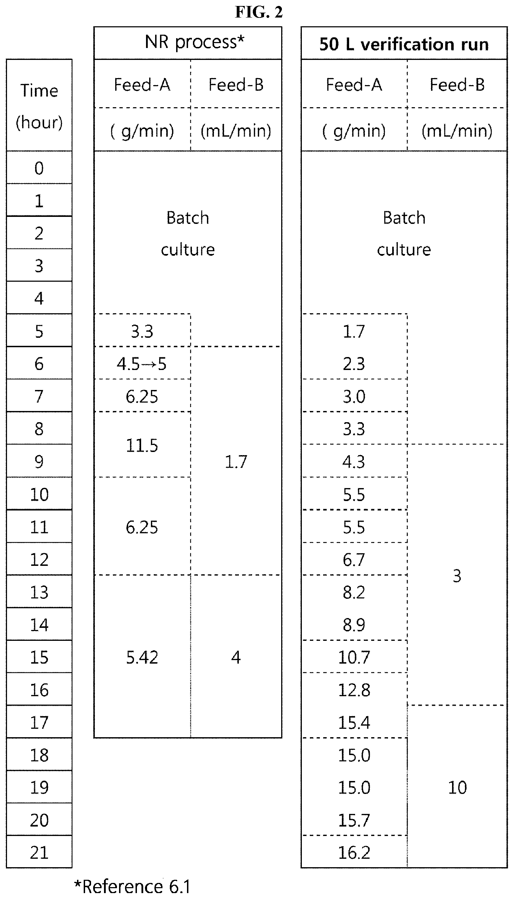

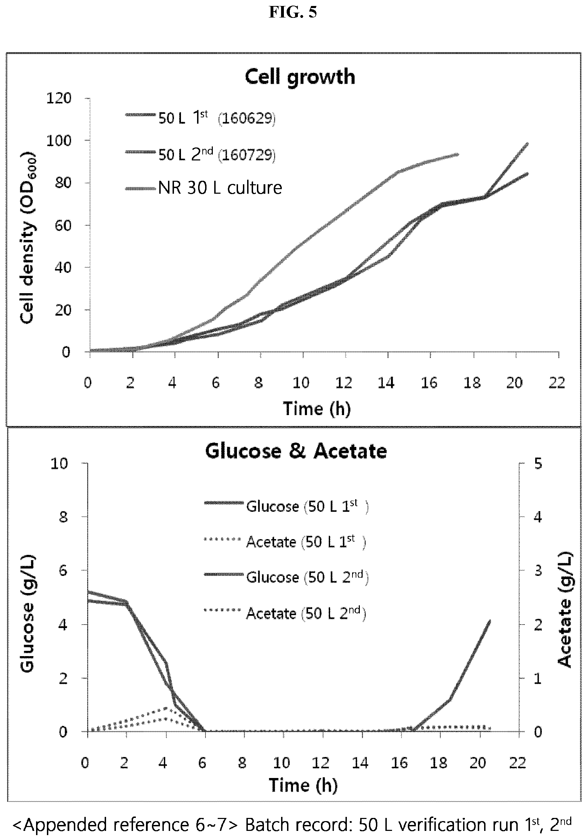

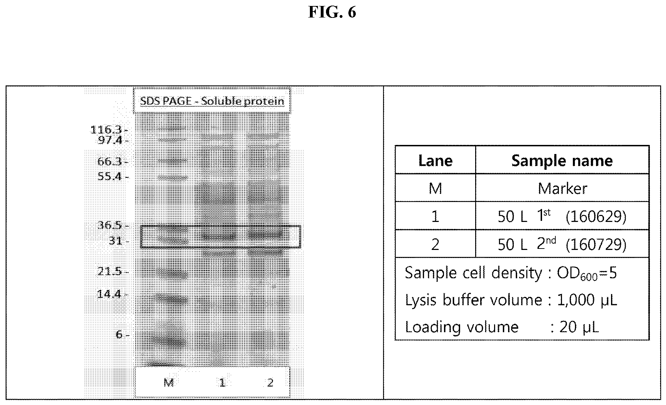

Based on results for 5 L scale verification culture performed by scale down of an NR process, 50 L scale process conditions were deduced (Tables 5 and 6, FIG. 2), 2 batches of 50 L sample production were conducted. Compared to the NR process (results for 30 L scale culture), the lag phase of the initial cell growth was long in both batches ($1^{st}$ and $2^{nd}$), but the final cell density reached $OD_{600}$=84 or, 98, which was similar to that of the NR process (FIG. 5, Table 22). An acetate concentration was 1 g/L or less in both batches, which was well controlled until the end of the culture, but in the case of the $2^{nd}$ batch (Batch No. 160729), glucose was accumulated up to 2 g/L in the second half (after 17 hours) of the culture. GST-Tβ4 expression was confirmed through SDS-PAGE (FIG. 6), and after continuous centrifugation of the culture broth, finally, 3.0 kg or 3.3 kg of cells were recovered (Table 22).

[Table 22]

| Batch N o. | Batch name | Final $OD_{600}$ | Volume of culture broth | Cell productivity (g/L) | Recovery type | Recovered cell weight (kg) |
|---|---|---|---|---|---|---|
| 160629 | 50 L $1^{st}$ | 84.3 | 36.7 | 128 | Cell Slurry | 3.3 |
| 160729 | 50 L $2^{nd}$ | 98.3 | 36.9 | 122 | Cell cake | 3.0 |

Culture results of 50 L verification run

**Experimental Example (2) Development of fed-batch fermentation process for improvement in GST-Tβ4 productivity**

**(2-1) Flask culture**

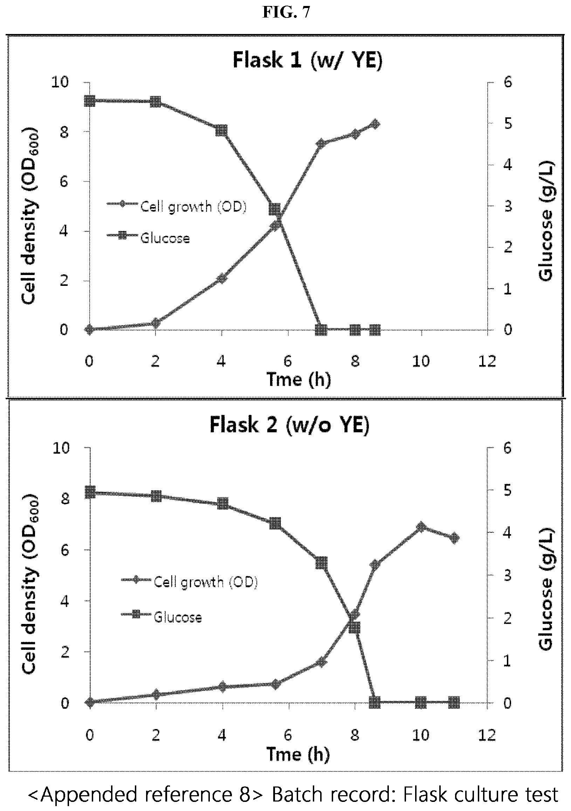

To confirm cell growth parameters and seed culture process time, required for calculating a feeding rate of a DoE fed-batch culture process, a flask culture experiment was performed. Flask 1 was carried out with the same medium composition as the initial medium of a fed-batch, and flask 2 was carried out with a composition excluding only YE from the initial medium. Flask 1 (containing YE) has a shorter lag phase than flask 2, and the maximum cell density ($OD_{600}$) was grown until 8.3 (flask 1) or 6.9 (flask 2) (FIG. 7). Based on the OD value and glucose concentration of an exponential phase, a cell yield (Yx/s) with respect to a glucose substrate and the maximum specific growth rate (μm) were calculated (Table 23). In the 5 L fed-batch culture experiment using a fermenter, a YE-containing medium composition was selected, and therefore, a feeding rate was calculated using the parameter value of flask 1. For a 5 L fed-batch seed culture process, $OD_{600}$ was ≥2, and culture time was determined as 4 hours or more.

[Table 23]

| Parameter | | Flask 1 | | Flask 2 | |
|---|---|---|---|---|---|
| Exponential phase | Time (h) | 4 | 7 | 5.6 | 8.6 |
| | Glucose (g/L) | 4.8 | 0 | 4.2 | 0 |
| | $OD_{600}$ | 2.1 | 7.5 | 0.7 | 5.4 |
| $Y_{X/S}$ | | 1.13 | | 1.11 | |
| $\mu_m$ ($h^{-1}$) | | 0.43 | | 0.66 | |
| $X_m$ ($OD_{600}$) | | 8.3 | | 6.9 | |
| HU024 cell growth parameters | | | | | |

**(2-2) Feeding strategy**

**(2-2-1) Exponential fed-batch parameter**

Referring to results of the flask 1 culture test that has been previously performed, each parameter for calculating the feeding rate of the C-source was set as shown in Table 24. In the flask batch culture of the YE-containing initial medium composition, the maximum specific growth rate (μm) was 0.43 $h^{-1}$ (Table 23), and since the rapid growth of cells in *E. coli* culture is disadvantageous for the production of a soluble protein-type product, a quasi-steady state was induced through precise control of a feeding rate to control a specific growth rate to be 0.3 to 0.14 $h^{-1}$.

[Table 24]

| Parameter | Set value |
|---|---|
| $V_0$ | 2.5 |
| $X_0$ | 8 |
| $Y_{X/S}$ | 1.13 |
| dt | 1 |
| Si | 500 |
| μ | 0.3~0.14 |
| gravity | 1.1 |
| $F_0$ | 0.012 |
| Exponential fed-batch parameters | |

**(2-2-2) Calculation of feeding rate (feed-A)**

A feeding rate of the feed-A medium was calculated using the following formulas. The first feeding rate at the time point of ending batch-culture was calculated by substituting each parameter value (Table 24) into Equation ④, and subsequently, a VX value (Equation ①), a V value (Equation ②), a X value (Equation ③) and a F value (Equation ④) were sequentially calculated every hour (dt). Since the calculated flow rate (F) is expressed as L/h, after conversion to mL/min, finally, the flow rate was converted to the units of g/min, which is the unit of speed of pumping equipment (Table 25).

$$VX = V_0 \cdot X_0 + F_0 * dt * Si * Y_{X/S} \quad \text{---------------------} \quad ①$$

$$V = V_0 + dt * F_0 * gravity \quad \text{---------------------} \quad ②$$

$$X = VX / V \quad \text{---------------------} \quad ③$$

$$F = \frac{X_0 \cdot V_0 \, (e^{\mu t} - 1)}{S_0 \cdot Y_{X/S} \cdot t} \quad \text{---------------------} \quad ④$$

[Table 25]

| t | X | V | VX | F (L/hr) | F (mL/min) | F (g/min) | μ |
|---|---|---|----|----------|-----------|-----------|---|
| 0~4 | | | | Batch culture | | | |
| 4 | 8 | 2.50 | 20.0 | 0.012 | 0.21 | 0.2 | 0.3 |
| 5 | 11 | 2.51 | 27.0 | 0.017 | 0.28 | 0.3 | 0.3 |
| 6 | 14 | 2.53 | 36.4 | 0.023 | 0.38 | 0.4 | 0.3 |
| 7 | 19 | 2.55 | 49.2 | 0.019 | 0.32 | 0.4 | 0.2 |
| 8 | 23 | 2.57 | 60.1 | 0.024 | 0.39 | 0.4 | 0.2 |
| 9 | 28 | 2.59 | 73.4 | 0.026 | 0.43 | 0.5 | 0.18 |
| 10 | 34 | 2.62 | 87.9 | 0.031 | 0.51 | 0.6 | 0.18 |
| 11 | 40 | 2.65 | 105.2 | 0.037 | 0.61 | 0.7 | 0.18 |
| 12 | 47 | 2.69 | 125.9 | 0.044 | 0.73 | 0.8 | 0.18 |
| 13 | 55 | 2.73 | 150.8 | 0.046 | 0.77 | 0.8 | 0.16 |
| 14 | 64 | 2.78 | 176.9 | 0.054 | 0.91 | 1.0 | 0.16 |
| 15 | 73 | 2.83 | 207.6 | 0.064 | 1.06 | 1.2 | 0.16 |
| 16 | 84 | 2.90 | 243.6 | 0.065 | 1.08 | 1.2 | 0.14 |
| 17 | 95 | 2.96 | 280.3 | 0.075 | 1.24 | 1.4 | 0.14 |
| 18 | 106 | 3.04 | 322.4 | 0.086 | 1.43 | 1.6 | 0.14 |
| 19 | 119 | 3.13 | 370.8 | 0.099 | 1.64 | 1.8 | 0.14 |
| 20 | 132 | 3.23 | 426.6 | 0.113 | 1.89 | 2.1 | 0.14 |

Calculation of exponential feeding rate

### (2-3) Confirmation of cell growth in fed-batch process

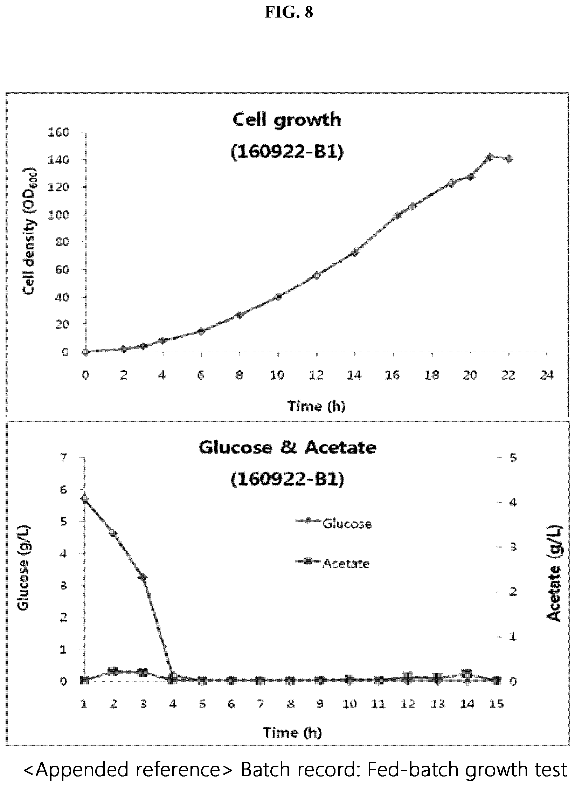

To confirm basic cell growth in a fed-batch process using a 5 L fermenter, fed-batch culture was conducted by supplying feed-A at the calculated feed-A feeding rate (Table 23) (FIG. 8). A total of 788.8 mL of the feed-A medium was supplied until the end of the culture, and the maximum cell density ($OD_{600}$) predicted when the substrate yield ($Y_{X/S}$) was applied by converting a total glucose amount of 397 g (containing initial glucose) added into an incubator into the final culture volume (3.3 L) was 136, almost similar to the maximum cell density ($OD_{600}$=142) as the actual culture result. A acetate concentration was well controlled to less than 1 g/L from the beginning to the end of the culture, and as the initial glucose in the initial media was rapidly consumed after 3 hours of culture, the concentration was well controlled close to 0 g/L until the end of culture even after the beginning of glucose feeding (4h), and since the quasi-steady state of the actual cell growth was well induced close to a set $\mu$ value, it was considered that the previously calculated feeding rate (Table 25) was appropriate. In addition, the cell growth result for the fed-batch culture experiment was utilized as a material for predicting the starting point of a DoE culture experiment.

**(2-4) Deduction of optimal culture conditions (DoE)**

**(2-4-1) Culture result**

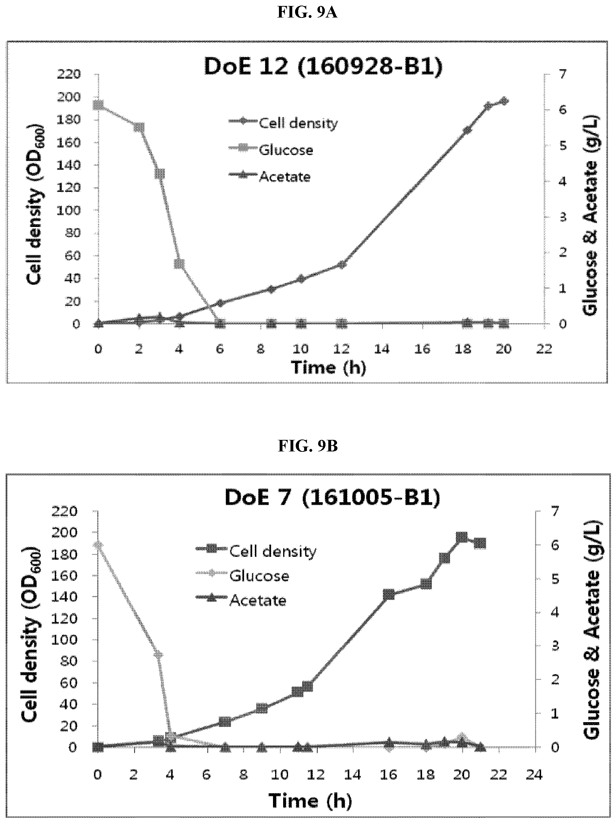

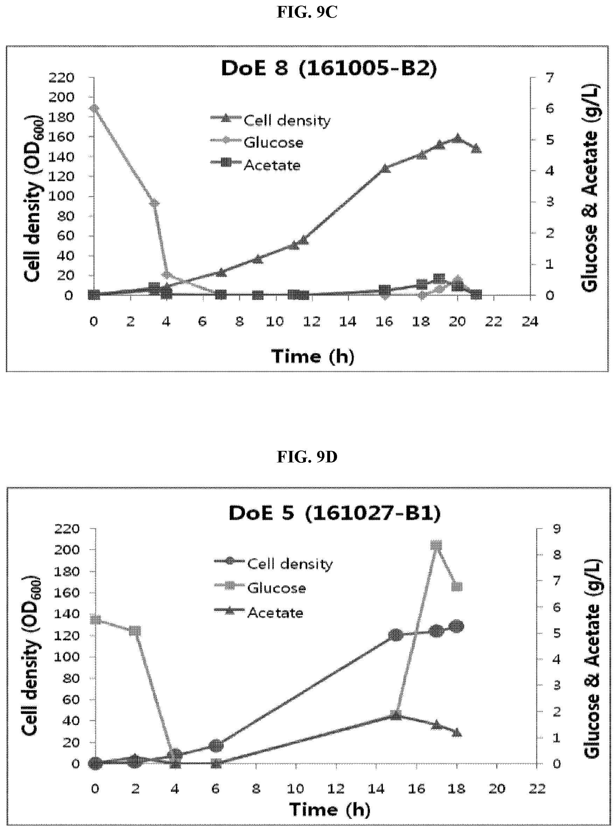

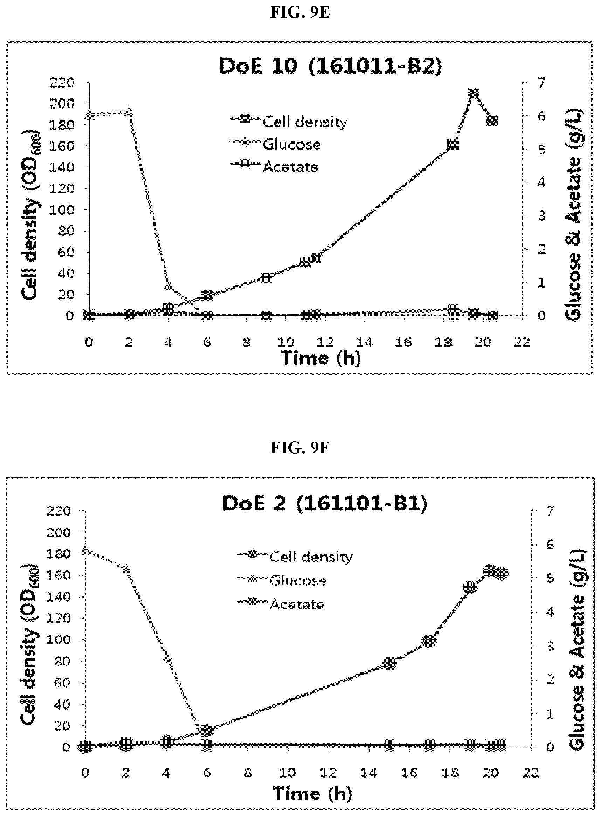

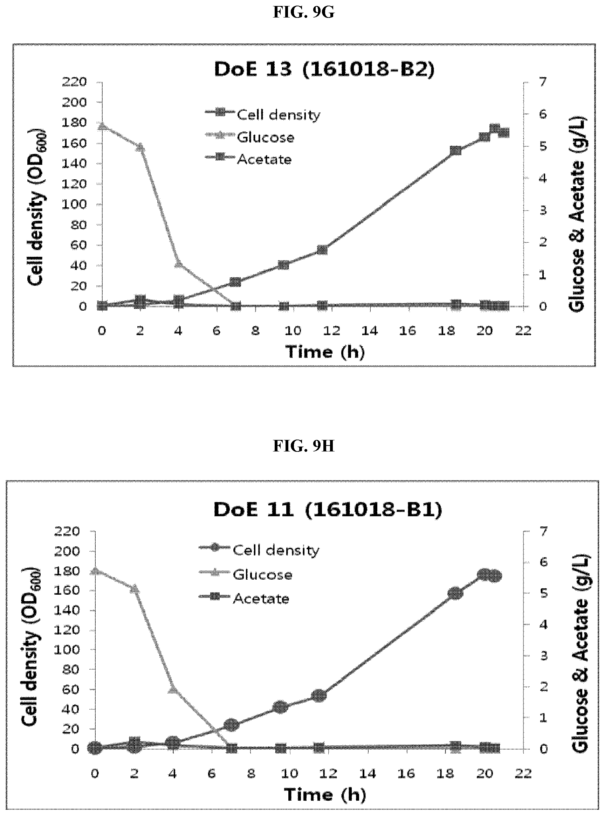

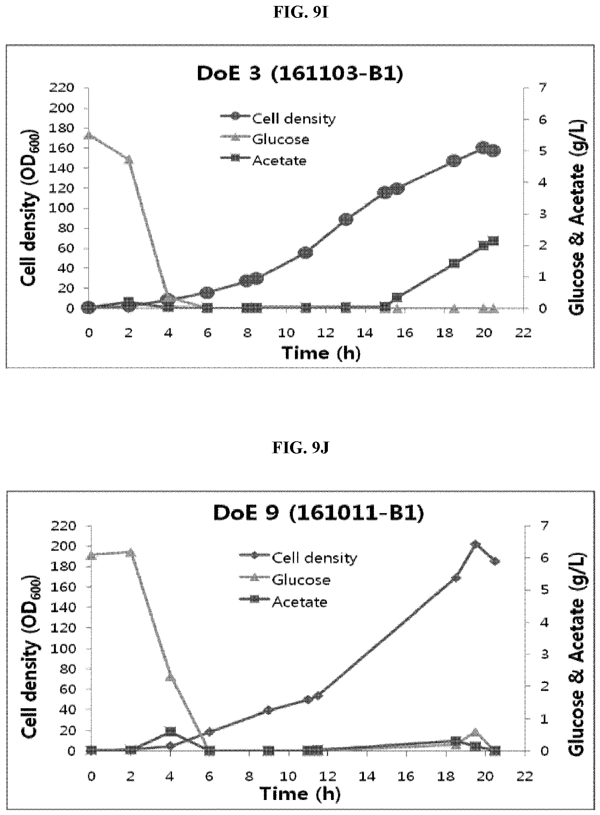

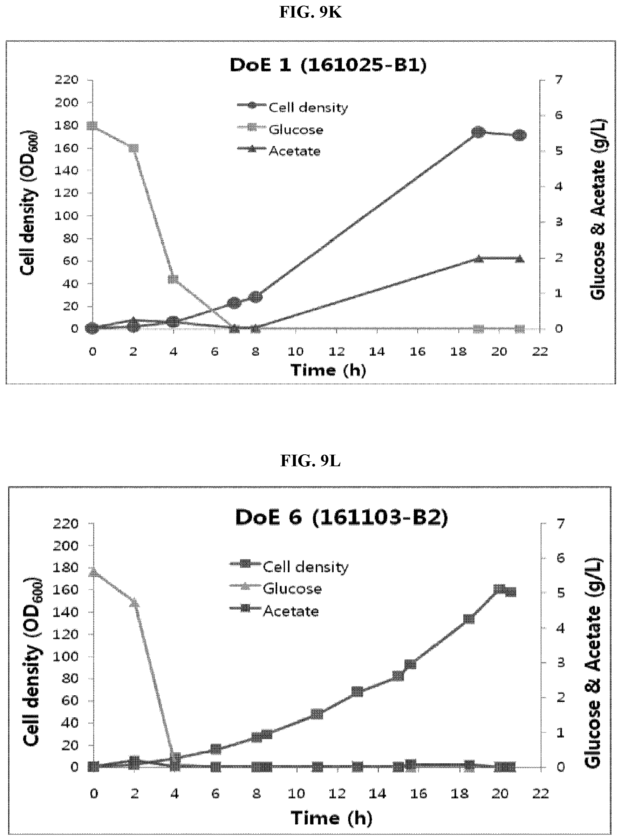

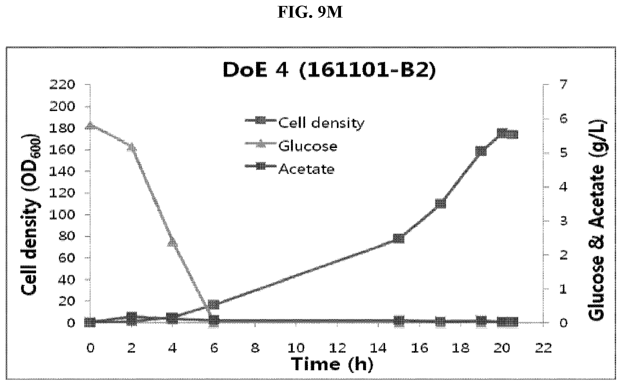

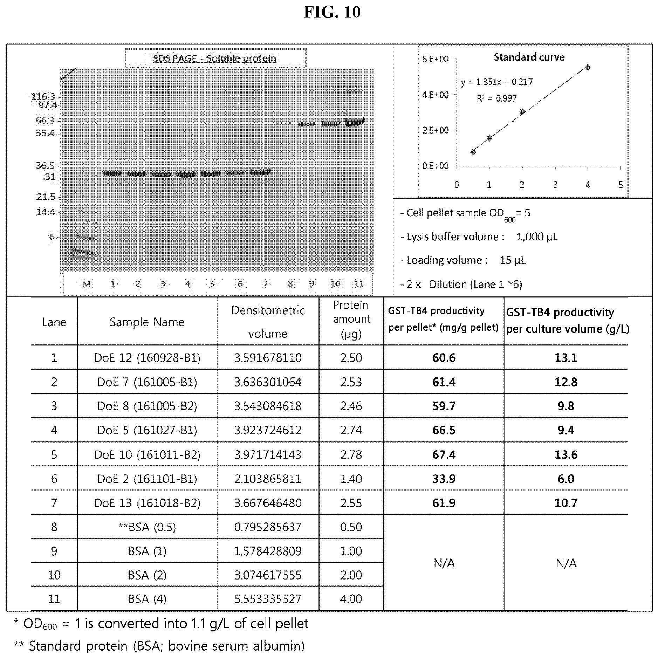

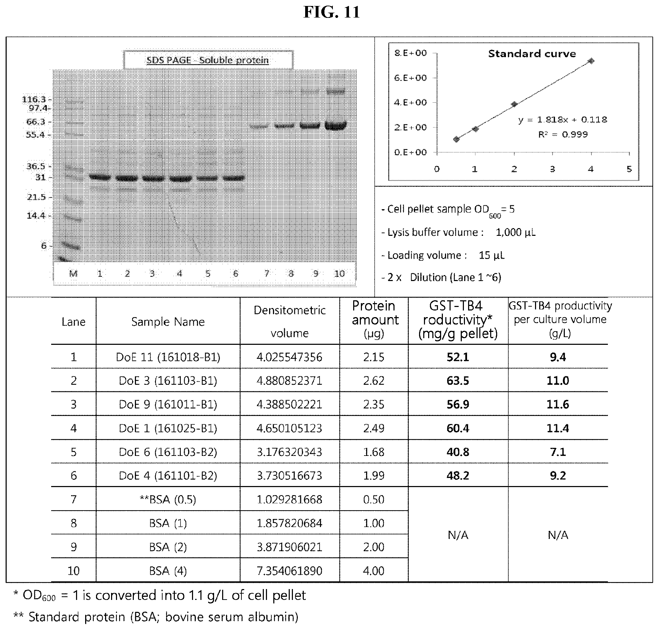

Thirteen runs of culture were performed according to DoE RSM design conditions. At the starting point of induction under each DoE condition, IPTG addition and a temperature shift as well as feed-B medium feeding were initiated, and a feed-B medium consisting of high concentration YE was only provided in the induction phase, so that culture was conducted with a strategy of maximizing cell growth and GST-T$\beta$4 productivity. Except the DoE 5th batch (Batch No. 161027-B1), the maximum cell density ($OD_{600}$) grew to 140 to 196, and was overall well controlled without excessive glucose and acetate accumulation (FIGS. 9A to 9M). In the DoE 5th batch (161027-B1), according to the RSM DoE experiment design, as the fastest induction starting condition ($OD_{600}$=19.6), glucose and acetate were accumulated the most in the culture broth, considered to be due to induction and temperature shift before sufficient cell growth. The mean cell productivity of the entire DoE batch runs was 185$\pm$31 g/L (wet cell weight), and the total mean GST-T$\beta$4 productivity confirmed through SDS-PAGE and gel band image analysis (FIGS. 10 and 11) was 56.4$\pm$10.0 mg/g (productivity per wet cell) and 10.4$\pm$2.2 g/L (productivity per culture volume), and cell productivity with respect to the mean value of 2 batches (primary, secondary) of a 50 L verification run was increased approximately 1.5-fold, and GST-T$\beta$4 productivity was increased approximately 2.5-fold (Table 26).

[Table 26]

| Batch No. | DoE No. | Initiation of induction (OD$_{600}$) | Temperature of induction (℃) | Final OD$_{600}$ | Cell productivity (g/L) | GST-Tβ productivity (mg/g) |
|---|---|---|---|---|---|---|
| 160928-B1 | DoE 12 | 52 | 31 | 196.1 | 202 | 60.56 |
| 161005-B1 | DoE 7 | 56 | 26.8 | 189.5 | 203 | 61.36 |
| 161005-B2 | DoE 8 | 56 | 35.2 | 148.6 | 140 | 59.68 |
| 161027-B1 | DoE 5 | 17 | 31 | 128.3 | 108 | 66.51 |
| 161011-B2 | DoE 10 | 54 | 31 | 183.9 | 183 | 67.37 |
| 161101-B1 | DoE 2 | 78 | 28 | 161.3 | 221 | 33.86 |
| 161018-B2 | DoE 13 | 55 | 31 | 156.4 | 183 | 61.92 |
| 161018-B1 | DoE 11 | 53 | 31 | 164.4 | 182 | 52.11 |
| 161103-B1 | DoE 3 | 29 | 34 | 157.0 | 198 | 63.51 |
| 161011-B1 | DoE 9 | 54 | 31 | 185.3 | 194 | 56.95 |
| 161025-B1 | DoE 1 | 28 | 28 | 171.0 | 172 | 60.43 |
| 161103-B2 | DoE 6 | 93 | 31 | 158.0 | 212 | 40.78 |
| 161101-B2 | DoE 4 | 77 | 34 | 173.8 | 202 | 48.17 |
| 160629 | 50 L 1 차 | 69 | 32 | 84 | 128 | 25.2 |
| 160729 | 50 L 2 차 | 70 | 32 | 98 | 122 | 19.9 |

Summary of DoE 13 runs culture process results

**(2-4-2) DoE statistical analysis**

**(2-4-2-1) Analysis of variance (ANOVA)**

ANOVA was conducted on cell productivity and GST-Tβ4 productivity values of all DoE-based batches (13 runs) using Minitab software (Table 27). $R^2$ values representing model explanatory power were 79.78% (GST-Tβ4 productivity) and 58.57% (cell productivity), determined as common levels, considering an experiment design without repetition (considering development period) and characteristics of a culture field in which several factors were involved in a response value. Since the p-values of induction OD were 0.002 (GST-Tβ4 productivity) and 0.029 (cell productivity), they were confirmed as significant factors with respect to both of the response values (GST-Tβ4 and cell productivity) at a confidence level less than 0.05. As the p-values of an induction temperature were 0.399 (GST-Tβ4 productivity) and 0.299 (cell productivity), it was determined that there is no effect on GSTTB4 and cell productivity results. Since p-values in the "induction OD*temperature" term were 0.374 and 0.413, it was confirmed that there was no "reciprocal" effect, referred to as the inter-condition interference between two factors, on the response values.

[Table 27]

| GST-Tβ4 productivity | | | Cell productivity | | |
|---|---|---|---|---|---|
| Term | Coefficients | P-value | Term | Coefficients | P-value |
| Constant | 59.780 | 0.000 | Constant | 188.800 | 0.000 |
| Induction OD | -9.788 | 0.002 | Induction OD | 25.010 | 0.029 |
| Temperature | 1.878 | 0399 | Temperature | -10.262 | 0.299 |
| Induction OD x Induction OD | -4.464 | 0.087 | Induction OD x Induction OD | -6.275 | 0.543 |
| Temperature x Temperature | -1.028 | 0.661 | Temperature x Temperature | -0.525 | 0.959 |
| Induction OD x Temperature | 2.809 | 0374 | Induction OD x Temperature | -11.250 | 0.413 |
| Curvature effect | - | 0.205 | Curvature effect | - | 0.819 |
| $R^2$ | - | 79.78% | $R^2$ | - | 58.57% |
| $R^2$-adjusted | - | 65.34% | $R^2$-adjusted | - | 28.98% |

Regression coefficient of RSM DoE experiment and results for ANOVA

#### (2-4-2-2) Residual plot

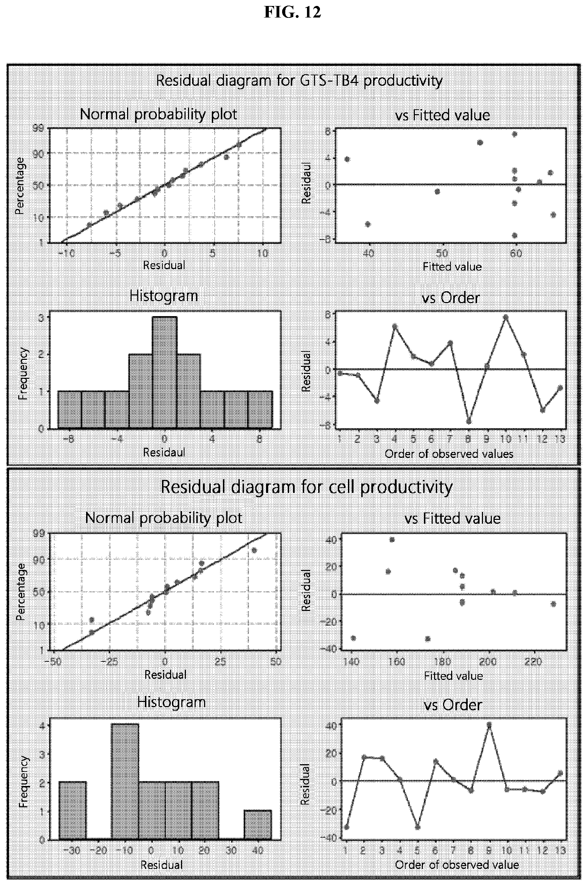

Referring to a result of a residual plot of the conducted DoE model results, generally, a constant variance was shown without specific tendency or singularity. In addition, the experiment was conducted independently and randomly according to time and an experimental order, and according to normal distribution, it was confirmed that a lack of fit was not shown (FIG. 12).

#### (2-4-2-3) Deduction of optimal conditions and determination of design space

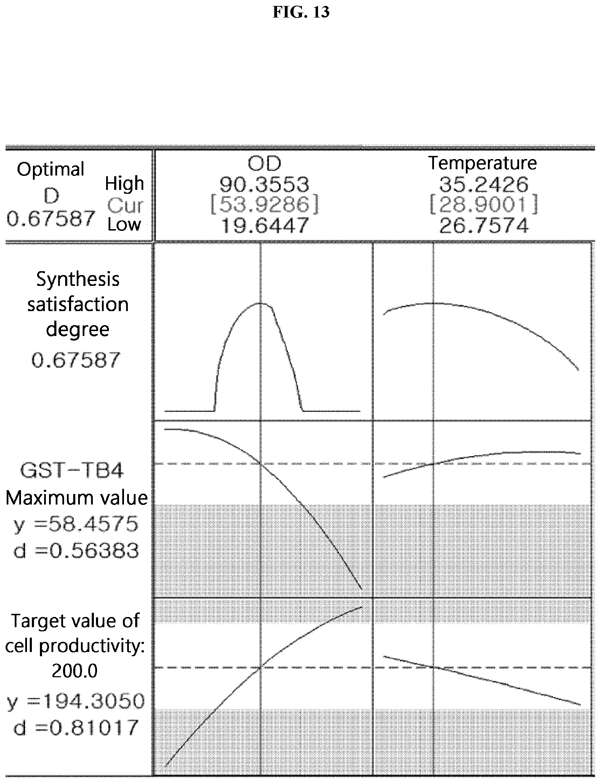

According to the ANOVA result of the culture experiment through DoE RSM design, optimal conditions were obtained using a response optimization tool of Minitab software. The optimal conditions presented at a level satisfying a synthesis satisfaction degree of 0.67 with respect to cell productivity and GST-Tβ4 productivity were an induction $OD_{600}$ of 53.9 and an induction temperature of 28.9 °C, and target response values were predicted to be 58.5 mg/g and 194 g/L, respectively (Table 28, FIG. 13). Since the suggested optimal conditions are close to center point conditions, the setting of factor levels for the initial DoE design was considered appropriate, and the lower limit of target productivity in the optimal process was set as the lowest point of the average of center points (Table 29).

[Table 28]

| Induction parameter | Optimal condition | Target (estimated) value of response value |
|---|---|---|
| Induction $OD_{600}$ | 53.9 | *GST-TB4 productivity: 58.5 mg/g pellet |
| Induction Temperature (°C) | 28.9 | *Cell productivity: 194 g pellet/L |

Optimization of Minitab response conditions

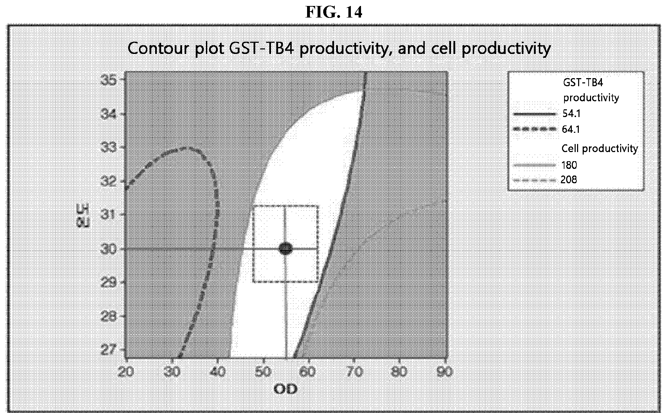

A contour plot plotted using the lowest point of the average center points (54.1 mg/g & 180 g/L) as the lower limit of the target standard, and the mean value of top five batches (64.1 mg/g & 208 g/L) as the upper limit of the target standard was confirmed (Table 29, FIG. 14). The white area of the contour plot indicates a design space satisfying both of GST-Tβ4 productivity and the target standard of cell productivity (≥54.1 mg/g, ≥180 g/L), and the final optimized process conditions deduced again based on the stable range of this space were determined to be an induction $OD_{600}$ of 54 ± 5 and an induction temperature of 30 ± 1 °C (Table 30).

[Table 29]

| Response value | Total mean | Top 5 batch mean | Mean of central points | Target criterion |
|---|---|---|---|---|
| Cell productivity (g/L) | 185 ± 31 | 208 ± 8 | 189 ± 9 | ≥ 180 |

(continued)

| Response value | Total mean | Top 5 batch mean | Mean of central points | Target criterion |
|---|---|---|---|---|
| GST-TB4 productivity (mg/g) | $56.4 \pm 10.0$ | $64.1 \pm 2.7$ | $59.8 \pm 5.7$ | $\geq 54.1$ |

Target standard of response values of optimal condition process for HU024 culture

[Table 30]

| Process factor | Optimal condition |
|---|---|
| Induction $OD_{600}$ | $54 \pm 5$ |
| Induction temperature | $30 \pm 1$ °C |

Optimal process conditions for HU024 culture

**(2-5) Confirmation of reproducibility of optimal culture conditions**

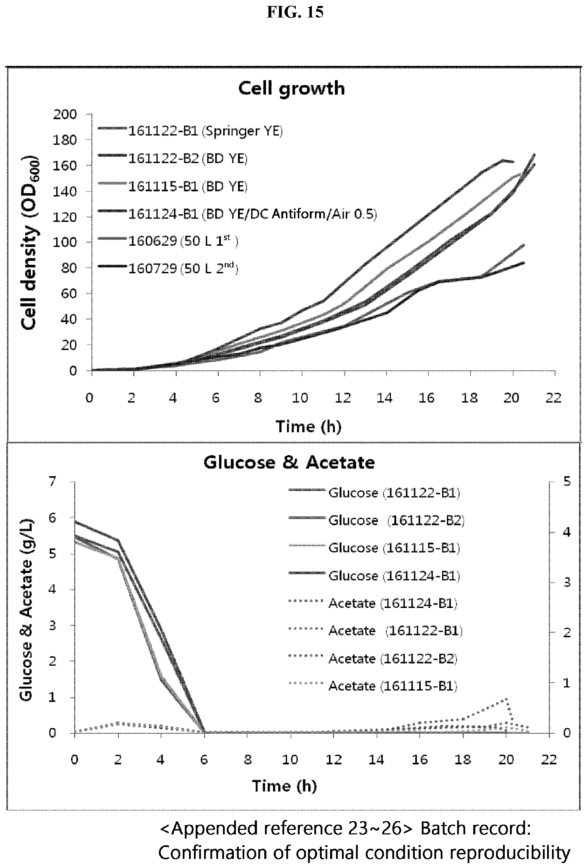

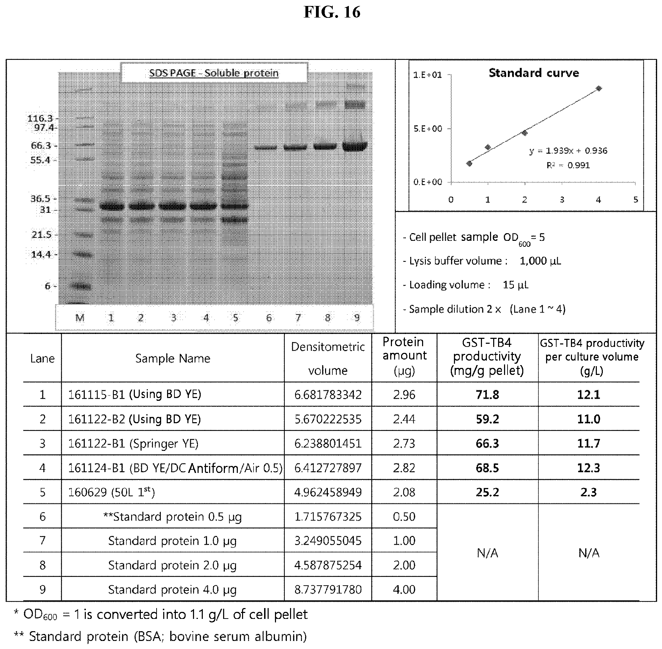

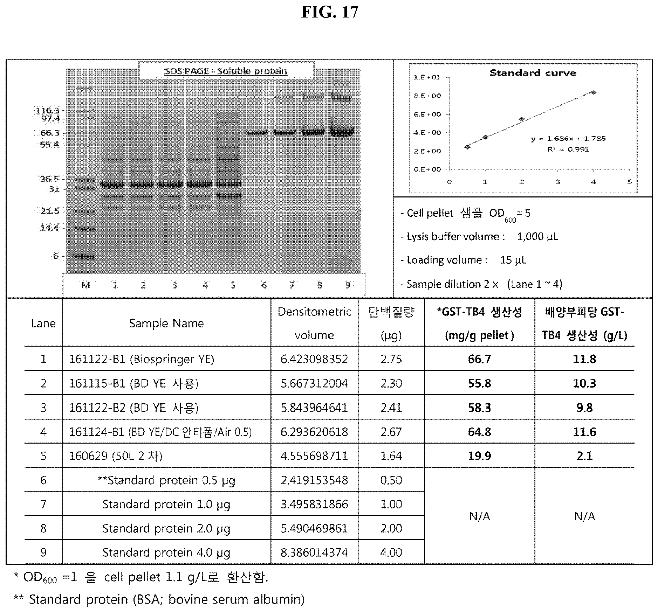

Culture for confirming response value reproducibility was performed with the final optimal conditions ($OD_{600}$ =54 $\pm$ 5, temperature: 30 $\pm$ 1°C) deduced from statistical analysis for the DoE RSM culture experiment results. A total of 4 batch culture processes were conducted (Table 17): 1 batch (Batch No. 161122-B1) using the same product as the YE product (Springer 0202) used in a DoE experiment; 2 batches (Batch No. 161115-B1, 161122-B2) using YE manufactured by BD; and 1 batch (161124-B1) with an antifoam product (Dow Corning) and a changed aeration condition (0.5 vvm). In the case of an antifoam manufactured by Dow Corning, the amount of antifoam used has increased more than 10 times compared to an existing antifoam (Sigma 204). However, all 4 batches showed similar growth, but the maximum cell density ($OD_{600}$) was 160 or more, and glucose and acetate concentrations were well controlled to 1 g/L or less (FIG. 15). Cell productivity was 182, 202, 208 or, 205 g/L (Table 32), the average of the two repeated analyses of GST-Tβ4 productivity was 66.5 $\pm$ 0.3, 57.5 $\pm$ 2.4, 65.1 $\pm$ 9.5 or 66.6 $\pm$ 2.6 mg/g (Table 31, FIGS. 16 and 17), and all of the 4 batches achieved the result of the target standard of optimal condition response values (Table 29) or more. As a result, the reproducibility of the 5 L scale optimal culture process condition established in this study was confirmed, and compared to the verification 50 L culture result that reproduced the NR process (Batch No. 160629, 160729), cell productivity was increased approximately 1.6-fold, and GST-Tβ4 productivity was increased approximately 2.8-fold (Table 32).

[Table 31]

| Batch name. | GST-TB4 productivity (mg/g pellet) | | | | GST-TB4 productivity per culture volume (g/L) | | |
|---|---|---|---|---|---|---|---|
| | Analysis <1> | Analysis <2> | Mean | | Analysis <1> | Analysis <2> | Mean |
| 161122-B1 (Springer YE*) | 66.3 | 66.7 | 66.5$\pm$0.3 | | 11.7 | 11.8 | 11.8$\pm$0.0 |
| 161122-B1 (using BD YE) | 71.8 | 58.3 | 65.1$\pm$9.5 | | 12.1 | 9.8 | 11.0$\pm$1.6 |
| 161122-B2 (using BD YE) | 59.2 | 55.8 | 57.5$\pm$2.4 | | 11.0 | 10.3 | 10.6$\pm$0.4 |
| 161124-B1 (BD YE/DC** antifoam/ Air0.5) | 68.5 | 64.8 | 66.6$\pm$2.6 | | 12.3 | 11.6 | 11.9$\pm$0.5 |
| 160629 (50L 1st, 2nd) | 25.2 | 19.9 | 22.5$\pm$3.7 | | 2.7 | 2.2 | 2.4$\pm$0.4 |

*YE: Yeast Extract
**DC: Dow Corning

Analysis result for GST-Tβ4 productivity under optimal conditions

[Table 32]

| Item | Optimal process condition deduced from DoE experiment | Confirmation of optimal condition reproducibility | | Change in antifoam, Aeration confirmation | Verification run 50 L 1st, 2nd |
|---|---|---|---|---|---|
| Batch No. | N/A | 161122-B1 | 161115-B1 161122-B2 | 161124-B1 | 160629 160729 |
| YE manufacturer[1] | Springer (0202) | Springer (0202) | BD | BD | Biospringer (0202) Biospringer (0251) Soy peptone |
| Antifoam manufacturer[2] | N/A | Antifoam 204 (amount used: 0.5~0.6 mL) | | Dow Corning (amount used: 10 mL) | Antifoam 204 (6 mL) |
| Aeration condition[3] | N/A | 1 vvm | | 0.5 vvm | 1 vvm |
| Induction $OD_{600}$ | 54 ± 5 | 53.8 | 52.4 51.3 | 54,4 | 69 70 |
| Induction Temperature (C) | 30 ± 1 | 30 | 30 | 30 | 32 |
| Cell productivity (g pellet/L) | ≥ 180 | 182 | 205 208 | 207 | 125 ± 4 |
| GST-TB4 productivity (mg/g pellet) | ≥ 54.1 | 66.5 ± 0.3 | 65.1 ±9.5 57.5 ± 2.4 | 66.6 ± 2.6 | 22.5 ± 3.7 |
| Kanamycin added in total culture broth[4] | O | X | X | X | O |

[1] Change of YE manufacturer: Springer product is food grade, BD product is bioproduct grade.
[2] Change of antifoam manufacturer: Sigma product is reagent grade, Dow Corning product is medical grade.
[3] Change of aeration condition: Reduced aeration volume to suppress excessive foam generation due to antifoam change.
[4] Elimination of kanamycin antibiotic-related regulatory issue.
Summary of results of confirming reproducibility of 5 L-scale optimal culture conditions

**Experimental Example (3) 50 L-scale optimal condition scale-up process (sample production)**

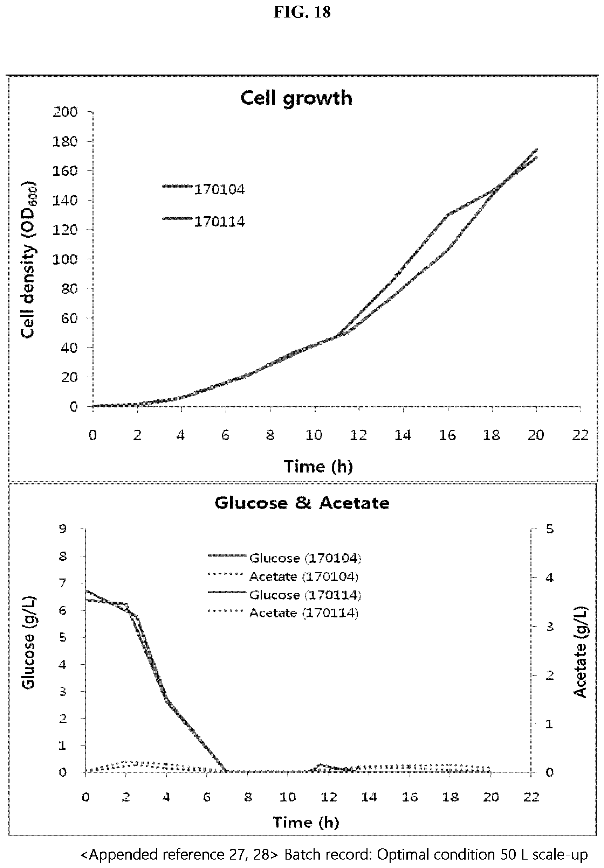

A 50 L culture process for sample production was conducted by scale-up of the optimal conditions for developing a 5 L-scale process. A feeding rate of a 50 L scale fed-batch culture was calculated by applying the same parameters as in the 5 L scale feeding strategy (Table 33). As a result of 2 batches (Batch No. 170104, 170114) of 50 L scale sample production culture, cell productivity was 207 and 208 g/L, GST-Tβ4 productivity was 76.9 and 69.7 mg/g (productivity per pellet), respectively (Table 34), the target standards of the optimal condition response values (Table 29, cell productivity ≥ 180 g/L & GST-Tβ4 productivity ≥ 54.1 mg/g) were satisfied, and growth and metabolism (glucose & acetate) trends also showed the same patterns as a 5 L-scale optimal condition-reproducible batch (FIG. 18). In the case of the batch (batch No. 170114) conducted as a sample production process, from the end of culture to the recovery of a culture broth, the temperature was cooled down to 10 °C in an incubator, followed by conducting an overnight (hereinafter, "O/N") holding test. The $OD_{600}$ values before and after O/N were 174.6 (before) and 171.5 (after), and the pellet productivity was measured to be 221 g/L (before) and 208 g/L (after), respectively (Table 34). Results for the sample production (batch No. 170114) are summarized in Table 35.

[Table 33]

| t | X | V | F(g/min) | μ |
|---|---|---|---|---|
| 0~4 | | Batch culture | | |
| 4 | 8 | 2.50 | **2.3** | 0.3 |
| 5 | 11 | 2.51 | **3.1** | 0.3 |
| 6 | 14 | 2.53 | **3.8** | 0.3 |
| 7 | 19 | 2.55 | **4.1** | 0.2 |
| 8 | 23 | 2.57 | **4.3** | 0.2 |
| 9 | 28 | 2.59 | **4.7** | 0.18 |
| 10 | 34 | 2.62 | **5.6** | 0.18 |
| 11 | 40 | 2.65 | **6.7** | 0.18 |
| 12 | 47 | 2.69 | **8.1** | 0.18 |
| 13 | 55 | 2.73 | **8.5** | 0.16 |
| 14 | 64 | 2.78 | **10.0** | 0.16 |
| 15 | 73 | 2.83 | **11.7** | 0.16 |
| 16 | 84 | 2.90 | **11.9** | 0.14 |
| 17 | 95 | 2.96 | **13.7** | 0.14 |
| 18 | 106 | 3.04 | **15.7** | 0.14 |
| 19 | 119 | 3.13 | **18.1** | 0.14 |
| 20 | 132 | 3.23 | **20.8** | 0.14 |

Exponential feeding rate in 50 L scale-up process

[Table 34]

| Batch No. | Induction Start (OD$_{600}$) | Induction temperature (°C) | Final OD$_{600}$ | Cell productivity (g/L) | GST-TB4 productivity (mg/g) |
|---|---|---|---|---|---|
| 170104 | 55.4 | 30 | 169.2 | 207 | 76.9 |
| 170114 | 50.4 | 30 | 174.6 (End of culture) 171.5 (10 °C O/N) | 221 (End of culture) 208 (10 °C O/N) | 69.7 |

Summary of results for 50 L optimal condition scale-up culture

[Table 35]

| Process step | | Result |
|---|---|---|
| Seed culture 1 | Culture time | 5 h |
| | Final cell concentration | OD$_{600}$ = 2.1 |
| Seed culture 2 | Culture time | 3 h |
| | Final cell concentration | OD$_{600}$ = 2.4, 2.5, 2.6, 2.6 |
| Main culture | Feed-A start | OD$_{600}$ = 6.2 (4 h) |
| | Induction start | OD$_{600}$ = 50.4 (11.5 h) |
| | Culture end | OD$_{600}$ = 174.6 (20 h) |
| | 15 °C overnight | OD$_{600}$ = 171.5 (39 h) |
| | Volume of final culture broth | 36.0 kg (20.8% of solid content) |

(continued)

| Process step | | Result |
|---|---|---|
| Continuous centrifugation (recovery of culture broth) | 1st recovery and washing with DIW | 48.7 kg (13.8% of solid content) |
| | 2nd recovery and washing with DIW | 52.7 kg (11.6% of solid content) |
| | 3rd recovery (final cell slurry) | 13.6 kg (36.2% of solid content) |
| Cell lysis | Lysis buffer suspension | -Cell slurry: 13.6 kg -20X lysis buffer: 1.63 kg -DIW: 17.4 kg -Total weight: 32.6 kg |
| | Measurement of 1st lysis $OD_{600}$ | 107.0 (before) / 13.2 (after) |
| | Measurement of 2nd lysis $OD_{600}$ | 13.2 (before) / 8.1 (after) |
| | Lysis rate | 7.5% |
| | Cell lysate | 32.7 kg |
| Continuous centrifugation (Recovery of supernatant of cell lysate) | | 24.8 kg |
| Filter washing (DIW) | C0HC washing | 22.0 L |
| | F0HC washing | 22.0 L |
| | Opticap XL 10 washing | 6.9 L |
| Depth filtration | Cell lysate supernatant (input) | 24.8 kg |
| | Flushing (DIW) | 11.6 kg |
| | Final recovery after membrane filter | 34.2 kg |
| Summary of results for 50 L sample production (batch No. 170114) | | |

**[2. Purification process]**

**Example (1) Background and preliminary experiment**

The Chinese process conducted with Q FF - GST FF - enzyme digestion - UF/DF - Q XL - Source 30RPC - Q HP - UF/DF was compared with a more optimized Huons process conducted with Q FF - GST FF - enzyme digestion - Q FF - UF/DF. Accordingly, based on the Huons process, experiments were conducted with the aim of improving purity, optimizing a unit process and minimizing a high cost of GST FF resins.

**(1-1) Resin screening of primary column**

To maximize a DBC for expensive GST columns, instead of a Q FF resin that had been used as a primary column in the Chinese process, Nuvia Q, Factrogel TMAE(M) was applied to confirm an effect of improving a DBC for a GST resin by the improvement in purity of an eluted fraction. When an elution pool purified using two types of resins was applied to a GST column, the DBC was approximately 42 to 44 mg/mL regardless of resin type (Tables 36 and 37). Therefore, it was decided to use Fractogel TMAE(M), which has a relatively high yield, in process development.

[Table 36]

| | Loading Sample | | | Fluted Pool | | | Yield (%) |
|---|---|---|---|---|---|---|---|
| | Cone. (mg/mL) | Volume (mL) | Amount (mg) | Cone. (mg/mL) | Volume (mL) | Amount (mg) | |
| AEX (Nuvia Q)* | 0.98 | 50 | 48.9 | 0.53 | 77 | 41.0 | **83.8** |

(continued)

| | Loading Sample | | | Fluted Pool | | | Yield (%) |
|---|---|---|---|---|---|---|---|
| | Conc. (mg/mL) | Volume (mL) | Amount (mg) | Conc. (mg/mL) | Volume (mL) | Amount (mg) | |
| Affinity** | 0.53 | 28.3 | 15 | 0.54 | 7.7 | **4.2** | |

*CV of Nuvia Q: 6.8 mL, Loading amount: 7.2 mg/mL of resin
**Affinity CV: 1 mL columns were used
Step yield for Nuvia Q resin and DBC for Affinity resin using Nuvia Q eluted sample

[Table 37]

| | Loading Sample | | | Eluted Pool | | | Yield (%) |
|---|---|---|---|---|---|---|---|
| | Conc. (mg/mL) | Volume (mL) | Amount (mg) | Conc. (mg/mL) | Volume (mL) | Amount (mg) | |
| AEX (TMAE (M))* | 1.17 | 50 | 58.6 | 0.66 | 87.3 | 57.5 | **98.1** |
| Affinity** | 0.66 | 23 | 15 | 0.44 | 10.0 | **4.4** | |

*CV of TMAE (M): 8.6 mL, loading amount: 6.8 mg/mL of resin
**Affinity CV: 1 mL columns were used
Step yield for TMAE(M) resin and DBC for Affinity resin using TMAE(M) eluted sample

## (1-2) 50L Verification Run Test

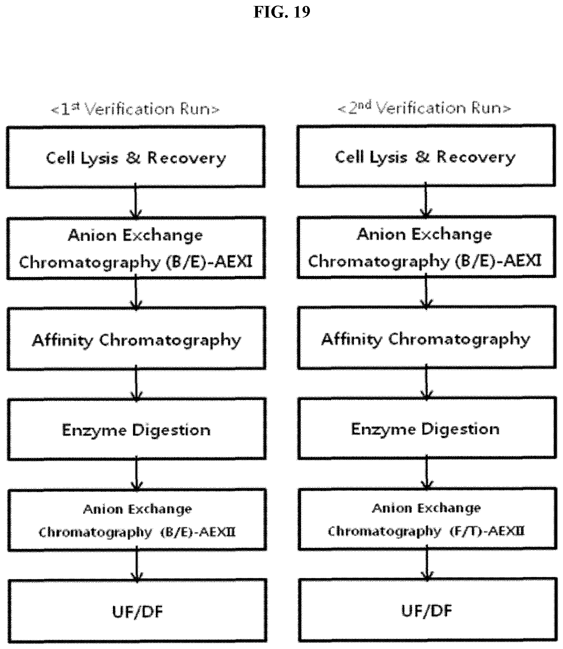

Based on the Huons process, the reproducibility of a small scale process was confirmed, and both of a 1st verification run and a 2nd verification run were carried out with a 50 L scale culture broth (FIG. 19, Tables 38 and 39).

[Table 38]

| Items | | Process conditions |
|---|---|---|
| Cell Lysis & Recovery Depth filtration | | Cell pellet 1366g, 1419 g (Batch No. 160629-50L) Microfluidizer: 15,000 psi, 3 pass centrifugation (8500 rpm, 50 min, 10 °C) |
| | | C0HC (0.11 m$^2$)x2ea + F0HC (0,11 m$^2$)x2ea + Opticap XL10 (0.45 $\mu$m) |
| | Resin | Fractogel EMD TMAE(M) |
| | Column scale | BPG 200 (i.d 20 cm, H 13 cm), CV 4145 mL |
| | Equilibration buffer | 20 mM Tris-HCl, pH 8.0 |
| | Elution buffer | 20 mM Tris-HCl, 500mM NaCl, pH 8.0 |
| | Linear flow rate | 61 cm/hr (Flow rate 319 mL/min) |

(continued)

| Items | | Process conditions |
|---|---|---|
| AEXI | Loading sample condition | pH 8.0 $\pm$ 0.1 Conductivity < 2.8 mS/cm |
| | Elution pooling criteria | Start point: UV$_{280nm}$ Value $\geq$ 100 mAU<br>End point: UV$_{280nm}$ Value $\leq$ 100 mAU |
| | Process | Equilibration buffer (2 CV)<br>Sample Loading<br>Unbound wash (with EQ buffer) (5 CV)<br>Elution buffer (5 CV)<br>2 M NaCl (4 CV)<br>0.5 M NaOH (5 CV)<br>2 M NaCl (3 CV)<br>Equilibration buffer (3 CV) |
| Affinity | Resin | Glutathione Sepharose 4 Fast Flow |
| | Column scale | BPG 200 (i.d 20cm, h 11cm), CV 3454mL |
| | Equilibration buffer | 20 mM Tris-HCl, 150 mM NaCl, pH 8.0 |
| | Elution buffer | 50 mM Tris-HCl, 10 mM Glutathione (Reduced), pH 8.0 |
| | Acidic buffer | 100 mM Sodium Acetate, 500 mM NaCl, pH 4.5 |
| | Alkaline buffer | 100 mM Tris-HCl, 500 mM NaCl, pH 8.0 |
| | Linear flow rate | 63 cm/hr (Flow rate 329 mL/min) |
| | Loading sample condition | Directly Loading |
| | Elution pooling criteria | Start point : UV$_{280nm}$ Value $\geq$ 100 mAU<br>End point : UV$_{280nm}$ Value $\leq$ 100 mAU |
| | Process | Equilibration buffer (2 CV)<br>Sample Loading<br>Unbound wash (with EQ buffer) (5 CV)<br>Elution buffer (5 CV)<br>Acidic buffer (5 CV)<br>Alkaline buffer (5 CV)<br>6M Guanidine hydrochloride (2 CV)<br>DW (2 CV)<br>Equilibration buffer (3 CV) |
| Thrombin Reaction & AEXII | Enzyme | Bovine Thrombin |
| | Enzyme ratio | 1 mg GST-T$\beta$4 per 2 unit thrombin |
| | Reaction time | 18 $\pm$ 2 H |
| | Reaction temp | 22 $\pm$ 2 °C |
| | Agitation | 60 rpm |
| | Resin | Fractogel EMA TMAE(M) |
| | Column scale | BPG 100 (i.d 10 cm, H 14.2 cm), CV 1115 mL |
| | Equilibration buffer | 20 mM Tris-HCl, pH 8.0 |
| | Elution buffer | 20 mM Tris-HCl, 100 mM NaCl, pH 8.0 |
| | Linear flow rate | 170 cm/hr (Flow rate 223 mL/min) |
| | Loading sample condition | pH 8.0 $\pm$ 0.1 Conductivity < 2.8 mS/cm |

(continued)

| Items | | Process conditions |
|---|---|---|
| | Elution pooling criteria | Start point : $UV_{214nm}$ Value $\geq$ 50 mAU<br>End point : $UV_{214nm}$ Value $\leq$ 50 mAU |
| | Process | Equilibration buffer (2 CV)<br>Sample Loading<br>Unbound wash (with EQ buffer) (5 CV)<br>Elution buffer (5 CV)<br>2 M NaCl (4 CV)<br>0.5 M NaOH (5 CV)<br>2 M NaCl (3 CV)<br>Equilibration buffer (3 CV) |
| Conditions for 1st 50L verification run process | | |

The 1st verification run was conducted in the same manner as used in the small scale process, and the results are shown as follows (Tables 39 and 40).

[Table 39]

| | | Loading Sample | | | Eluted Pool | | | Yield (%) | Qunatitation |
|---|---|---|---|---|---|---|---|---|---|
| | | Conc. (mg/mL) | Volume (mL) | Amount (mg) | Cone. (mg/mL) | Volume (mL) | Amount (mg) | | |
| Cell breakage (160602_50) | | | | | | 1144 | | | |
| Depth Filtration | | | 1,144 | | | 1326 | | | Densitometer |
| Microfiltration | | | 1030 | | | 966.5 | | | |
| AEXI | Preparation of LS | 2.45 | 200.0 | 489.8 | 1.662 | 309.0 | 513.6 | 104.9% | Densitometer |
| | Process | L66 | 257.0 | 427.1 | 124 | 385.0 | 475.5 | 111.3% | Densitometer |
| Gluathione S4FF | | 124 | 175.6 | 216.9 | 1.99 | 81.9 | 163.1 | 75.2% | Densitometer |
| Thrombin | | 139 | 79.0 | 157.4 | 0.28 | 91.0 | 25.5 | 16.2% | UV/SEC |
| AEX II | Preparation of LS | 0.28 | 89.0 | 24.9 | 0.08 | 254.0 | 20.8 | 83.6% | SEC |
| | Process | 0.08 | 251.0 | 20.6 | 032 | 21.0 | 7 | 32.6% | SEC |
| UF/DF | | 032 | 18.0 | 5.8 | 9.90 | 0.5 | 5 | 85.9% | SEC/RP |
| Total Yield (%) | | | | | | | | 3.3% | |

Step Yield of HU024 Small Scale verification run

[Table 40]

| | | Loading Sample | | | Eluted Pool | | | Yield (%) | Qunatitation |
|---|---|---|---|---|---|---|---|---|---|
| | | Conc. (mg/mL) | Volume (mL) | Amount (mg) | Cone. (mg/mL) | Volume (mL) | Amount (mg) | | |
| Cell breakage * | | | | | | 11,065 | | | |
| Depth Filtradon&MF* | | | 11,605 | 43,968 | | 27,795 | 37,815 | | Densitometer |
| AEXI* | | 136 | 27,358 | 37,308 | 2.14 | 16,981 | 36.322 | 96.1% | Densitometer |
| Gluathione S4FF** | | 2.14 | 16,981 | 36,322 | 3.21 | 6,845 | 21,972 | 60.5% | Densitometer |
| Thrombin | | 321 | 6,845 | 21,972 | 0.48 | 7,850 | 3,768 | 17.1% | UV/SEC |
| AEX II (1.1L CV) | Flow through | 0.09 | 41,060 | 3,695 | 0.06 | 33,494 | 1,909 | 51.7% | RP |
| | Elution | | | | 1.19 | 955 | 1,140 | 30.9% | RP |
| UF/DF*** | | 0.06 | 33,494 | 1,909 | 9.95 | 188 | 1,866 | 97.7% | RP |
| Total Yield (%) | | | | | | | | 4.3% | |

*Recovery & AEXI were the results obtained by 2 cycles
** Affinity Process was the result obtained by 3 cycles
*** Resulted from flow through fraction only
Step yield of 1st HU024 verification run

EP 3 835 313 A1

Referring to the small scale purification results and the 1st verification run results, many target proteins were confirmed from flow through (F/T) fractions in an AEXII process (AEX process after thrombin digestion) (Table 40). Accordingly, in the 2nd verification, the process was conducted in a flow through mode (Table 41).

[Table 41]

| Items | | Process conditions |
|---|---|---|
| Cell Lysis & Recovery Depth filtration | | Cell pellet 1500 g, 1530 g (Batch No. 160729.-50L)<br>Microfluidizer: 15,000 psi, 3 pass<br>Tubular continuous centrifugation (15000 rpm, feed rate 700 mL/min) |
| | | C0HC (0.11 m$^2$)x2ea + F0HC (0,11 m$^2$)x2ea + Opticap XL10 (0.45 $\mu$m) |
| AEXI | Column scale | BPG 200 (i.d 20 cm, H 13 cm), CV 4145 mL |
| | Equilibration buffer | 20 mM Tris-HCL pH 8.0 |
| | Elution buffer | 20 mM Tris-HCl, 500 mM NaCl, pH 8.0 |
| | Linear flow rate | 61 cm/hr (Flow rate 319 mL/min) |
| | Loading sample condition | pH 8.0 $\pm$ 0.1 Conductivity < 2.8 mS/cm |
| | Elution pooling criteria | Start point: UV$_{280nm}$ Value $\geq$ 100 mAU<br>End point: UV$_{280nm}$ Value $\leq$ 100 mAU |
| | Process | Equilibration buffer (2 CV)<br>Sample Loading<br>Unbound wash (with EQ buffer) (5 CV)<br>Elution buffer (5 CV)<br>2 M NaCl (4 CV)<br>0.5 M NaOH (5 CV)<br>2 M NaCl (3 CV)<br>Equilibration buffer (3 CV) |
| Affinity | Column Scale | BPG 200 (i.d 20 cm, h 11 cm), CV 3454 mL |
| | Equilibration buffer | 20 mM Tris-HCl, 150 mM NaCl, pH 8.0 |
| | Elution buffer | 50 mM Tris-HCl, 10 mM Glutathione (Reduced), pH 8.0 |
| | Acidic buffer | 100 mM Sodium Acetate, 500 mM NaCl, pH 4.5 |
| | Alkaline buffer | 100 mM Tris-HCl, 500 mM NaCl, pH 8.0 |
| | Linear flow rate | 63 cm/hr (Flow rate 329 mL/min) |
| | Loading sample condition | Direct Loading |
| | Elution pooling criteria | Start point: UV$_{280nm}$ Value $\geq$ 100 mAU<br>End point: UV$_{280nm}$ Value $\leq$ 100 mAU |
| | Process | Equilibration buffer (2 CV)<br>Sample Loading<br>Unbound wash (with EQ buffer) (5 CV)<br>Elution buffer (5 CV)<br>Acidic buffer (5 CV)<br>Alkaline buffer (5 CV)<br>6 M Guanidine hydrochloride (2 CV)<br>DW (2CV)<br>Equilibration buffer (3CV) |
| | Enzyme | Bovine Thrombin |

(continued)

| Items | | Process conditions |
|---|---|---|
| Thrombin Reaction & AEXII | Enzyme ratio | 1 mg GST-Tβ4 per 2 unit thrombin |
| | Reaction time | 18 ± 2 H |
| | Reaction temp | 22 ± 2 °C |
| | Agitation | 60 rpm |
| | Column scale | BPG 100 (i.d 10 cm, H 14.2 cm), CV 1115 mL |
| | Equilibration buffer | 20mM Tris-HCl, 50 mM NaCl, pH 8.0 |
| | Linear flow rate | 170 cm/hr (Flow rate 223 mL/min) |
| | Loading sample condition | pH 8.0 ± 0.1<br>Conductivity < 2.8 mS/cm |
| | Elution pooling criteria | Start point: $UV_{214nm}$ Value ≥ 50 mAU<br>End point: $UV_{214nm}$ Value ≤ 50 mAU |
| | Process | Equilibration buffer (2 CV)<br>Sample Loading<br>Unbound wash (with EQ buffer) (5 CV)<br>Elution buffer (5 CV)<br>2 M NaCl (4 CV)<br>0.5 M NaOH (5 CV)<br>2 M NaCl (3 CV)<br>Equilibration buffer (3 CV) |

*Each process resin was the same as used In 1st verification run

Conditions for 2nd 50L verification run process

[Table 42]

| | Loading Sample | | | Eluted Pool | | | Yield (%) | Qunatitation |
|---|---|---|---|---|---|---|---|---|
| | Conc. (mg/mL) | Volume (mL) | Amount (mg) | Cone. (mg/mL) | Volume (mL) | Amount (mg) | | |
| Cell breakage* | | | | | 14,036 | | | |
| Depth Filtration&MF* | | 14,036 | 52,159 | | 42,720 | 45,196 | | Densitometer |
| AEXI* | 1.06 | 42,202 | 44,644 | 1.50 | 27,330 | 40,995 | 91.8% | Densitometer |
| Glutathione S4FF** | 1.50 | 25,866 | 39,699 | 3.83 | 6,919 | 26,500 | 66.8% | Densitometer |
| Thrombin | 3.83 | 6,901 | 26,431 | 0.55 | 7,907 | 4,349 | 16.5% | UV/SEC |
| AEX II | 0.37 | 12,049 | 4,349 | 0.24 | 16,672 | 4,018 | 92.4% | SEC |
| UF/DF*** | 0.24 | 16,672 | 4.018 | 9.87 | 329 | 3,250 | 80.9% | RP |
| Total Yield (%) | | | | | | | 7.6% | |

*Recovery & AEXI were the results obtained by 2 cycles ** Affinity Process was the result obtained by 3 cycles ***UF/DF were the UF/DF value obtained by reprocessing twice due to endotoxin

Step yield of 2nd HU024 verification run

In the 1st verification run, the purities of the F/T fractions in the AEXII process were confirmed to be 99.4 % (RP-HPLC) and 97.9 % (SE-HPLC), whereas the purities of eluted fractions were confirmed to be non-standard (Table 43). In the case of the 2nd Verification run, when the AEXII process was conducted in a flow through mode, a purification yield was improved (4.3 --> 7.6 %), but the purity was slightly reduced (97.9 --> 97.0 %), compared to the 1st verification (Table 44).

[Table 43]

| Sample | | HCP | | | Endotoxin (EU/mg) | Purity (%) (SEC-HPLC) | Purity (%) (RP-HPLC) |
|---|---|---|---|---|---|---|---|
| | | Conc. (mg/mL) | HCP Conc. (ng/mL) | HCP (ppm) | | | |
| AEXI | | 2.14 | 10,193 | 4,793 | 34,963 | | |
| Gluathione S4FF** | | 3.21 | 1,041 | 324 | 3,121 | | |
| Thrombin | | 0.48 | 982 | 2.045 | 29,042 | | |
| AEX II | Flow through | 0.06 | - | - | 0.08 | | |
| | Elution | 1.19 | 143 | 120 | 0.21 | | |
| UF/DF | Flow through | 9.97 | 217 | 22 | 0.76 | **97.9** | **99.4** |
| | Elution | 7.93 | 1178 | 149 | 1.49 | **93.4** | **93.4** |

Quality profile for 1st verification run

[Table 44]

| Sample | HCP | | | Endotoxin (EU/mg) | Purity (%) (SEC-HPLC) | Purity (%) (RP-HPLC) |
|---|---|---|---|---|---|---|
| | Cone. (mg/mL) | HCP Conc. (ng/mL) | HCP (ppm) | | | |
| AEXI | 1.50 | 21,690 | 14,460 | 98,973 | | |
| Gluathione S4FF** | 3.83 | 1,252 | 327 | 1,120 | | |
| Thrombin | 0.55 | 1,250 | 2,273 | 6,411 | | |
| AEX II | 0.24 | 14 | 58 | 0.02 | | |
| UF/DF | 11.5 | 560 | 49 | 2.58 | **96.9** | **97.1** |
| UF/DF (Reprocessing) | 9.87 | - | - | 0.35 | **97.0** | **98.7** |

*reprocessing was the sample to which 2 cycles of AEX(F/T) --->AEX(B/E) was applied
Quality profile for 2nd verification run

However, in the two verification runs, the SE-HPLC purity of the final purified extract was less than 98%. Therefore, the process development was planned to improve purity to be 98% or more, in addition to the optimization of the unit process.

## (1-3) Optimization of cell disruption conditions

For the unit process optimization, it was checked whether to improve cell disruption efficiency.

## (1-3-1) Experiment of improving lysis buffer

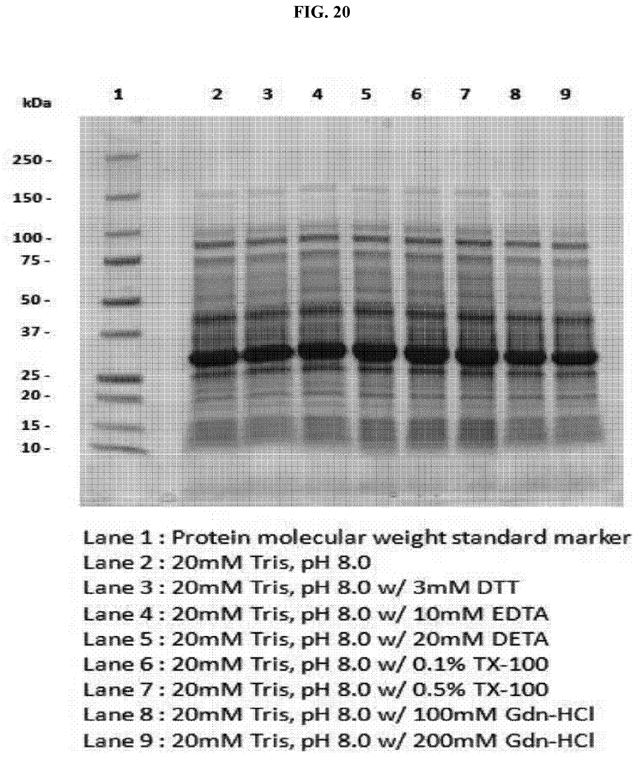

The possibility of improving cell disruption efficiency was confirmed. When EDTA and Triton X-100 as a detergent were added, the increase in recovered target protein content was insignificant (approximately 4 to 6% increase), so that it was determined to stick to the previous conditions (FIG. 20 and Table 45).

[Table 45]

| Conditions | Control | 3 mM DTT | **10 mM EDTA** | 20 mM EDTA |
|---|---|---|---|---|
| Band Vol (OD*mm$^2$) | 21.7 | 21.1 | 23.3 | 22.3 |
| Percentage (%) | 100.0 | 97.0 | **106.7** | 103.5 |
| Conditions | 0.1 % TX-100 | **0.5 % TX-100** | 0.1 M Guanidine-HCl | 0.2 M Guanidine-HCl |
| Band Vol (OD*mm$^2$) | 22.1 | 22.3 | 16.7 | 15.9 |
| Percentage (%) | 102.7 | **104.1** | 78.4 | 74.8 |
| *Basic buffer composition: 20mM Tris-HCl, pH 8.0<br>Conditions for comparison GST-Tβ4 band density by cell disruption buffer | | | | |

### (1-3-2) Impurity removal experiment

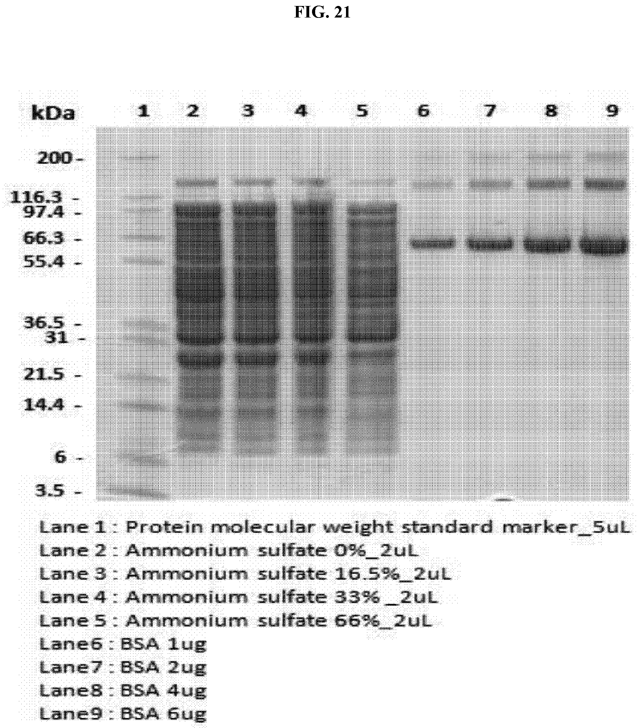

According to NR (Chinese process), the possibility of removing impurities by utilizing salting-out caused by the addition of ammonium sulfate was checked. In the NR process, a process of adding 5.6 g (33%) of ammonium sulfate per 100 mL of a lysate solution was applied, whereas in this experiment, a GST-Tβ4 amount in a lysate solution treated with 16.5%, 33% or 66% of ammonium sulfate was confirmed. As the ammonium sulfate content increased, the GST-Tβ4 content was decreased by 20%, 37% or 43%, compared to a sample without ammonium sulfate (FIG. 21 and Table 46). When the ammonium sulfate was used, the GST-Tβ4 contents were also reduced, so that it was determined not to add ammonium sulfate.

[Table 46]

| Condition | GST-Tβ4 Amount (ug)* | Relative rate of target Loss |
|---|---|---|
| 0 % Ammonium sulfate | 2.4 | 0% |
| 16.5 % Ammonium sulfate | 1.9 | 20 % |
| 33.0 % Ammonium sulfate | 1.5 | 37 % |
| 66.0 % Ammonium sulfate | 1.4 | 43 % |

\* Quantitation by Densitometer
Relative reduction of GST-Tβ4 amount by adding ammonium sulfate

### (1-3-3) Changes of disruption conditions

Microfluidizer conditions were changed. The cell disruption conditions used in two verification runs (30%cell slurry, a pressure of 15,000 psi, and disruption repeated three times) were changed into the cell disruption conditions currently applied in a Binex GMP facility (15% cell slurry, a pressure of 11,000 psi, and disruption repeated two times). When the changed disruption conditions were applied, a degree of reducing optical density (OD$_{600}$) of a sample after disruption was equal to or higher than that of the conventional method (Table 47). The changed conditions were applied from the development of a purification process using a 5 L scale culture broth.

[Table 47]

| Step | Cell disruption condition | | | |
|---|---|---|---|---|
| | 15,000psi, 3pass, 30% slurry | | 11,000psi, 2pass. 15% slurry | |
| | 50L 1st Run | 50L 2nd Run | Depth test | 50L 3rd Run |
| Cell slurry (OD$_{600}$) | 148 | 141.5 | 95.6 | 107 |
| 1st Disruption (OD$_{600}$) | 62.7 | 47.6 | 17.2 | 13.2 |
| 2nd Disruption (OD$_{600}$) | 35.2 | 24.6 | 5.6 | 8.1 |
| 3rd Disruption (OD$_{600}$) | 22.9 | 13.5 | N/A | N/A |

(continued)

| Step | Cell disruption condition | | | |
|---|---|---|---|---|
| | 15,000psi, 3pass, 30% slurry | | 11,000psi, 2pass. 15% slurry | |
| | 50L 1st Run | 50L 2nd Run | Depth test | 50L 3rd Run |
| End OD/initial OD(%) | 15.4 | 9.5 | 5.8 | 7.5 |

Cell optical density according to different conditions of disruption

### (1-3-4) Depth filtration sizing test

According to the first result of a filter test provided by Merck Millipore, C0HC and F0HC were selected, and applied to verification runs. Filter clogging occurred during a process, and the cause thereof was not analyzed yet. Afterward, the same filter was used in 5L and 50L processes, but clogging was not found. Therefore, it was confirmed that clogging occurred regardless of filter type, and clogging was expected to be resolved by changing disruption conditions.

After changing the disruption conditions, a second filter test was requested at Merck Millipore, and X0HC was suggested. A filter test was requested at Satorius, and thus Sartoclear PC2 was selected (Table 48). As a result, a depth filter manufactured by Merck Millipore was used, and a 5L process and a 3rd 50L verification run were conducted with C0HC and F0HC (Merck report Revision #2 <2016.06.17>, Merck report revision #1 <2016.10.20>, Sartorius report <Doc No. SKB201604>).

[Table 48]

| Company | Depth filter type | Nominal retention $\mu$m | Sterile filter | Pore size($\mu$m) |
|---|---|---|---|---|
| Merck Millipore | X0HC | 0.1 | Durapore | 0.45 |
| Sartorius | PC2 | 0.3 | Sartopore2 | 0.45, 02 (Double layer) |

Information on depth filter and sterile filter recommended by each vendor

### Example (2) Analysis conditions for each process sample

### (2-1) UV quantification method

Protein quantification was performed by the following equation (Equation 1), and used for protein quantification of a process sample for an affinity process.

### [Equation 1]

$$\text{Protein Concnetration (mg/mL)} = \frac{\text{Abs (280nm)}}{1.385}$$

### (2-2) SEC-HPLC analysis

Analysis was conducted on a process sample after thrombin digestion, and SEC-HPLC analysis for the process sample was conducted according to a sample size-exclusion chromatography (SEC-HPLC) quantification test and a Gly-TB4 SEC-HPLC purity test (according to Standard Operating Procedure (SOP) for pharmaceuticals) revised on Nov. 16, 2016) enabling purity measurement in a Gly-TB4 process enabling quantitative measurement of Gly-TB4 using conditions enabling GST and Gly-TB4 separation. The conditions are as follows.

[Table 49]

| Items | Conditions |
|---|---|
| Column | TSKgel G2000swxl (7.8 x 300 mm, 5μm particle) TSKgel Guard column (6.0 x 40 mm) |
| Flow rate | 0.6 mL/min |
| Injection volume | 20 μL |
| Mobile phase | 50 mM Sodium phosphate, 0.3 M NaCl, 10% ACN, pH 7.2 ± 0.1 |
| Column temperature | 30 ± 5 °C |
| Autosampler temperature | 5 ± 5 °C |
| Wavelength | 214 nm |
| Running time | 30 min |
| Sample preparation | Dilution with formulation buffer (Formulation Buffer: 20 mM Sodium phosphate, 150 mM NaCl, pH 8.0) |

SEC-HPLC Analysis Method and Conditions

### (2-3) RP-HPLC analysis

Analysis was conducted on a process sample after thrombin digestion, and RP-HPLC analysis for the process sample was conducted according to a Gly-TB4 RP-HPLC quantification test enabling quantification measurement and a Gly-TB4 RP-HPLC purity test enabling purity measurement (according to SOP for pharmaceuticals revised on Nov. 16, 2016) using conditions enabling Gly-TB4 and impurity separation. The conditions are as follows. In addition, the purities of purified extracts obtained from 1st and 2nd verification runs that had been conducted before SOP revision were considered to be unreliable measurement values.

[Table 50]

| Items | Conditions | | |
|---|---|---|---|
| SOP ver. | 1.0 | | 2.0 |
| Column | Agilent zorbax 300SB C18 (4.6 x 250 mm, 5 μm particle)<br>Agilent 300SB C18 guard column (4.6 x 12.5 mm) | | |
| Flow rate | 0.5 mL/min | | 0.8 mL/min |
| Injection volume | 20 μL | | |
| Mobile phase | A : Water (w/ 0.1 % TFA)<br>B : ACN (w/ 0.1 % TFA) | | A : Water (w/ 0.1 % TFA)<br>B : 80 % ACN (w/ 0.085 % TFA) |
| Column temperature | 30 ± 5 °C | | 45 ± 2 °C |
| Autosampler temperature | 5 ± 2 °C | | |
| Wavelength | 220 nm | | |
| Running time | 45 min | | 45min |
| Gradient method | | | |

| Time(min) | A (%) | B (%) |
|---|---|---|
| 0 | 85 | 15 |
| 5 | 85 | 15 |
| 25 | 50 | 50 |
| 25.1 | 10 | 90 |
| 30 | 10 | 90 |
| 30.1 | 85 | 15 |
| 45 | 85 | 15 |

| Time(min) | A (%) | B (%) |
|---|---|---|
| 0 | 91 | 9 |
| 5 | 91 | 9 |
| 30 | 64 | 36 |
| 31 | 10 | 90 |
| 35 | 10 | 90 |
| 36 | 91 | 9 |
| 45 | 91 | 9 |

| Sample preparation | Dilution with formulation buffer |
|---|---|

Method and Conditions for RP-HPLC analysis

#### (2-4) Endotoxin analysis

Endotoxin quantification was carried out according to the endotoxin test method using PTS of Gly-TB4 developed by Binex, thereby identifying endotoxin in the process sample.

#### (2-5) Host cell protein (HCP) analysis

HCP quantification was carried out according to the HCP analysis of Gly-TB4 developed by Binex, thereby identifying the tendency of removing HCPs from the process sample.

#### (2-6) Host cell DNA (HCD) analysis

HCD quantification was carried out according to residual *E. coli* HCD analysis of Gly-TB4 developed by Binex, thereby confirming a residual HCD amount of the final product.

#### (2-7) SDS-PAGE analysis

SDS-PAGE analysis was carried out according to electrophoresis of Gly-TB4 using conditions for separating Gly-TB4 and impurities by size, thereby qualitatively confirming impurity profiles, and cell recovery and an AEXI eluted pool were quantitatively analyzed with BSA as a reference or an affinity pool using a densitometer, as needed.

**Example (3) Anion Exchange Chromatography (AEXI)**

**(3-1) Preliminary experiment**

A preliminary experiment was conducted for improving purity by lowering an NaCl concentration in an elution buffer as much as possible.

**(3-1-1) Confirmation of target protein elution site**

A NaCl concentration at which GST-Tβ4 was eluted was screened under a linear gradient condition in an NaCl range of 0-500 mM using 2 mg of purified GST-Tβ4 (160709, 2nd column eluted fraction IPC sample acquired from 1st verification run). As a result, the location of the apex of the eluted main peak was identified at 20 mS/cm, and it was confirmed that an elution was conducted in a range from 200 to 300 mM NaCl.

**(3-1-2) GST-Tβ4 quantitative analysis (simple purification)**

In the case of an AEXI process, it was impossible to measure a step yield through UV quantification due to a large quantity of impurities in a loading sample and an elution sample. An analysis method for plotting the standard curve of the peak area of a standard material using an affinity column and confirming the amount of a target protein was intended to be developed (Table 51).

[Table 51]

| Items | | Process conditions |
|---|---|---|
| Resin | | Glutathione Sepharose 4 Fast Flow |
| Column | | LRC 10 (ID 1.0 cm, Height 1.2 cm), Column Volume (0.94 mL) |
| Flow rate | | 1 mL/min |
| Process | Equilibration | 20 mM Tris-HCl, 150 mM NaCl, pH 8.0 (5 CV) |
| | Sample Injection | 500 μL (Dilution with 20mM Tris-HCl, 500mM NaCl, pH 8.0) |
| | Unbound wash | 20 mM Tris-HCl, 150 mM NaCl, pH 8.0 (5 CV) |
| | Elution | 50 mM Tris-HCl, 10 mM Glutathione, pH 8.0 (5 CV) |
| Conditions for GST-Tβ4 Quantity Analysis | | |

0.3, 0.6, 0.9 and 1.2 mg/mL of standard materials were prepared through UV quantification (absorbance coefficient: 1.385) by buffer exchange of the 1st verification run affinity pool with a dilution buffer (20 mM Tris-HCl, 500 mM NaCl, pH8), and analyzed by the methods listed in Table 51, and then an integrated area was calculated using an AKTA operation program (Unicon 5.11). A linearity curve was plotted using the calculated values, resulting in obtaining a curve having a linearity of 0.99 or more, and AEXI process samples (loading sample and elution pool) were analyzed.

**(3-2) Flow rate verification test for shortening process time**

**(3-2-1) Purpose of experiment**

The process was conducted while a linear velocity was maintained at 61cm/hr, and the linear velocity was increased up to 200 cm/hr to shorten the time required for a unit process. The maintenance of a step yield and a purity was confirmed.

**(3-2-2) Experimental method**

The AEXI process was performed as shown in the following table (Table 52). Experiments were carried out by applying flow rates of 61 cm/hr and 200 cm/hr, respectively. Samples obtained before and after the process were analyzed by the method described in (3-1-2), and impurity profiles were confirmed through SDS-PAGE.

[Table 52]

| Items | | Process conditions |
|---|---|---|
| Resin | | Fractogel EMD TMAE(M) |
| Column | | XK16 (ID 1.6 cm, Height 20 cm), Column Volume (40 mL) |
| Flow rate | | 61 cm/hr, 200 cm/hr |
| Sample preparation | | Cell cake (Batch No. 160629-50L) → Prepare 15 % (w/w) slurry 11,000 psi, 2pass disruption → Centrifuge (8,500rpm, 50min, 10°C) Microfiltration (0.45 + 0.22 μm Double Layer) → Adjust pH and conductivity with 1 M Tris (pH 8.0, ≤ 2.8 mS/cm) |
| Process | Equilibration | 20 mM Tris-HCl, pH 8.0 (2 CV) |
| | Sample loading | Cell Lysate adjusted pH and conductivity (4.2~4.9 mg GST Tβ4/mL) |
| | Unbound wash | 20 mM Tris-HCl, pH 8.0 (5 CV) |
| | Elution | 20 mM Tris-HCl, 500 mM NaCl, pH 8.0 (5 CV) (100mAU - 100mAU) |
| | Strip | 2 M NaCl (3 CV) |
| | CIP | 0.5 M NaOH (3 CV) |
| | Strip | 2 M NaCl (2 CV) |
| | Equilibration | 20 mM Tris-HCl, pH 8.0 (3 CV) |

Conditions for AEXI Process

### (3-2-3) Result of experiment

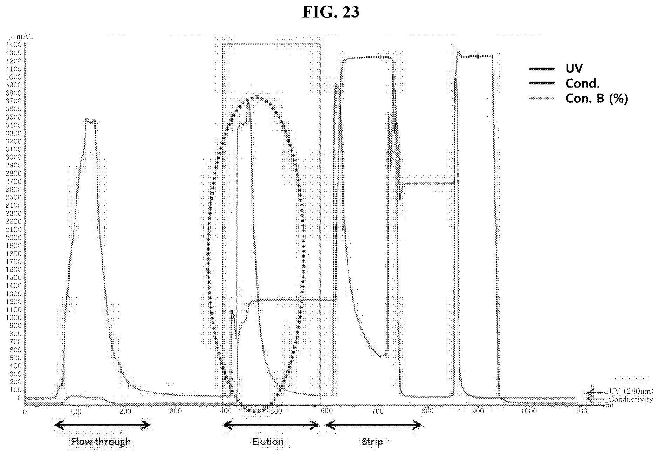

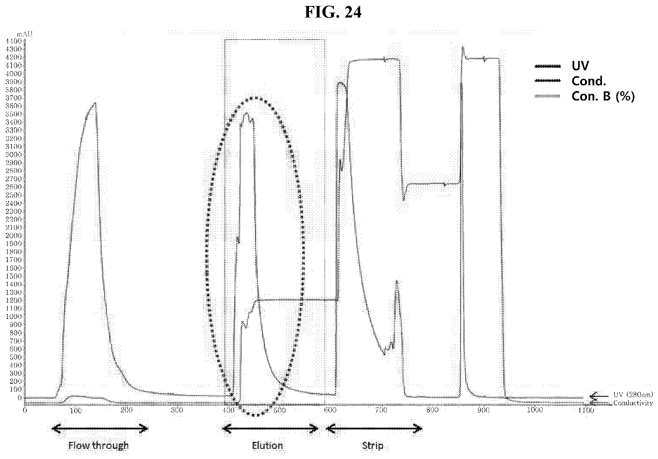

The chromatograms according to a flow rate after the AEXI process are shown in FIGS. 23 and 24, and the area indicated by a red dotted circle corresponds to an elution peak.

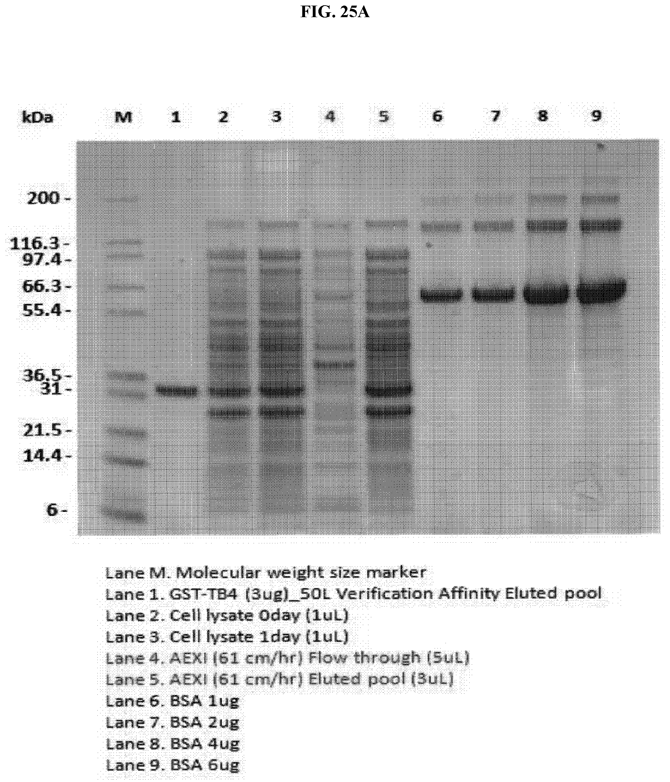

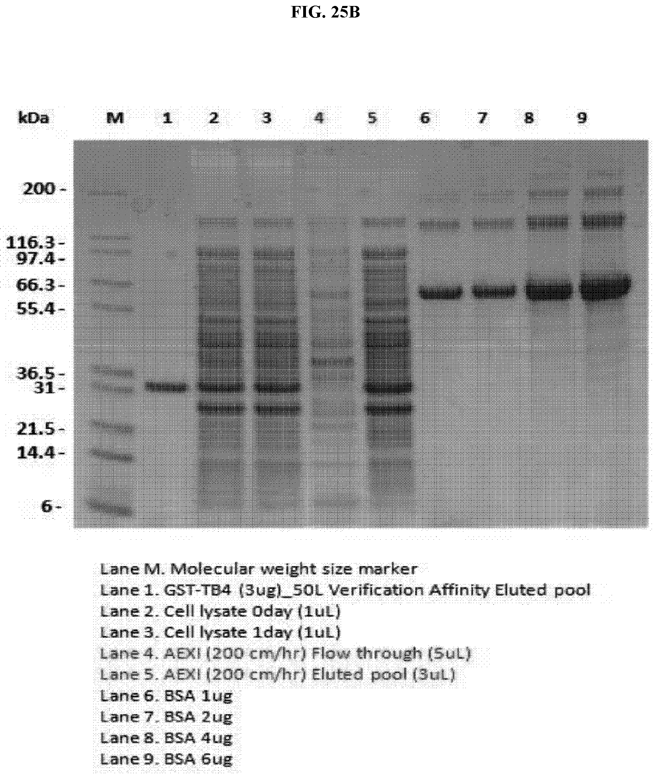

As a result of a step yield (Table 53) and SDS-PAGE analysis (FIG. 25) for an elution pool under each condition, it was confirmed that, even when the flow rate in the AEXI process was increased to 200 cm/hr, there was no effect on step yield and purity. However, since the load amount was approximately 5 mg/mL resin, reconfirmation was required when the load amount increased.

[Table 53]

| Sample | Start material | | Elution pool | | | Yield (%) |
|---|---|---|---|---|---|---|
| | Volume (mL) | Amounts (mg) | Concentration (mg/mL) | Volume (mL) | Amounts (mg) | |
| 61 cm/hr | 80 | 167.5 | 0.93 | 160 | 149 | 89% |
| 200 cm/hr | 80 | 167.5 | 0.93 | 155 | 144 | 86% |

Yield for AEXI Linear flow rate study

### (3-3) AEXI elution condition test for purity improvement

### (3-3-1) Purpose of experiment

Considering the economic feasibility of an expensive affinity column (GST FF), a DBC was intended to be maximized. To this end, the purity of a loading sample was raised as high as possible. Therefore, the experiment was conducted by changing a 500 mM NaCl condition into a 200 mM NaCl condition, and then a step yield and purity were comparatively analyzed.

### (3-3-2) Experimental method

An experimental method was basically the same as shown in Table 51 of (3-2-2) (Cell cake batch No. 160603 used in this experiment), and the experiment was conducted with an elution buffer having an NaCl concentration of 500 mM or 200 mM NaCl. HU024 was identified by analyzing samples before and after the process according to the method described in (2-1-2), and impurity profiles were confirmed through SDS-PAGE.

### (3-3-3) Result of experiment

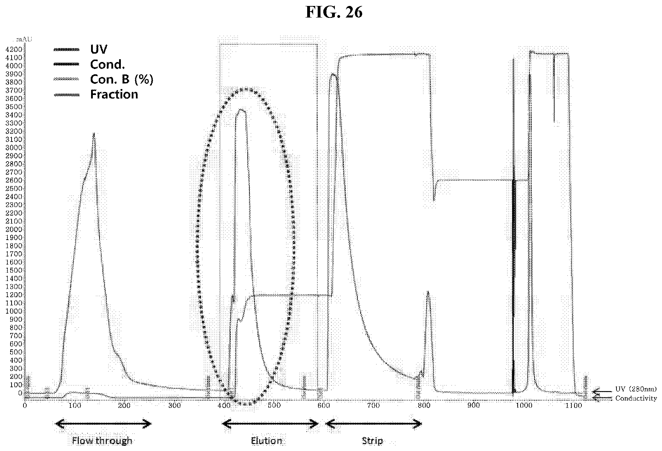

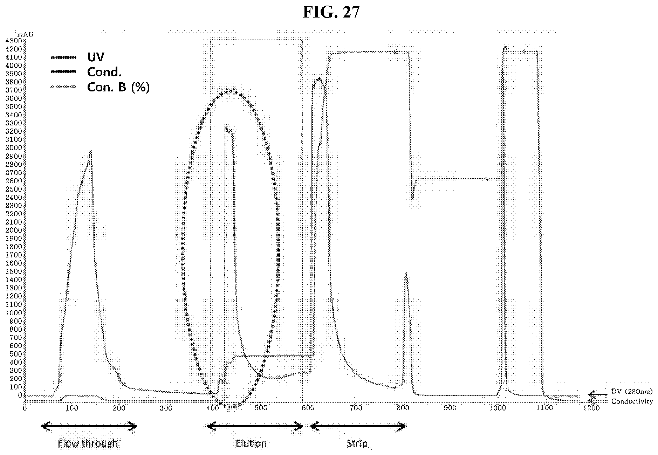

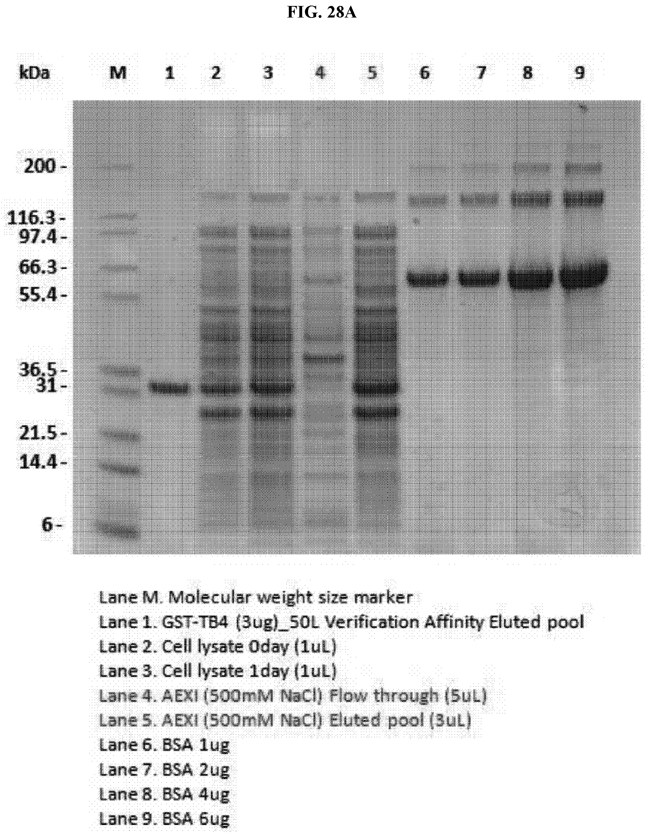

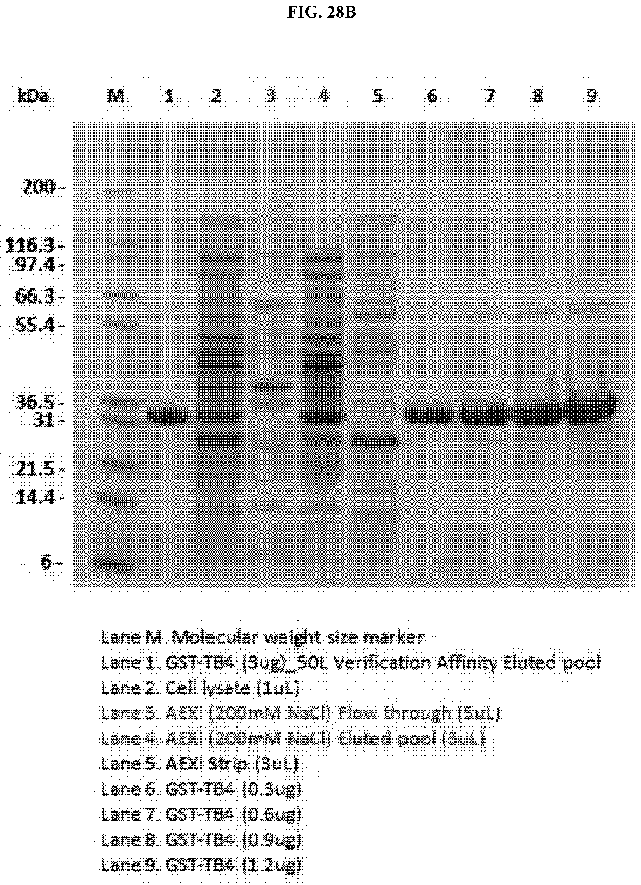

The chromatograms according to an elution buffer composition after the AEXI process are shown in FIGS. 26 and 27, and the area indicated by a red dotted circle corresponds to an elution peak.
According to the step yield result (Table 54) and SDS-PAGE analysis result (FIG. 28) for the elution pools by condition, there was no difference in AEXI yield, and as a result of the SDS-PAGE analysis, the major impurity peak below the target band was observed in the strip pool, rather than the elution pool, thus, a purity improving effect was expected. However, it was confirmed that an elution volume increased with a decreased NaCl concentration. In addition, when the load amount is increased by 5 mg/mL resin or more, additional experiments may be required.

[Table 54]

| Sample | Start material | | Elution pool | | | Yield (%) |
|---|---|---|---|---|---|---|
| | Volume (mL) | Amounts (mg) | Concentration (mg/mL) | Volume (mL) | Amounts (mg) | |
| Control (500 mM NaCl) | 80 | 142 | 0.79 | 146 | 115 | 81% |
| 200 mM NaCl | 80 | 142 | 0.69 | 179 | 123 | 87% |

Yield for AEXI Elution buffer study

### (3-4) AEXI Loading sample condition study

### (3-4-1) Purpose of experiment

The conductivity of a loading sample in the AEXI column is known as an important process variable by which a target protein is able to bind to the column. This experiment was conducted to confirm a conductivity range of an applicable loading sample, and the change in impurity content and step yield according to the variation.

### (3-4-2) Experimental method

An experimental method was basically the same as shown in Table 51 of (3-2-2) (Cell cake batch No. 160603 used in this experiment), and the loading samples were prepared to have a conductivity of 10 mS/cm (80 mM NaCl), 8 mS/cm (50 mM NaCl), and ≤ 2.8 mS/cm as a control, and an equilibrium buffer was prepared to have an NaCl concentration of 80 mM, 50 mM or 0 mM. The process was conducted with an elution buffer containing 200 mM NaCl. HU024 in the samples before and after the process was analyzed in the same manner as described in (2-1-2), and impurity profiles were confirmed through SDS-PAGE.

### (3-4-3) Result of experiment

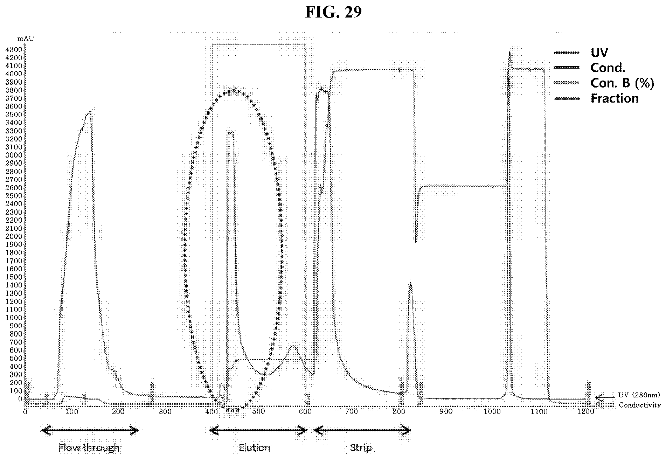

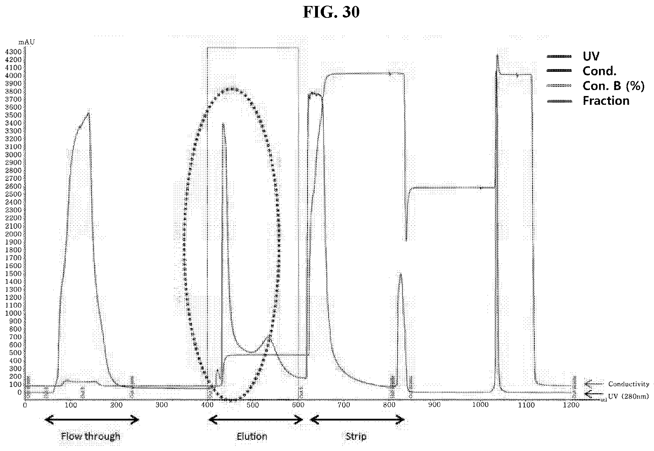

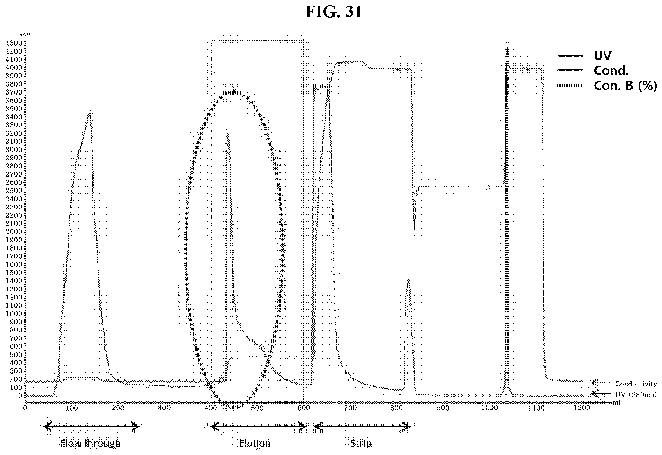

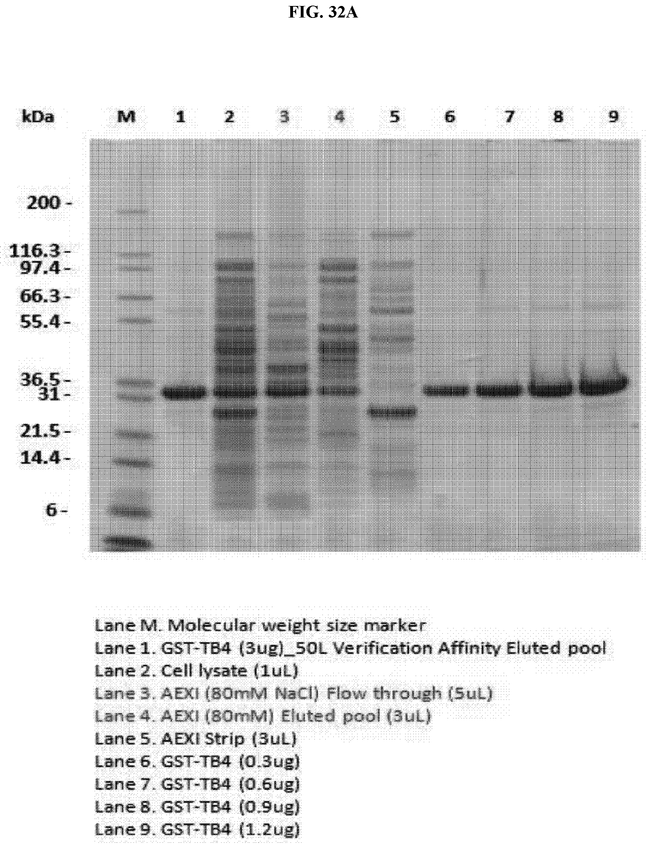

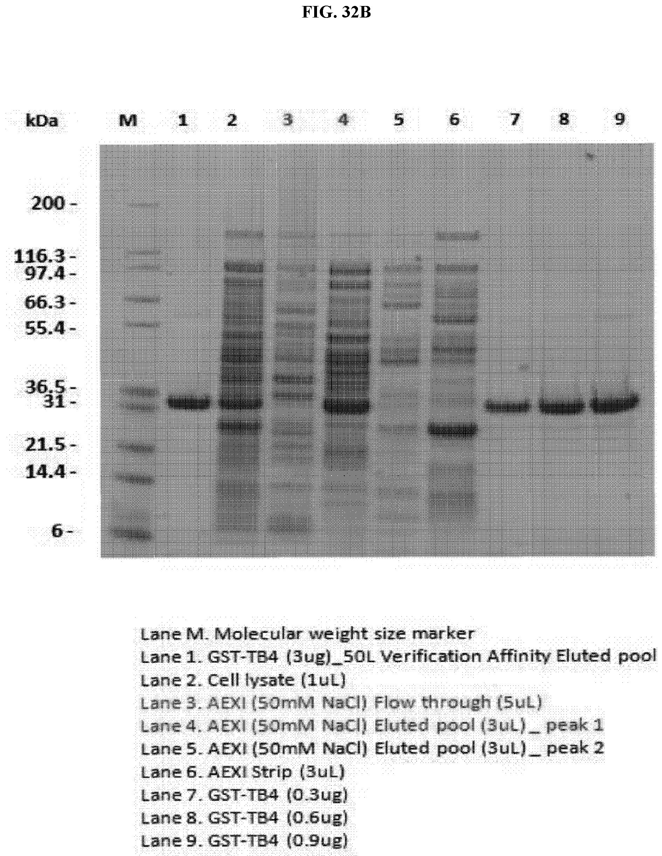

The chromatograms according to the change in conductivity of the loading samples and equilibration buffer after AEXI process are shown in FIGS. 29 to 31, and the area indicated by a red dotted circle corresponds to an elution peak.
Referring to the yields (Table 55) and SDS-PAGE result (FIG. 32) for the elution pool by condition, in the cases of the control (0 mM NaCl) and 50 mM NaCl, the yields were 81% and 97%, respectively, but in the case of 80 mM NaCl, the yield was 19.1%, and then F/T fractions were analyzed. According to the analysis result, it was confirmed that approximately 57% of the target proteins were observed in the F/T fractions. According to SDS-PAGE, it was confirmed that there was no difference in purity between the control and the 50 mM NaCl condition.

[Table 55]

| Salt conc. of load sample | Start material | | Elution pool | | | Yield (%) | Remark |
|---|---|---|---|---|---|---|---|
| | Volume (mL) | Amounts (mg) | Conc. (mg/mL) | Volume (mL) | Amounts (mg) | | |
| Control (0mM) | 80 | 198 | 0.87 | 184 | 161 | 81% | N/A |
| 50mM NaCl | 80 | 171 | 0.82 | 201 | 165 | 97% | N/A |
| 80mM NaCl | 80 | 158 | 0.17 | 180 | 30.1 | 19% | F/T |
| | | | 0.34 | 268 | 89.9 | 57% | Elution |

Step yield with different conductivities of loading sample for AEXI

According to the result of the test for the conductivities of a loading sample and an equilibrium buffer, it can be confirmed that, when the conductivities of loading samples were ≤ 2.8 mS/cm and 8.0 mS/cm (50 mM NaCl), a step yield and purity were the same. However, when the conductivity was 10 mS/cm (80 mM NaCl), the target protein was lost by F/T, so that it was confirmed to be inappropriate to apply, and it was confirmed that the conductivity of a loading sample in the AEXI process up to 8.0 mS/cm does not significantly affect AEXI binding. However, when the load amount was increased by 5 mg/mL resin or more, additional experiments were needed.

## (3-5) AEXI Dynamic Binding capacity (DBC) Test

### (3-5-1) Purpose of experiment

A DBC for GST-Tβ4 with respect to AEXI was to be confirmed.

### (3-5-2) Experimental method

An experimental method was basically the same as shown in Table 51 of (3-2-2) (Cell cake batch No. 160603 used in this experiment), but a LRC10 column was packed with 2 mL of Fractogel TMAE(M), a working linear flow rate was 150 cm/hr (Flow rate 2 mL/min), and compositions for an elution buffer and an EQ buffer were the same as the conditions established in process development (EQ: 20 mM Tris-HCl, 50 mM NaCl, pH 8.0, Elution: 20 mM Tris-HCl, 200 mM NaCl, pH 8.0). The process was conducted with a load amount of 10 mg, 8 mg or 7.5 mg GST-Tβ4/mL of resin (the conventional experiment condition was <5mg/mL of resin). F/T and elution pools were analyzed by SDS-PAGE, and the location of the target protein was confirmed.

### (3-5-3) Result of experiment

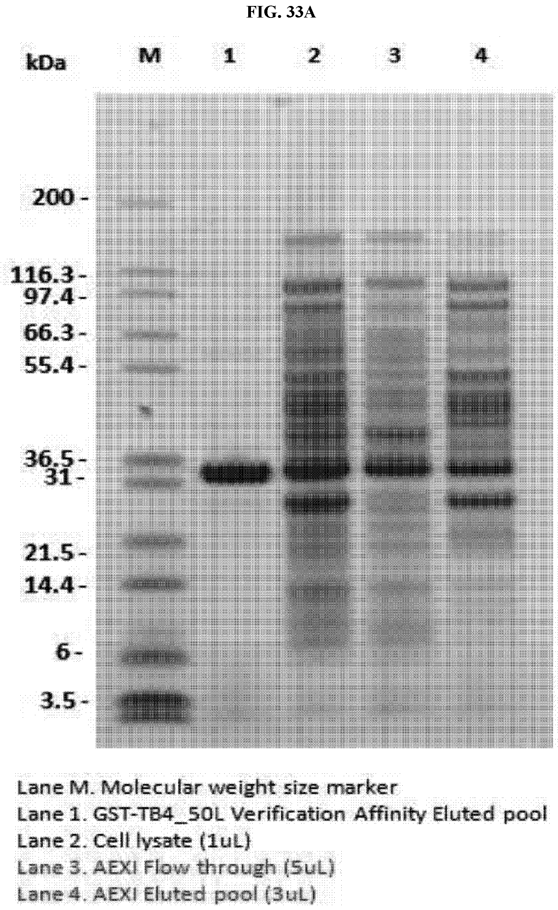

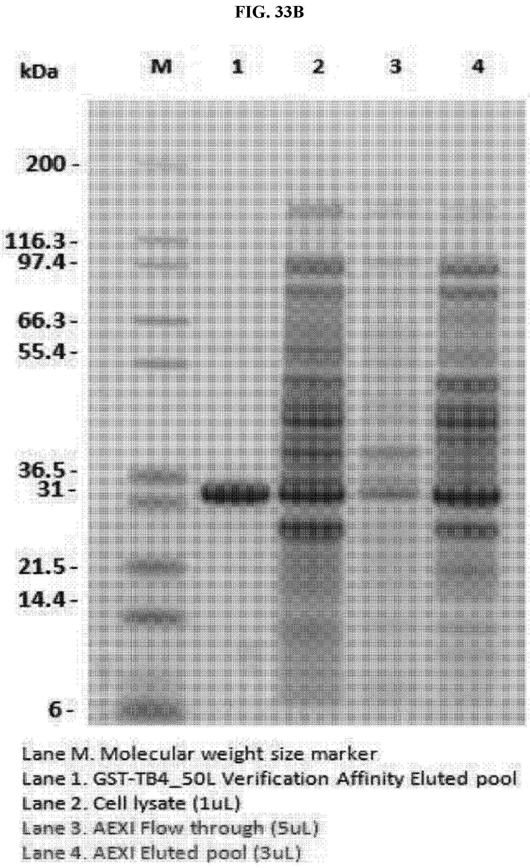

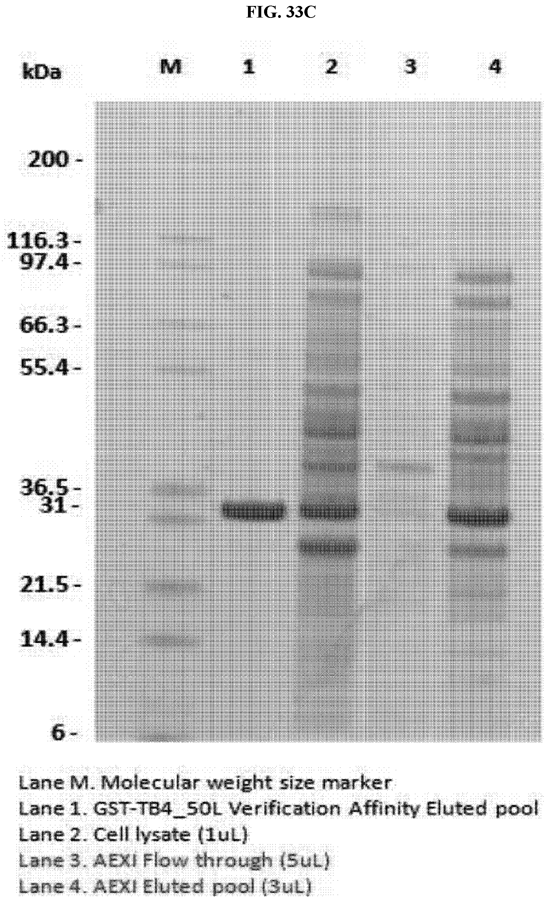

The result of analyzing the F/T and elution pools through SDS-PAGE is shown in FIG. 33. When 7.5 mg of GST-Tβ4 was injected, the target protein was not found in the F/T pool, and when 8.0 or 10.0 mg of GST-Tβ4 was injected, it was confirmed that the larger the load amount, the more target bands in the F/T pool. Accordingly, the DBC of AEXI was expected to be 7.5 mg/mL or less.

### (3-6) Hold time Study for AEXI process samples

### (3-6-1) Purpose of experiment

To secure the storage condition and period that can be applied to GMP production, in an AEXI process, eluted fraction samples were stored in a cold room (2 to 8 °C) and at room temperature (18 to 22 °C) for 7 days, followed by confirming the change in purity.

### (3-6-2) Experimental method

An experimental method was basically the same as shown in Table 52 of (3-2-2) (Cell cake batch No. 160906 used in this experiment), and the experiment was conducted under the conductivity of a loading sample of 8 mS/cm (50 mM NaCl), and an elution condition of 200 mM NaCl. An eluted pool was subjected to sterile filtration, divided into microtubes by 500 uL, and stored for 7 days in refrigeration and room temperature conditions. The sampled samples were stored at -70 °C, completely frozen, and simultaneously thawed one day after the storage period, followed by SDS-PAGE.

### (3-6-3) Result of experiment

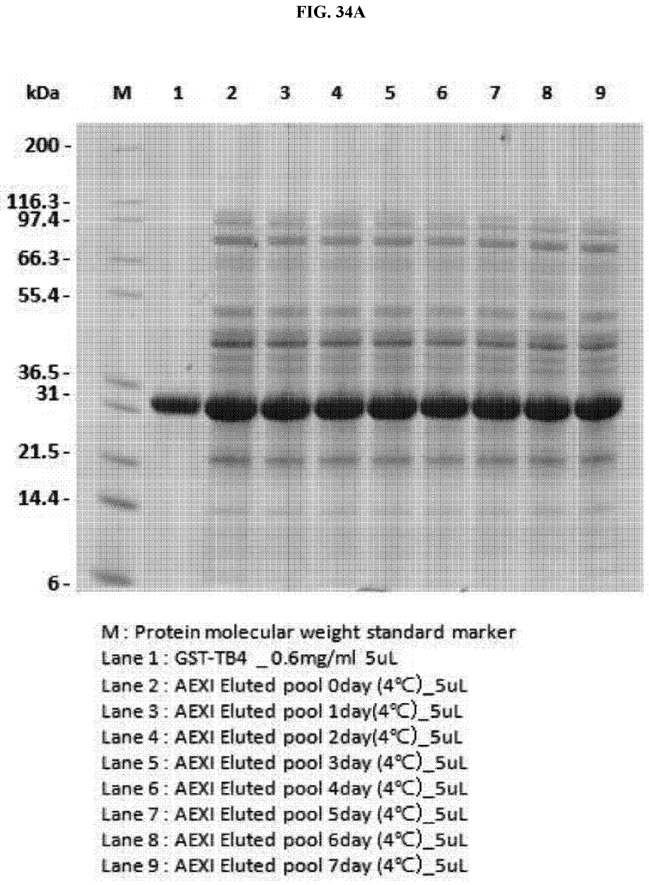

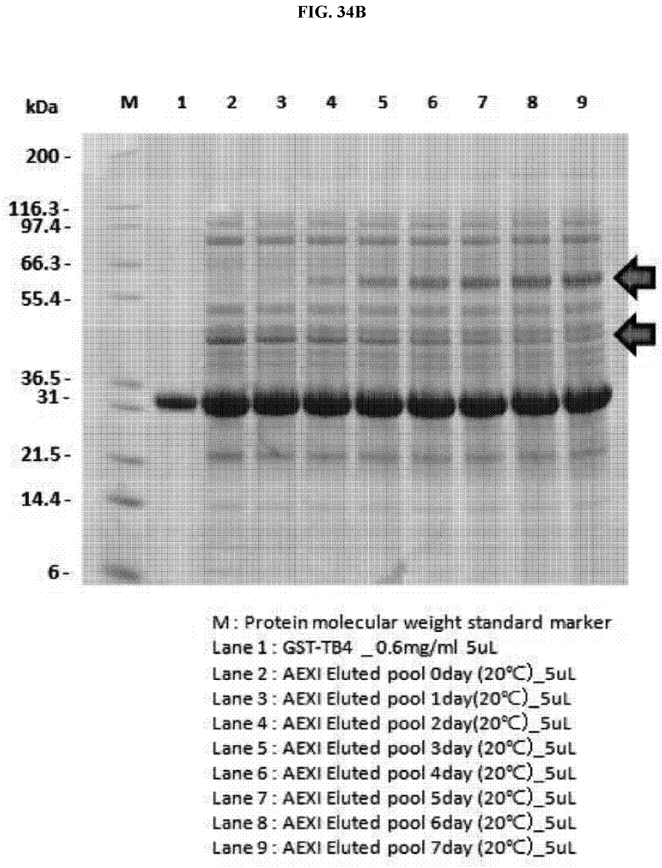

The SDS-PAGE result is shown in FIG. 34. There was no significant difference between the samples stored in the refrigerator. However, from day 2 of the room temperature storage, it was confirmed that the intensity of the band corresponding to approximately 55.4 kDa increased, and the intensity of the band corresponding to approximately 40 kDa decreased. Accordingly, it was confirmed that the AEXI process sample was stable for 7 days in a cold room condition (2 to 8 °C), but unstable in a room temperature (18 to 22 °C) condition.

### (3-7) Summary of AEXI process

[Table 56]

| Items | Process conditions | |
|---|---|---|
| Resin | Fractogel EMD TMAE(M) | |
| Dynamic binding capacity | Not more than 7.5 mg/mL | |
| Equilibration buffer | 20 mM Tris-HCl, 50 mM NaCl, pH 8.0 | |
| Elution buffer* | 20 mM Tris-HCl, 200 mM NaCl, pH 8.0 | |
| Process temperature | 15-25 °C | |
| Linear flow rate | 200 cm/hr | |
| Loading sample condition | pH 8.0 $\pm$ 0.1 (adjustment with 1 M Tris) Conductivity max 8.0 mS/cm (50 mM NaCl) | |
| Process | Equilibration | 2 CV |
| | Sample loading | Sample |
| | Unbound wash (with Equilibration buffer) | 5 CV |
| | Elution | 5 CV |
| | Strip (2 M NaCl) | 5 CV |
| | CIP (0.5 M NaOH) | 4 CV |
| | Strip (2 M NaCl) | 2 CV |
| | DW | 3 CV |
| | Storage (20 % Ethanol, 150 mM NaCl) | 3 CV |
| Elution pooling criteria | Start point : UV$_{280nm}$ Value $\geq$ 100 mAU End point: UV$_{280nm}$ Value $\leq$ 100 mAU | |
| Hold time for AEXI Eluted pool | Stable at 2 ~ 8°C for up to 7 days | |
| * According to client's request, finally determined as 300 mM NaCl Summary of AEXI process | | |

**Example (4) Glutathione Sepharose 4 Fast Flow (Affinity Chromatography)**

**(4-1) Flow rate condition test for shortening process time**

**(4-1-1) Purpose of experiment**

In a conventional process, the linear velocity was maintained at 90 cm/hr, and the linear velocity was increased up to 180 cm/hr to shorten the time required for a unit process. The maintenance of a step yield was confirmed.

**(4-1-2) Experimental method**

The affinity process was performed as shown in the following table (Table 57). Experiments were carried out by applying flow rates of 90 cm/hr and 180 cm/hr. HU024 in the samples before and after the process was analyzed in the manner described in (2-1), thereby confirming a step yield.

[Table 57]

| Resin | | Glutathione Sepharose 4 Fast Flow |
|---|---|---|
| Column | | LRC10 (ID 1.0 cm, Height 15 cm), Column Volume (11.8 mL) |
| Flow rate | | 90 cm/hr, 180 cm/hr |
| Sample preparation | | AEXI Eluted pool (EQ 50 mM NaCl, Elution 200 mM NaCl) |
| Process | Equilibration | 20 mM Tirs-HCl, 150 mM NaCl, pH 8.0 (2 CV) |
| | Sample loading | AEXI Eluted pool, directly loading (4.4 mg GST-Tβ4/mL resin) |
| | Unbound wash | 20 mM Tris-HCl, 150 mM NaCl, pH 8.0 (5 CV) |
| | Elution | 50 mM Tris-HCl, 10 mM Glutathione (Reduced), pH 8.0 (5 CV) (50 mAU ~ 50 mAU) |
| | Acidic buffer | 100 mM Sodium acetate, 500 mM NaCl, pH 4.5 (5 CV) |
| | Alkaline buffer | 100 mM Tris-HCl, 500 mM NaCl, pH 8.0 (5 CV) |
| | CIP | 6 M Guanidine hydrochloride (2 CV) |
| | DW | DW (2 CV) |
| | Equilibration | 20 mM Tirs-HCl, 150 mM NaCl, pH 8.0 (3 CV) |
| Conditions for affinity process | | |

**(4-1-3) Result of experiment**

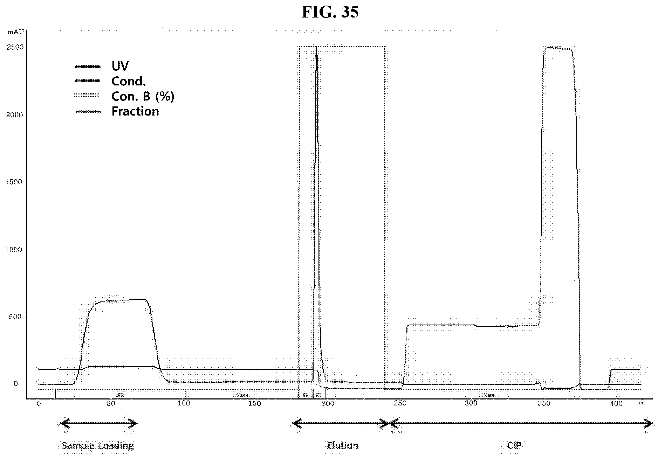

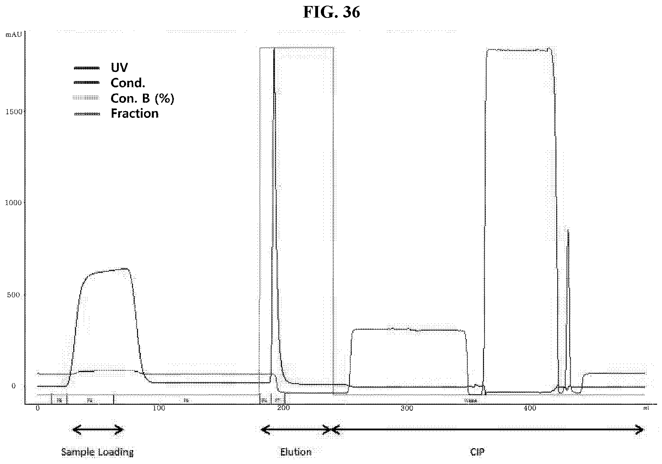

Chromatograms by condition according to the affinity process are shown in FIGS. 35 and 36.
Referring to the yields for the elution pool by condition (Table 58), it was confirmed that there was no effect on a step yield even when the flow rate in the affinity process was increased to 180 cm/hr. However, since the load amount was approximately 4.4 mg/mL resin, reconfirmation was required when the load amount increased.

[Table 58]

| Sample | Start material | | Elution pool | | | Yield (%) |
|---|---|---|---|---|---|---|
| | Volume (mL) | Amounts (mg) | Concentration (mg/mL) | Volume (mL) | Amounts (mg) | |
| 90 cm/hr | 50 | 0.82 | 2.91 | 8..8 | 25.5 | 62% |
| 180 cm/hr | 50 | 0.82 | 2.33 | 11.2 | 26.1 | 64% |

Yield for affinity linear flow rate study

**(4-2) Dynamic binding capacity (DBC) test**

**(4-2-1) Purpose of experiment**

A DBC of GST-Tβ4 with respect to an affinity column was to be confirmed.

**(4-2-2) Experimental method**

An affinity process was conducted under the condition shown in Table 57 of (4-2), and a LRC10 column was packed with 1 mL of Glutathione Sepharose 4 Fast Flow resin, and a linear flow rate of 76.4 cm/hr (flow rate: 1 mL/min, however, 150 cm/hr during elution) was applied. A loading sample was overloaded by injecting an AEXI eluted pool (GST-Tβ4 41 mg) obtained through an AEXI optimized process, compared with a DBC (≒ 11 mg GST-tagged protein/mL medium) in a resin manual, and after the affinity process, the process sample was analyzed through the UV quantification method described in (2-1) UV quantification method.

**(4-2-3) Result of experiment**

The content of an eluted fraction obtained by elution after overloading in 1 mL of resin was measured (Table 59), and the DBC of the glutathione Sepharose 4 fast flow with respect to GST-Tβ4 was confirmed to be 9.3 mg/mL.

[Table 59]

| Items | Condition & Results | | | | |
|---|---|---|---|---|---|
| Loading sample | AEXI eluted pool (GST-Tβ4 41mg, volume 50mL) | | | | |
| Eluted pool | Conc. (mg/mL) | Volume (mL) | Amounts (mg) | Resin volume (mL) | DBC (mg/mL) |
| | 1.36 | 6.5 | 8.75 | 0.94 | 9.31 |

DBC for Glutathione Sepharose 4 Fast Flow

**(4-3) AEXI process scale-up and affinity process reproducibility experiments**

**(4-3-1) Purpose of experiment**

An AEXI process scale-up experiment (XK16 --> XK50) was conducted.

Reproducibility and scale-up experiments were performed by applying the affinity process conditions (linear flow rate in sample loading: 80 cm/hr, linear flow rate in elution: 150 cm/hr, LRC10 --> XK50 condition).

**(4-3-2) Experimental method**

The same conditions specified in (3-7) were applied to the AEXI process, and XK16 (h: 20 cm) or XK50 (h: 20 cm) was applied to each column. The affinity process was conducted in the same manner as shown in the table below (Table 60), and LRC10 (h: 15 cm) or XK50 (h: 15 cm) was applied to each column.

[Table 60]

| Items | Process conditions |
|---|---|
| Resin | Glutathione Sepharose 4 Fast Flow |
| Flow rate | Sample Loading & Unbound Wash 80 cm/hr<br>Elution step : 150 cm/hr |
| Loading sample | AEXI Eluted pool (w/o adjustment of pH or conductivity) |

(continued)

| Items | | Process conditions |
|---|---|---|
| Process | Equilibration | 20 mM Tris-HCl, 150 mM NaCl, pH 8.0 (2 CV) |
| | Sample loading | AEXI eluted pool, directly loading |
| | Unbound wash | 20 mM Tris-HCl, 150 mM NaCl, pH 8.0 (5 CV) |
| | Elution | 50 mM Tris-HCl, 10 mM Glutathione, pH 8.0 (5 CV) |
| | Acidic buffer | 100 mM Sodium Acetate, 500 mM NaCl, pH 4.5 (5 CV) |
| | Alkaline buffer | 100 mM Tris-HCl, 500 mM NaCl, pH 8.0 (5 CV) |
| | CIP* | 6 M Guanidine hydrochloride (2 CV) |
| | DW* | DW (2 CV) |
| | Storage | 20 % Ethanol |
| Elution pooling criteria | | Start point: $UV_{280nm}$ Value $\geq$ 100 mAU |
| | | End point : $UV_{280nm}$ Value $\leq$ 100 mAU |

*When CIP flows, the linear flow rate was set to be 80 cm/hr, and when DW flows, the same flow rate needs to be applied. (Max pressure of resin: 1 bar)
Conditions for affinity chromatography

### (4-3-3) Result of experiment

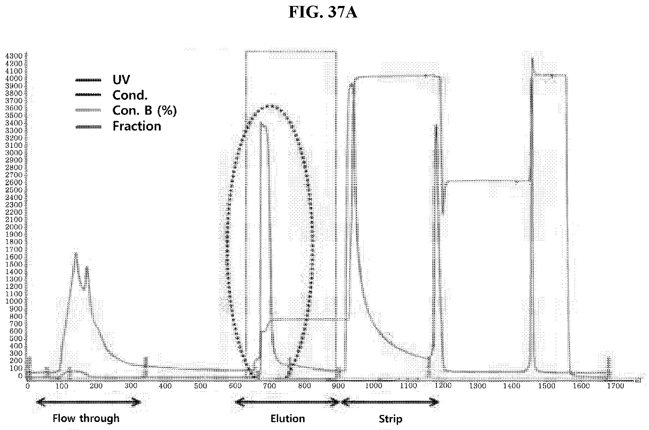

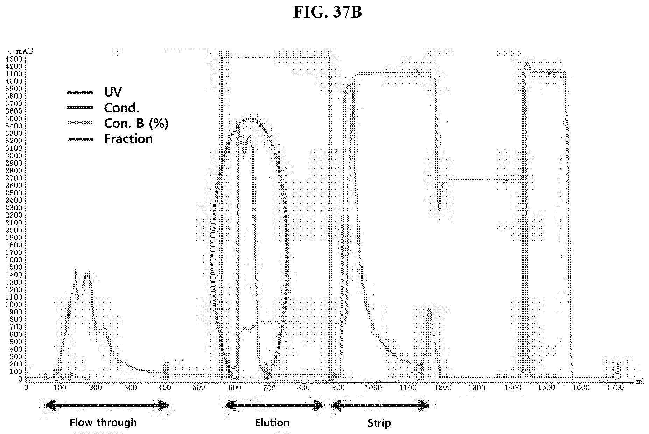

As a result of the AEXI column scale-up, a chromatogram is shown in FIG. 37, and step yields are shown in Table 61.

[Table 61]

| Sample | Start material | | Elution pool | | | Yield (%) |
|---|---|---|---|---|---|---|
| | Volume (mL) | Amounts (mg) | Concentration (mg/mL) | Volume (mL) | Amounts (mg) | |
| XK16 scale* | 50 | 269 | 4.02 | 71 | 287 | 107% |
| XK50 scale* | 450 | 2,601 | 4.25 | 595 | 2,529 | 97% |

*Loaded at a load amount of 6.7 mg/mL of resin

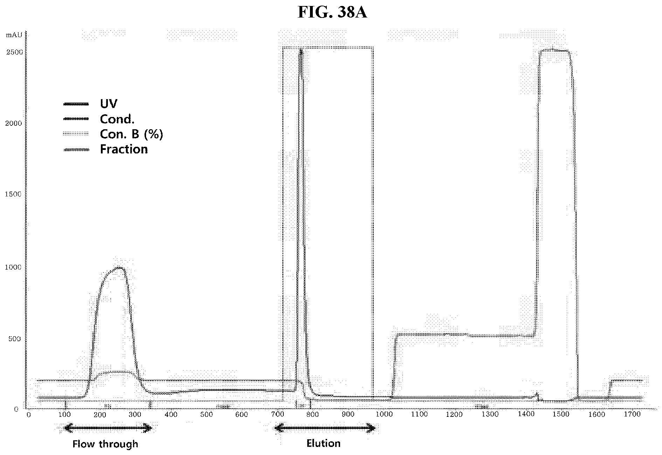

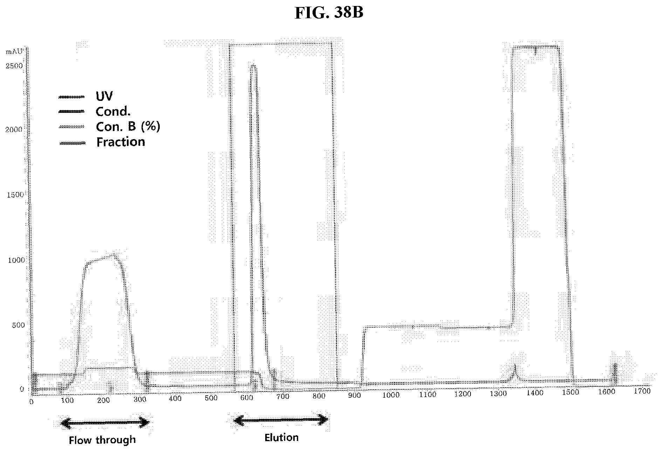

AEXI step yield for scale up study from XK16 to XK50
Scale-up was confirmed by measuring similar step yields in the two types of columns. The eluted fractions were then applied to affinity columns (FIG. 38 and Table 62).

[Table 62]

| Sample | Start material | | Elution pool | | | Yield (%) |
|---|---|---|---|---|---|---|
| | Volume (mL) | Amounts (mg) | Concentration (mg/mL) | Volume (mL) | Amounts (mg) | |
| LRC10 scale* | 25 | 101 | 6.43 | 9 | 61 | 60% |
| XK50 scale* | 595 | 2,529 | 8.32 | 221 | 1,842 | 73% |

*Loaded at a loading amount of 8.6 mg/mL of resin

Affinity step yield for feasibility test at LRC10 and XK50 scale

The experiments were conducted by setting a loading amount (8.6 mg/mL of resin) of a sample to be 90% of the DBC (9.3 mg/mL of resin) determined in the previous experiment and applying a loading rate of 80 cm/hr and an elution rate of 150 cm/hr, confirming that step yields were 60% and 73%.

### (4-4) Affinity column cleaning

### (4-4-1) Background

In the 1st batch of a 50L verification run, an endotoxin content measured after 6 M GHCl-treated CIP was measured was higher than 0.1 EU/mL, so that additional CIP methods were conducted (Table 63).

[Table 63]

| Run No. | Process condition | Endotoxin (EU/mL) |
|---|---|---|
| CIP Run1 | 6M Guanidine hydrochloride(Gdn-HCl) (2CV) - contact time 34min | > 1 EU/mL |
| CIP Run2 | 6M Gdn-HCl(2CV) - contact time 34min -> DW -> 2M NaCl (2CV) | 0.58 EU/mL |
| CIP Run3 | 400mM Potassium phosphate(KPi) (1CV) - contact time 17min | 0.27 EU/mL |
| CIP Run4 | 0.1N NaOH (contact time 10min) -> 400mM KPi | 0.24 EU/mL |
| CIP Run5 | 0.1N NaOH (contact time 34min) -> 2M NaCl (2CV) -> DW -> 20% EtOH (3CV, contact 69min) | 0.085 EU/mL |
| Endotoxin removal in affinity column by various CIP methods | | |

A CIP method (CIP Run5) to which 0.1N NaOH and ethanol recommended by GE were applied was applied to a subsequent process (Table 63).

### (4-5) Hold time study for affinity process samples

### (4-5-1) Purpose of experiment

Affinity process samples were stored for 7 days in cold room (2 to 8 °C) and room temperature (18 to 22 °C) conditions, and then the change in purity was confirmed to set storage conditions and period for the process samples.

### (4-5-2) Experimental method

An experimental method was basically the same as shown in Table 58 of (4-2), and as loading samples, process samples except the AEXI hold time study samples in (3-6) were used. The eluted pool was subjected to sterile filtration, divided into microtubes by 500 uL and stored for 7 days in the cold room and room temperature conditions. The sampled samples were stored at -70 °C, completely frozen, simultaneously thawed one day after the end of the storage period, followed by SDS-PAGE.

### (4-5-3) Result of experiment

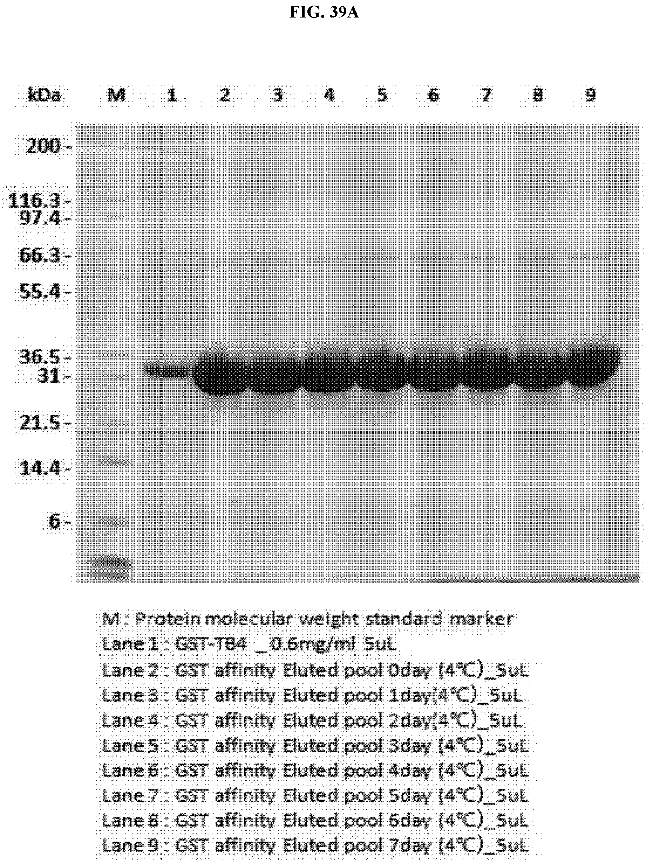

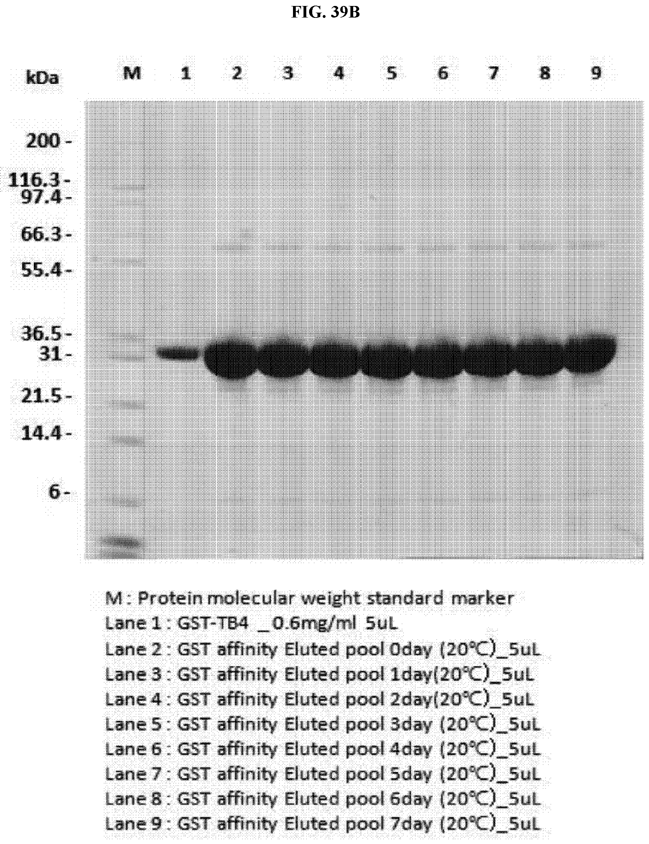

The result of SDS-PAGE after the experiments for the storage conditions (room temperature and cold room) is shown in FIG. 39. No significant difference was found in the SDS-PAGE result of the samples stored in refrigeration and room temperatures. Accordingly, it was confirmed that the affinity process samples were stable for 7 days in refrigeration (2 to 8 °C) or room temperature (18 to 22 °C) storage.

### (4-6) Summary of affinity process

[Table 64]

| Items | Process conditions |
|---|---|
| Resin | Glutathione Sepharose 4 Fast Flow |

(continued)

| Items | Process conditions | |
|---|---|---|
| Dynamic binding capacity | Not more than 9.3 mg/mL | |
| Equilibration buffer | 20 mM Tris-HCl, 150 mM NaCl, pH 8.0 | |
| Elution buffer | 50 mM Tris-HCl, 10 mM Glutathione (reduced), pH 8.0 | |
| Process temperature | 15~25 °C | |
| Linear flow rate | Sample Loading, unbound wash 80 cm/hr<br>Elution 150 cm/hr | |
| Loading sample condition | AEXI eluted pool, directly loading | |
| Process | Equilibration | 2 CV |
| | Sample loading | Sample |
| | Unbound wash (with Equilibration buffer) | 5 CV |
| | Elution | 5 CV |
| | Acidic buffer | 5 CV |
| | Alkaline buffer | 5 CV |
| | CIP (6 M Guanidine hydrochloride)* | 2 CV |
| | DW* | 2 CV |
| | Storage (20 % Ethanol) | 3 CV |
| Elution pooling criteria | Start point: $UV_{280nm}$ Value $\geq$ 100 mAU<br>End point: $UV_{280nm}$ Value $\leq$ 100 mAU | |
| Hold time for Affinity eluted pool | Stable at 2 ~ 8°C and 18 ~ 22°C for up to 7 days | |

*When CIP flows, the linear flow rate is set to be 80 cm/hr, and when DW flows, the same flow rate is applied. (Max pressure of resin: 1 bar)

Summary of affinity process

## Example (5) Thrombin Digestion

### (5-1) Background

When the thrombin digestion condition (2.6 mg GST-Tβ4) was applied according to the Chinese process, it was confirmed that, when a target protein concentration in an affinity pool is low, enzyme cleavage efficiency was low (when 2 mg/unit of thrombin was added at the concentration of the affinity pool of 0.3 mg/mL, only approximately 50% reacted in SDS-PAGE). Accordingly, in this experiment, 1 mg GST-Tβ4/2 units of thrombin was applied, and the reaction time was fixed at 16 hours, and then the similarity of enzyme cleavage efficiencies in the range of 13 to 27 °C was confirmed to set a reaction temperature range.

[Table 65]

| Items | Process conditions |
|---|---|
| Enzyme | Chromatopur Bovine Thrombin (MP bio) (Cat # 02199907) |
| Enzyme / Protein Ratio | 1 mg GST-Tβ4 / 2 unit Thrombin |
| Mix Agitation | 60 rpm |
| Reaction Time | 16 hrs |

Conditions for thrombin digestion

**(5-2) Preparation of thrombin digestion samples**

To perform a thrombin digestion study, AEXI and affinity processes were conducted, and the process conditions were the same as those specified in process development. The process-completed affinity pool was divided into conical tubes by 40 mL, stored in a cold room (-20 °C), and thawed as needed.

**(5-3) Thrombin digestion study by reaction temperature**

**(5-3-1) Purpose of experiment**

Under a thrombin digestion reaction condition, a temperature range was to be additionally set.

**(5-3-2) Experimental method**

The affinity elution pool (209.1 mg GST-Tβ4) prepared in (5-2) was used, and Thrombin was dissolved in 20 mM Tris-HCl (pH 8.0) of 15%(v/v) of the volume of the affinity pool to be reacted, and then applied to treat the elution pool. Afterward, reactions were performed with the method specified in Table 65, but the reactions were simultaneously conducted at 15 ± 2 °C (cold room), 20 ± 2 °C (room temperature) and 25 ± 2 °C (incubator). The reaction-completed samples were subjected to SEC-HPLC specified in (2-2) to quantify Gly-Tβ4, thereby confirming yields (2016-037, p 13, 18).

**(5-3-3) Result of experiment**

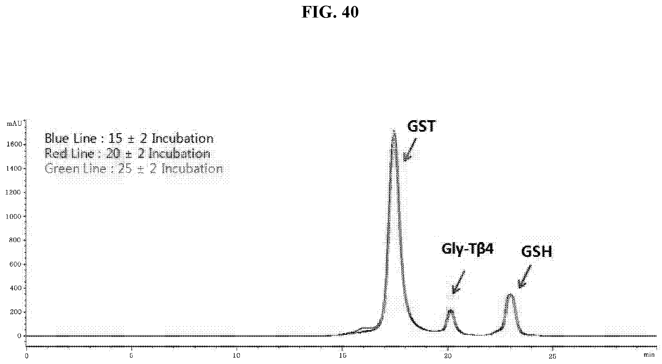

The SEC-HPLC results for the samples that had been subjected to thrombin digestion by reaction temperature are shown in FIG. 40. When the chromatogram and yields were examined (Table 66), no significant difference according to temperature was confirmed. Therefore, it was confirmed that the thrombin reaction temperature condition ranging from 13 to 27 °C does not affect a yield and purity.

[Table 66]

| Sample | Start material | | Elution pool | | | Yield (%) |
| --- | --- | --- | --- | --- | --- | --- |
| | Volume (mL) | Amounts (mg) | Conc. (mg/mL) | Volume (mL) | Amounts (mg) | |
| 15 ± 2 ℃ | 34.5 | 209 | 1.07 | 34.5 | 36.9 | 17.7% |
| 20 ± 2 ℃ | 34.5 | 209 | 1.09 | 34.5 | 37.5 | 17.9% |
| 25 ± 2 ℃ | 34.5 | 209 | 1.07 | 34.5 | 37.1 | 17.7% |

Step yield for cleavage reaction mixtures

**Example (6) 4th process study**

**(6-1) Background**

According to the result of the 1st 50L verification run, the loss of a target protein due to an F/T fraction in the AEXII process was observed, and it was confirmed that the purities of RP-HPLC and SEC-HPLC of an elution pool are lower than the F/T Fraction. Based on the result of the experiment, the condition of the 2nd verification run was changed to an F/T mode, so that a purity and a yield were improved. However, in SE-HPLC of the final purified extract, it was necessary to further improve purity to be 97.0%, and in non-optimized RP-HPLC, the purity was confirmed at 98.7%, and therefore, 4th column introduction was considered.

**(6-2) Sample production for developing 4th column process**

To ensure a sample required for a 4th column process experiment, a sample was prepared by performing an AEXII process in an F/T mode, which is the 2nd verification run condition. For sample production, a cell lysate was prepared for a depth sizing test of two 5L DoE batches (Batch No. 161019-B 1, B2). After the sizing test, 7099 mg of Gly-Tβ4 was secured by performing AEXI, affinity, thrombin digestion and AEXII processes using the remaining cell lysate (approximately 1200 mL). While the AEXI and affinity processes were conducted in the same manner as described in (3-7) and (4-1), a column scale was applied to the AEXI (XK50, h: 20 cm) and affinity (XK50, h: 15 cm) processes, and the AEXII process was conducted by the method specified in Table 67. The obtained sample was subjected to microfiltration (0.2 μm), divided by 100 mL, and stored at -20 °C, followed by the development of the 4th column process using this.

[Table 67]

| Items | | Process conditions |
|---|---|---|
| Resin | | Fractogel EMD TMAE(M) |
| Column | | XK50 (ID 5.0 cm, Height 20 cm), Column Volume (392.5 mL) |
| Flow rate | | 200 cm/hr (65.41 mL/min) |
| Sample preparation | | After Thrombin digestion, Adjust pH conductivity (pH 8.0, conductivity 6 mS/cm) with DW |
| Process | Equilibration | 20 mM Tris-HCl, 50 mM NaCl, pH 8.0 (2 CV) |
| | Sample loading | Thrombin digestion sample |
| | Unbound wash | 20 mM Tris-HCl, 50 mM NaCl, pH 8.0 (5 CV) |
| | Strip | 2 M NaCl (3 CV) |
| | CIP | 0.5 M NaOH (3 CV) |
| | Strip | 2 M NaCl (2 CV) |
| | Equilibration | 20 mM Tris-HCl, 50 mM NaCl, pH 8.0 (3 CV) |
| Elution criteria | | Start point: UV$_{(214nm)}$ Value ≥ 50 mAU (Flow through pooling) End point : UV$_{(214nm)}$ Value ≤ 50 mAU |
| AEXII process conditions | | |

The step yields and purities of the secured 3rd column eluted fractions were analyzed (Tables 68 and 69). While the SE-HPLC purity was improved up to 98.9%, the RP-HPLC purity was 97.6%. However, when analyzed by the optimized RP-HPLC analysis method, purity fell to 92.2%, and thus it was intended to improve purity to 99.0% or more.

[Table 68]

| Sample | Start material | | Elution pool | | | Yield (%) | HCP (ppm) |
|---|---|---|---|---|---|---|---|
| | Volume (mL) | Amounts (mg) | Conc. (mg/mL) | Volume (mL) | Amounts (mg) | | |
| Cell Lysate | | | 5.07 | 1,212 | 6,145 | - | - |
| AEXI | 1,212 | 6,145 | 3.45 | 1,927 | 6,648 | 108% | 1,916 |
| Affinity | 1,835 | 6,329 | 7.79 | 625 | 4,871 | 77% | 137 |
| Thrombin Reaction | 625 | 4,871 | 1.09 | 707 | 771 | 16% | 821 |
| AEXII | 1069 | 770 | 0.33 | 2125 | 710 | 92% | 17 |

Step yield of AEX II for preparing load sample on 4th column

[Table 69]

| Sample | SEC-HPLC | | | RPC-HPLC (ver. 1.0) | | |
|---|---|---|---|---|---|---|
| | Test 1 | Test 2 | Average | Test 1 | Test 2 | Average |
| UF/DF | 98.9 | 99.0 | 98.9 | 97.4 | 97.8 | 97.6 |

Purity analysis after UF/DF for preparing load sample on 4th column

## (6-3) Resin scouting

### (6-3-1) Cation Exchange Chromatogram (CEX) process

To improve purity, SP FF, which is a basic CEX resin, was applied (Table 70).

[Table 70]

| Items | | Process conditions |
|---|---|---|
| Resin | | SP Sepharose Fast Flow |
| Column | | LRC10 (ID 1.0 cm, Height 16.5 cm), Column volume (13.0 mL) |
| Flow rate | | 200 cm/hr (2.63 mL/min) |
| Sample preparation | | AEXII adjusted pH and conductivity (pH 4.5, 5.0, conductivity ≤ 2.8 mS/cm) |
| Process | Equilibration | 20 mM Sodium Acetate, pH 4.5, 5.0 (2 CV) |
| | Sample loading | AEXII sample adjusted pH and conductivity (11.8 mg Gly-Tβ4) |
| | Unbound wash | 20 mM Sodium Acetate, pH 4.5, 5.0 (5 CV) |
| | Elution | 20 mM Sodium Acetate, 1.0 M NaCl, pH 4.5, 5.0 (10 CV, 0 ~ 100 %B Linear gradient) |
| | Strip | 2 M NaCl (3 CV) |
| | CIP | 0.5 M NaOH (3 CV) |
| | Strip | 2 M NaCl (2 CV) |
| | Equilibration | 20 mM Sodium Acetate, pH 4.5, 5.0 (3 CV) |

CEX process conditions for 4th column

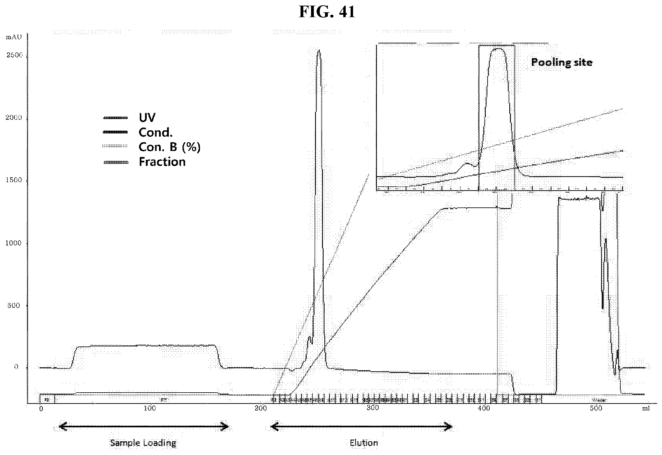

The chromatogram for the CEX process is shown in FIG. 41. Two separated peaks were shown, and step yields of the processes proceeded with pH 4.5 and 5.0 were confirmed (Table 71).

[Table 71]

| Sample | Start material | | Elution pool | | | Yield (%) |
|---|---|---|---|---|---|---|
| | Volume (mL) | Amounts (mg) | Conc. (mg/mL) | Volume (mL) | Amounts (mg) | |
| pH 4.5 | 130 | 11.8 | 1.15 | 10 | 11.5 | 97.5% |
| pH 5.0 | 62.5 | 8.8 | 0.24 | 25 | 6.1 | 69.3% |

Yield for CEX process by pH

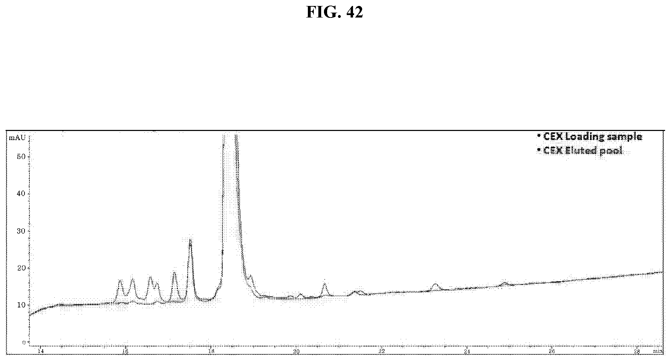

In the case of pH 5.0, no separated peak was found, and the yield was confirmed to be lower than that in the case of the pH 4.5 condition. When the F/T fraction was analyzed, a target protein was identified, and thus it was expected that the above result was caused by the target protein. Accordingly, only the pH 4.5 condition was subjected to RP-HPLC analysis, and the analysis result is shown in FIG. 42.

It was confirmed that many impurity peaks before and after the main peak were removed, and the RP-HPLC purities of a loading sample and an elution sample were confirmed to be 92.2% and 97.6%, respectively. However, the impurity peak present before the main peak was not removed, and referring to a UF/DF result (Table 69), there was no significant difference from the RP-HPLC purity (97.6%), so that a hydrophobic resin, instead of a CEX-type resin, was to be confirmed to ensure a purity of 99% or more.

## (6-3-2) Hydrophobic Interaction Chromatography (HIC) process

Considering the separation between the main and impurity peaks and a target peak in RP-HPLC, hydrophobic columns were tested, and process conditions are shown in Table 72. Two resins (butyl and phenyl) were tested according to the degree of hydrophobicity, and as salt conditions, both NaCl and Na$_2$SO$_4$ were used.

[Table 72]

| Items | | Process conditions |
|---|---|---|
| Resin | | Butyl Sepharose 4 Fast Flow<br>Phenyl Sepharose 6 Fast Flow (High sub) |
| Column | | LRC10 (ID 1.0 cm, Height 15.0 cm), Column volume (11.8 mL) |
| Flow rate | | Sample Loading 80 cm/hr (1.04 mL/min)<br>Elution 150 cm/hr (1.96 mL/min) |
| Sample preparation | | AEXII adjusted pH and conductivity with 4 M NaCl and 2 M Na$_2$SO$_4$ (pH8.0, Conductivity $\geq$ 90 mS/cm) |
| Process | Equilibration | 20 mM Tris-HCl, 2 M NaCl, 1.0 M Na$_2$SO$_4$, pH8.0 (2 CV) |
| | Sample loading | AEXII sample adjusted pH and conductivity (16.2 mg Gly-Tβ4) |
| | Unbound wash | 20 mM Tris - HCl, 2 M NaCl, 1.0 M Na$_2$SO$_4$, pH8.0 (5 CV) |
| | Elution | 20 mM Tris-HCl, pH8.0<br>(10 CV, 0 ~ 100 %B Linear gradient) |
| | Strip | DW (3 CV) |
| | CIP | 0.5 M NaOH (3 CV) |
| | Strip | 100 mM Sodium Acetate, pH 4.5 (2 CV) |
| | Equilibration | 20 mM Tris-HCl, 2 M NaCl, 1.0 M Na$_2$SO$_4$, pH8.0 (3 CV) |

HIC process conditions for 4th column

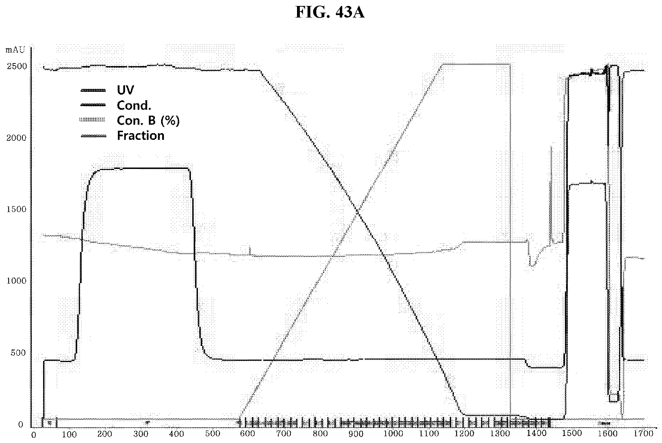

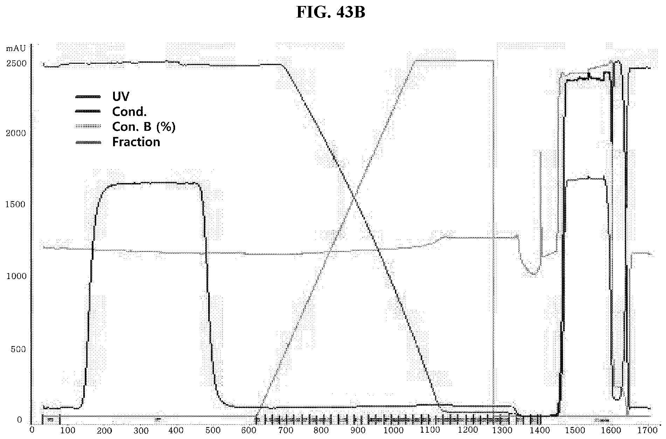

The process chromatogram is shown in FIG. 43. A high peak was formed in the front of the chromatogram, and there was no peak observed in the elution step.

As a result, the target protein did not bind to both of the resins, and thus there was no additional progression.

## (6-3-3) Mixed-Mode Chromatography (MMC) process

As a result of the CEX process and the HIC resin study, the removal of main impurities (17.5 min peak when RP-HPLC analysis method ver. 1.0 was applied) was not confirmed. Therefore, Capto MMC was applied.

## (6-3-3-1) Experimental method

In the case of the MMC resin, for the binding condition, a process was conducted at low pH to induce a CEX-mode operation, and usually, an elution method is used by changing the pH to neutral and giving pH and salt gradients (Reference Document 6.2). According to the experiment, the pH of an EQ buffer was changed to 4.5 to 5.0, and the pH of an elution buffer was changed to 4.5 to 7.5, confirming that, as the elution pH increased, a peak area became smaller, and the resolution of a main impurity and a target protein decreased. Accordingly, it was determined that the Capto MMC condition is suitable to be performed at low pH. Therefore, in the low pH (EQ, elution buffer, pH 4.5) condition, the process was conducted under the conditions in Table 73.

[Table 73]

| Items | | Process conditions |
|---|---|---|
| Resin | | Capto MMC |
| Column | | LRC10 (ID 1.0 cm, Height 15.0 cm), Column volume (11.8 mL) |
| Flow rate | | 200 cm/hr (2.61 mL/min) |
| Sample preparation | | Dilution with Equilibration by equivalent volume of AEXII sample |
| Process | Equilibration | 50 mM Sodium Acetate, pH4.5 (2 CV) |
| | Sample loading | AEXII sample adjusted pH and conductivity (6.1 mg Gly-Tβ4) |
| | Unbound wash | 50 mM Sodium Acetate, pH4.5 (5 CV) |
| | Elution | 50 mM Sodium Acetate, 1 M NaCl, pH 4.5 (15 CV, 0 ~ 100 %B Linear gradient) |
| | Strip | 2 M NaCl (3 CV) |
| | CIP | 0.5 M NaOH (3 CV) |
| | Strip | 2 M NaCl (2 CV) |
| | Equilibration | 50 mM Sodium Acetate, pH4.5 (3 CV) |
| MMC process conditions for 4th column | | |

The fractionation of an elution pool was performed to carry out RP-HPLC analysis, and purities and yields were confirmed by collecting the pools having a purity of 98% or more.

### (6-3-3-2) Result of experiment

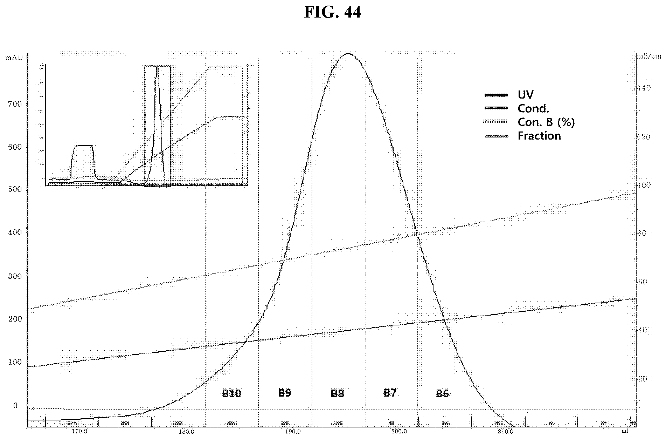

The chromatograms for Capto MMC are shown in FIG. 37. In the elution step, the formation of a single peak was confirmed, and the RP-HPLC (ver 1.0) result for each fraction is shown in FIG. 44. As a result, in the fractions in the front of the peak, the main impurity peak was highly confirmed, whereas almost no main impurity peaks were found in the fractions in the middle to back of the peak.

Referring to the RP-HPLC purity analysis result by eluted fraction, it can be confirmed that the main impurity peaks are concentrated in the front fractions. In pooling of the B8, B7 and B6 fractions, 99.6% purity was able to be ensured at 70% step yield (FIG. 74).

[Table 74]

| Sample | Conc. (mg/mL) | Volume (mL) | Amounts (mg) | Purity (%) | Yield (%) |
|---|---|---|---|---|---|
| Capto MMC Loading Sample | 0.15 | 40 | 6.1 | - | - |
| B10 Fraction | 0.05 | 5 | 0.27 | 84.3 | - |
| B9 Fraction | 0.14 | 5 | 0.69 | 92.6 | - |
| B8 Fraction | 0.35 | 5 | 1.74 | 98.7 | - |
| B7 Fraction | 0.35 | 5 | 1.74 | 99.8 | - |
| B6 Fraction | 0.16 | 5 | 0.77 | 99.4 | - |
| B8~B6 Fraction pooling | 0.29 | 15 | 4.3 | 99.6 | 70.5 |

Yield for Capto MMC process

**(6-4) 4<sup>th</sup> process optimization study 1**

**(6-4-1) Purpose of experiment**

A step elution condition optimization experiment for improving a step yield was conducted.

**(6-4-2) Experimental method**

Target elution conditions by step were confirmed, and it can be confirmed that the eluted condition is elution with 200 mM NaCl or more. Accordingly, a washing step condition was to be confirmed by applying 185 mM NaCl, which is lower than the salt concentration at which a target can be eluted, and the basic purification conditions are shown in Table 73 of (6-3-3-1), but the NaCl concentration of the elution buffer was changed to 400 mM. In addition, process reproducibility when loading amounts were applied under the confirmed conditions was to be confirmed.

**(6-4-3) Result of experiment**

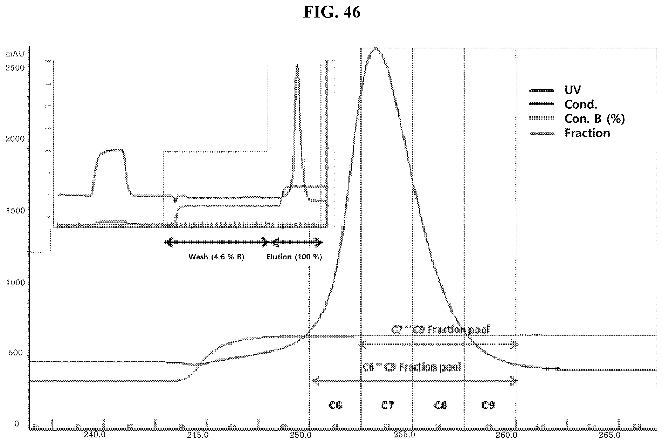

The chromatogram obtained by applying 185 mM NaCl (conductivity 20.7 to 21.2 mS/cm) in the washing step is shown in FIG. 46. After loading at 0.5 mg/mL of resin, the elution step fractions (C6-C9) were pooled into two types to perform RP-HPLC. As a result, it was confirmed that the purities are 98.6% (C6-C9 pool) and 99.0% (C7-C9 pool), which are 98% or more, and the yield is 78.7% in the C6-C9 fraction pool (Table 75).

[Table 75]

| Sample | Conc. (mg/mL) | Volume (mL) | Amounts (mg) | Purity (%) | Yield (%) |
|---|---|---|---|---|---|
| Capto MMC Loading sample | 0.15 | 40 | 6.1 | - | - |
| C6~C9 Fraction pool | 0.24 | 20 | 4.8 | 98.6 | 78.6 |
| C7~C9 Fraction pool | 0.27 | 15 | 4.1 | 99.0 | 67.3 |

Step yield of MMC step elution with load amounts of 0.5mg/mL of resin)

[Table 76]

| Sample | Conc. (mg/mL) | Volume (mL) | Amounts (mg) | Purity (%) | Yield (%) |
|---|---|---|---|---|---|
| Capto MMC Loading sample | 0.3 | 159 | 47.8 | - | - |
| Wash | 0.35 | 80.0 | 28.3 | 99.6 | 59.2 |
| Elution | 0.43 | 35 | 15.3 | 99.7 | 32.0 |

Step yield of MMC step elution with load amounts of 4.0mg/mL of resin
However, when 47.8 mg of Gly-Tβ4 was loaded (4.0 mg/mL of resin), most of the target protein flowed out in the washing step, and the yield was confirmed to be 32% (Table 76), so that it was impossible to be applied to the process.

**(6-5) 4<sup>th</sup> process optimization study 2**

**(6-5-1) Purpose of experiment**

Under the condition of a loading amount of 3 mg Gly-Tβ4 / mL of resin, which is higher than 0.5 mg (conventional amount) and lower than 4.0 mg, purities and step yields according to the NaCl concentration conditions (100, 120 and 140 mM) of the washing step were confirmed.

**(6-5-2) Experimental method**

An experimental method is the same as specified in Table 77. After the process, samples were analyzed by RP-HPLC (ver 2.0), and according to the analysis result, the purities and step yields of the process samples were confirmed.

[Table 77]

| Items | | Process conditions |
|---|---|---|
| Resin | | Capto MMC |
| Column | | LRC10 (ID 1.0 cm, Height 15.0 cm), Column volume (11.8 mL) |
| Flow rate | | 200 cm/hr (2.61 mL/min) |
| Sample p preparation | | Adjust pH with 1M Acetic acid (do not dilution) |
| Process | Equilibration | 20 mM Sodium Acetate, pH4.5 (2 CV) |
| | Sample loading | AEXII sample adjusted pH (34 mg Gly-Tβ4) |
| | Unbound wash | 20 mM Sodium Acetate, pH4.5 (5 CV) |
| | Washing | 20 mM Sodium Acetate, 100 mM, 120 mM 140 mM NaCl, pH 4.5 (20 CV) |
| | Elution | 50 mM Sodium Acetate, 500 mM NaCl, pH 4.5 (5 CV) |
| | Strip | 2 M NaCl (3 CV) |
| | CIP | 0.5 M NaOH (3 CV) |
| | Strip | 2 M NaCl (2 CV) |
| | Equilibration | 20 mM Sodium Acetate, pH4.5 (3 CV) |
| MMC process conditions with loading of 3 mg/mL resin | | |

**(6-5-3) Result of experiment**

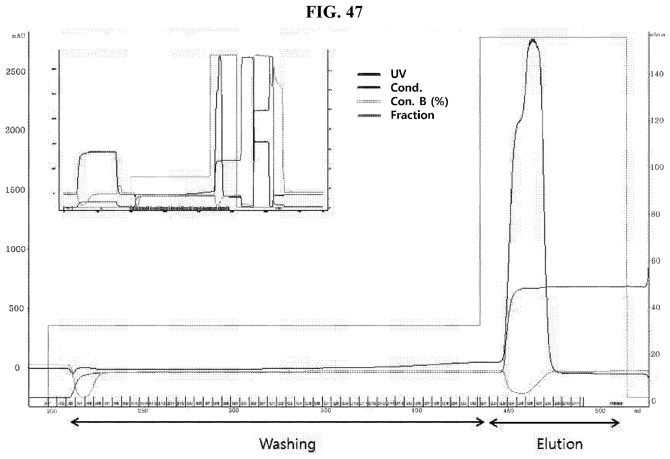

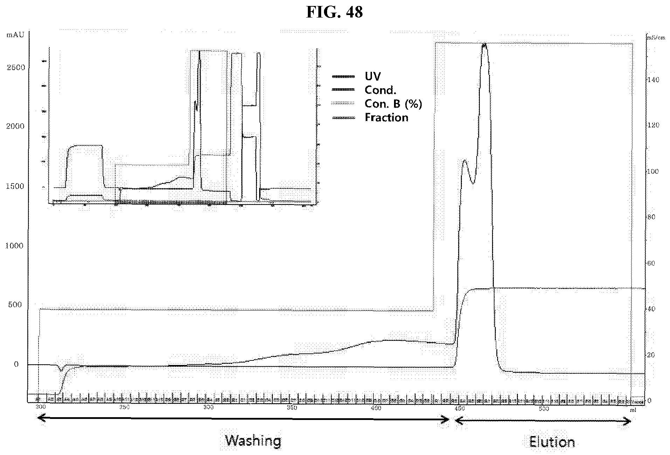

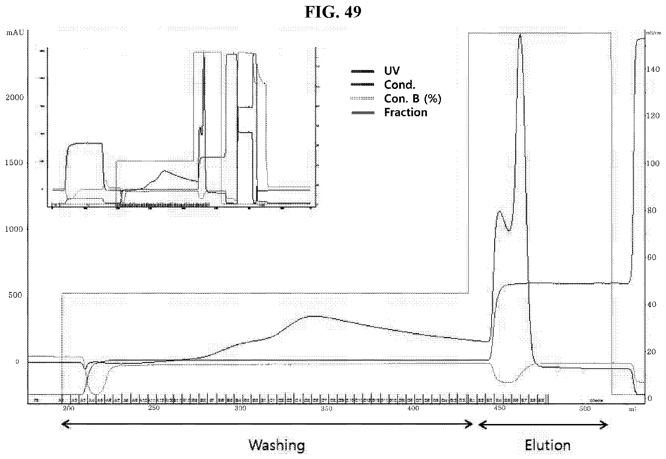

The chromatograms according to NaCl concentrations in a washing step are shown in FIGS. 47, 48 and 49. In the elution step, it was confirmed that the peak is split into two. It was assumed that elution is delayed by the decrease in pH due to a salt in an elution buffer. The two split elution peaks were fractioned and analyzed, followed by pooling and then reanalysis (Table 78).

[Table 78]

| Sample | | Conc. (mg/mL) | Volume (mL) | Amounts (mg) | Purity (%) | Yield (%) |
|---|---|---|---|---|---|---|
| 100mM NaCl | LS | 0.3 | 115 | 34.6 | - | |
| | Wash | - | - | - | - | |
| | Elution pool | 1.32 | 25 | 32.9 | 93.7 | **95.0** |
| 120mM NaCl | LS | 0.3 | 116 | 34.7 | - | |
| | Wash | 0.05 | 186 | 9.7 | - | **27.9** |
| | Elution pool | 0.85 | 30 | 25.6 | 99.3 | **73.7** |
| 140mM NaCl | LS | 0.3 | 113 | 33.9 | - | **-** |
| | Wash | 0.09 | 165 | 15.3 | - | **45.0** |
| | Elution pool | 0.54 | 30 | 16.1 | 99.4 | **47.4** |

Purity and Step yield of MMC with increasing NaCl for washing

When washing was conducted with 100 mM NaCl, it was confirmed that the yield was high at 95%, but the mean purity in an elution pool was approximately 93.7%, which was insufficient for removing impurities. When washing was conducted with 140 mM NaCl, it was confirmed that the purity was 99% or more, but the yield was very low at 47%. Accordingly, the NaCl concentration in the washing step, which is the most suitable in terms of a purity and an yield, was confirmed to be 120 mM, and here, it was confirmed that the yield was 74% and the purity was 99% or more.

## (6-6) 4th process dynamic binding capacity (DBC) test

### (6-6-1) Purpose of experiment

A DBC of MMC with respect to Gly-Tβ4 was to be confirmed.

### (6-6-2) Experimental method

An experimental method was conducted as specified in Table 79, and all binding proteins without a washing step were eluted at one time, and quantified by RP-HPLC.

[Table 79]

| Items | | Process conditions |
|---|---|---|
| Resin | | Capto MMC |
| Column | | LRC10 (ID 1.0 cm, Height 1.2 cm), Column volume (1 mL) |
| Flow rate | | 76.4 cm/hr (1.0 mL/min) |
| Sample preparation | | Adjust pH with Capto MMC Equilibration buffer |
| Process | Equilibration | 20 mM Sodium Acetate, pH4.5 (10 CV) |
| | Sample loading | AEXII sample adjusted pH (26.4 mg Gly-Tβ4) |
| | Unbound wash | 20 mM Sodium Acetate, pH4.5 (50 CV) |
| | Elution | 50 mM Sodium Acetate, 500 mM NaCl, pH 4.5 (20 CV) |
| | Strip | 2 M NaCl (10 CV) |
| | CIP | 0.5 M NaOH (10 CV) |
| | Strip | 2 M NaCl (10 CV) |
| | Equilibration | 20 mM Sodium Acetate, pH4.5 (10 CV) |
| Capto MMC process conditions for DBC test | | |

### (6-6-3) Result of experiment

The RP-HPLC result for the MMC process samples obtained by overloading in1 mL of resin is shown in Table 80, and the DBC of Capto MMC with respect to Gly-Tβ4 was confirmed to be 9.6 mg/mL. This result has a difference from the previous experiment result, and that is, in the 4.0 mg/mL of resin condition, most of the protein was present in wash fractions. Accordingly, a condition difference between the two experiments (flow rate 200 vs 76 cm/hr) was suspected.

[Table 80]

| Items | Condition & Results | | | | |
|---|---|---|---|---|---|
| Loading sample | AEXII Eluted pool (Gly-Tβ4 26.4 mg, volume 160mL) | | | | |
| Eluted pool | Conc. (mg/mL) | Volume (mL) | Amounts (mg) | Resin volume (mL) | DBC (mg/mL) |
| | 0.64 | 15 | 9.6 | 1.0 | 9.6 |

DBC for Capto MMC process

**(6-7) Summary of Capto MMC Process**

[Table 81]

| Items | Process conditions | |
|---|---|---|
| Resin | Capto MMC | |
| Dynamic binding capacity | 9.6 mg/mL (Max DBC) | |
| Equilibration buffer | 20mM Sodium Acetate, pH 4.5 | |
| Wash buffer | 20mM Sodium Acetate, 120mM NaCl, pH 4.5 | |
| Elution buffer | 20mM Sodium Acetate, 500mM NaCl, pH4.5 | |
| Process temperature | 15~25°C | |
| Linear flow rate* | 76 or 200 cm/hr (RT 6min) | |
| Loading sample condition | pH 4.5 $\pm$ 0.1 (adjustment with Acetic acid, but do not dilution) | |
| Process | Equilibration | 2CV |
| | Sample Loading | Sample |
| | Unbound Wash (with Equilibration buffer) | 5CV |
| | Wash | 20CV |
| | Elution | 5CV |
| | Strip (2M NaCl) | 5CV |
| | CIP (0.5M NaOH) | 4CV |
| | Strip (2M NaCl) | 2CV |
| | DW | 3CV |
| | Storage (20% Ethanol) | 3CV |
| Elution pooling criteria | Start point: from elution step<br>End point : UV$_{214nm}$ Value $\leq$ 100mAU | |
| * to be more studied later<br>Summary for Capto MMC process | | |

**Example (7) Process study**

**(7-1) Background**

The conductivity condition of a load sample and the NaCl concentration in an elution buffer in an AEXI process were changed, and the AEXII process was changed to a bind-elute mode, and a process using the Nuvia HR-S resin as a 4th column was determined as the final process. In addition, the purity criterion of the final purified extract was confirmed to be 97% or more (in RP-HPLC). Accordingly, a task of reproducing the process in small-scale experiments and scaling up the process was conducted.

[Table 82]

| Items | | Process conditions | |
|---|---|---|---|
| | | Binex | Huons |
| AEXI | Resin | Fractogel EMD TMAE(M) | |
| | Equilibration buffer | 20 mM Tris-HCl, 50 mM NaCl, pH 8.0 | 20mM Tris-HCl, pH 8.0 |
| | Elution buffer* | 20 mM Tris-HCl, 200 mM NaCl, pH 8.0 | 20 mM Tris-HCl, 300 mM NaCl, pH 8.0 |
| | Linear flow rate | 200 cm/hr (RT 6min) | |
| | Loading sample condition* | pH 8.0 ± 0.1 Conductivity max 8.0 mS/cm | pH 8.0 ± 0.1 Conductivity 2.0 ± 0.2 mS/cm |
| | Elution pooling criteria | Start point: $UV_{280nm}$ Value ≥ 100 mAU End point: $UV_{280nm}$ Value ≤ 100 mAU | |
| Affinity | | **Same as above conditions** | |
| Thrombin Reaction | | **Same as above conditions** | |
| AEXII | Resin | Fractogel EMD TMAE(M) | |
| | Mode* | F/T | B/E |
| | Equilibration Buffer | 20 mM Tris-HCl, 50 mM NaCl, pH 8.0 | 20 mM Tris-HCl, pH 8.0 |
| | Elution Buffer* | N/A | 20 mM Tris-HCl, 80 mM NaCl, pH 8.0 |
| | Linear Flow Rate | 200 cm/hr (RT 6min) | |
| | Loading Sample Condition* | pH 8.0 ± 0.1 Conductivity < 6.5 mS/cm | pH 8.0 ± 0.1 conductivity <2 mS/cm <10 Dilution with DW> |
| | Elution pooling criteria | Start point: $UV_{214nm}$ Value ≥ 50 mAU End point: $UV_{214nm}$ Value ≤ 50 mAU | N/A |

(continued)

| Items | | Process conditions | |
|---|---|---|---|
| | | Binex | Huons |
| 4th Column | Resin* | Capto MMC | Nuvia HR-S |
| | Mode | B/E | B/E |
| | Equilibration Buffer | 20mM Acetate, pH 4.5 | |
| | Wash | 20 mM Acetate, 120 mM NaCl, pH 4.5 | 20 mM Acetate, 100 mM NaCl, pH 4.5 |
| | Elution Buffer | 20 mM Acetate, 500 mM NaCl, pH 4.5 | 20 mM Acetate, 180 mM NaCl, pH 4.5 |
| | Linear Flow rate | To be more studied(76 or 200 cm/hr) | 200 cm/hr (R.T. 6min) |
| | Loading Sample Condition* | pH 4.5 $\pm$ 0.1 (adjust only pH) | pH 4.5 $\pm$ 0.1 Conductivity 2.0 $\pm$ 0.2 mS/cm |
| | Elution pooling criteria | Start point: from elution step End point: $UV_{214nm}$ Value $\leq$ 100 mAU | N/A |

Conditions for comparison DSP

### (7-2) Sample preparation for 3rd and 4th column process studies

As a sample used in this experiment, the affinity pool specified in (5-2) was used (GST-Tβ4 concentration 6.97 mg/mL), and prepared by initiating thrombin digestion in the manner specified in Table 65 one day before the 3rd process.

### (7-3) 3rd column process study (AEXII - B/E mode)

### (7-3-1) Confirmation of 3rd column process dynamic binding capacity (DBC)

### (7-3-1-1) Purpose of experiment

A DBC for AEXII was to be confirmed. However, the experiment was performed by changing a flow rate from 200 cm/hr (conventional) to 153 cm/hr.

### (7-3-1-2) Experimental method

An experimental method was conducted as in Table 83, and after overloading a sample (5 mg/mL of resin) in a column, an eluted fraction was quantified by SEC-HPLC to confirm the amount of eluted Gly-Tβ4.

[Table 83]

| Items | Process conditions |
|---|---|
| Resin | Fractogel EMD TMAE(M) |
| Column | LRC10 (ID 1.0 cm, Height 2.5 cm), Column volume (2 mL) |
| Flow rate | 153 cm/hr (2 mL/min) |
| Sample preparation | After thrombin digestion, Adjust pH and conductivity (pH 8.0, conductivity < 2mS/cm - with DW) |

(continued)

| Items | | Process conditions |
|---|---|---|
| Process | Equilibration | 20 mM Tris-HCl, pH8.0 (10 CV) |
| | Sample loading | Thrombin digestion sample adjusted pH and conductivity <Loading amounts 10.5 mg Gly-Tβ4> |
| | Unbound wash | 20 mM Tris-HCl, pH 8.0 (50 CV) |
| | Elution | 20 mM Tris-HCl, 80 mM NaCl, pH 8.0 (20 CV) |
| | Strip | 2 M NaCl (10 CV) |
| | CIP | 0.5 M NaOH (10 CV) |
| | Strip | 2 M NaCl (10 CV) |
| | Equilibration | 20 mM Tris-HCl, pH 8.0 (10 CV) |
| AEXII process conditions for DBC test | | |

**(7-3-1-3) Result of experiment**

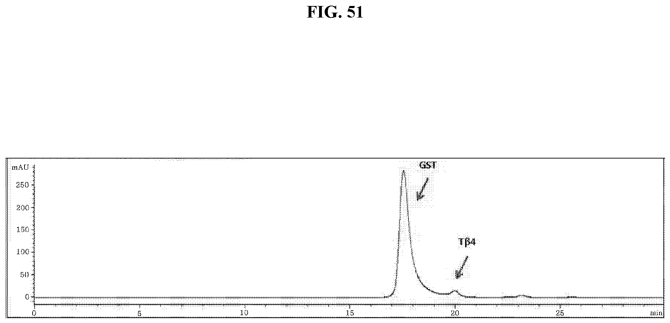

The SEC-HPLC result for the elution pool obtained by being overloaded in 2 mL of resin is shown in FIG. 51. When the elution pool was overloaded in AEXII, GST was also found, and when AEXII had the following conditions, the Gly-Tβ4 DBC attached to AEXII was confirmed to be 1.7 mg Gly-Tβ4/mL (Table 84). However, when the flow rate was changed from 153 cm/hr to 200 cm/hr, additional experiments were required.

[Table 84]

| Items | Condition & Results | | | | |
|---|---|---|---|---|---|
| Loading sample | Thrombin digestion Sample (Gly-Tβ4 10.5 mg, volume 100 mL) | | | | |
| Eluted pool | Conc. (mg/mL) | Volume (mL) | Amounts (mg) | Resin volume (mL) | DBC (mg/mL) |
| | 0.048 | 70 | 3.4 | 2 | 1.7 |
| Loading sample | Thrombin digestion Sample (Gly-Tβ4 8.96 mg, volume 85 mL) | | | | |
| Eluted pool | Conc. (mg/mL) | Volume (mL) | Amounts (mg) | Resin volume (mL) | DBC (mg/mL) |
| | 0.049 | 70 | 3.4 | 2 | 1.7 |

DBC for AEXII process

**(7-3-2) 3rd column process reproducibility experiment**

**(7-3-2-1) Purpose of experiment**

Reproducibility (step yield: within 80%, purity: RP-HPLC 90 to 96% level) was to be confirmed by applying only approximately 90% to the set DBC and increasing a flow rate to 200 cm/hr.

**(7-3-2-2) Experimental method**

An experimental method for the 3rd process was conducted as shown in Table 85 below, and after the process was completed, quantification was performed through SEC-HPLC, followed by purity analysis through RP-HPLC.

[Table 85]

| Items | | Process conditions |
|---|---|---|
| Resin | | Fractogel EMD TMAE(M) |
| Column | | XK16 (ID 1.6 cm, Height 20 cm), Column volume (40 mL) |
| Flow rate | | 200 cm/hr (6.67 mL/min) |
| Sample preparation | | After thrombin digestion, Adjust pH and conductivity (pH 8.0, conductivity < 1mS/cm - with DW) |
| Process | Equilibration | 20 mM Tris-HCl, pH8.0 (2 CV) |
| | Sample loading | AEXII sample adjusted pH and conductivity |
| | Unbound wash | 20 mM Tris-HCl, pH 8.0 (5 CV) |
| | Elution | 20 mM Tris-HCl, 80 mM NaCl, pH 8.0 (5 CV) |
| | Strip | 2 M NaCl (4 CV) |
| | CIP | 0.5 M NaOH (3 CV) |
| | Strip | 2 M NaCl (2 CV) |
| | Equilibration | 20 mM Tris-HCl, pH 8.0 (3 CV) |
| Elution criteria | | Start point : UV$_{(214nm)}$ ≥ 50 mAU<br>End point: UV$_{(214nm)}$ ≤ 50 mAU |

AEXII process conditions

### (7-3-2-3) Result of experiment

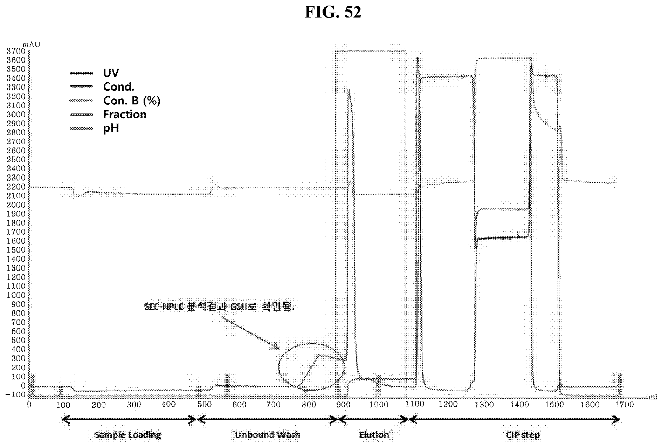

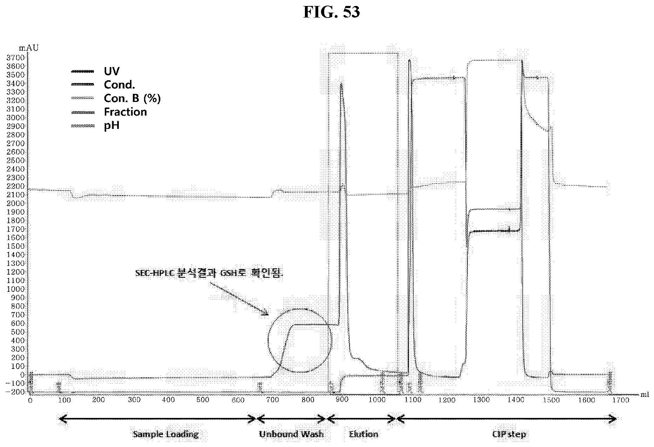

The chromatograms obtained by loading 1.0 mg and 1.5 mg Gly-Tβ4 per mL of resin are shown in FIGS. 52 and 53, respectively.

Before elution, a high UV peak was found, and as a result of analyzing the peak, it was confirmed that there was no target protein, and the peak was caused by impurities (GSH). The yields and purities for the processes were confirmed as shown in Table 86.

[Table 86]

| Sample | | Cone. (mg/mL) | Volume (mL) | Amounts (mg) | Purity (%) | Yield (%) |
|---|---|---|---|---|---|---|
| 1mg Gly-Tβ4 / Resin mL | Loading sample | 0.11 | 395 | 41.7 | - | - |
| | Elution | 0.29 | 113 | 33.0 | 93.6 | 79.1 |
| 1.5mg Gly-Tβ4 / Resin mL | Loading sample | 0.11 | 579 | 61.4 | - | |
| | Elution | 0.34 | 146 | 49.1 | 92.2 | 80.0 |

Quality and quantity profile for AEXII process

No significant differences in yield and purity with respect to loading amounts were observed. Therefore, reproducibility for the DBC and conditions for AEXII was able to be confirmed.

### (7-3-3) Experiment for reducing volume of 3rd process loading sample

### (7-3-3-1) Purpose of experiment

It was intended to confirm the effect of 4-fold dilution for reducing the volume of a loading sample that increased when the conventional 10-fold dilution factor was applied according to a change to a B/E mode.

**(7-3-3-2) Experimental method**

An experimental method was conducted in the same manner as specified in Table 85, and a loading sample was diluted 4-fold with DW to maintain a conductivity at 2 to 3 mS/cm. The sample after the process was quantified by SEC-HPLC, and a purity was analyzed by RP-HPLC.

**(7-3-3-3) Result of experiment**

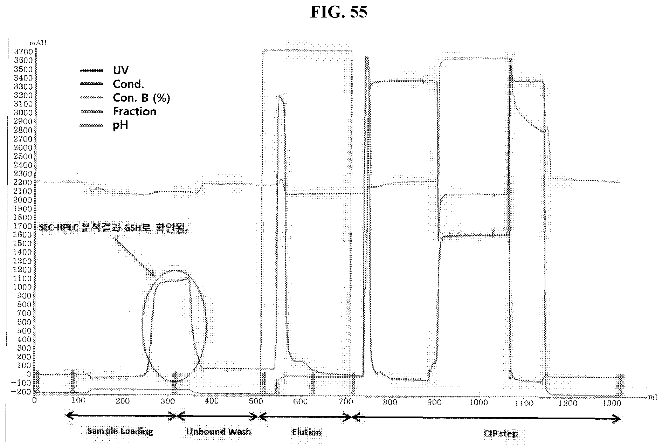

The chromatogram obtained by performing the process with a change in conductivity of a loading sample is shown in FIG. 55.

[Table 87]

| Sample | | Cone. (mg/mL) | Volume (mL) | Amounts (mg) | Purity (%) | **Yield (%)** |
|---|---|---|---|---|---|---|
| 1mg Gly-Tβ4 / Resin mL | Loading sample | 0.27 | 94 | 24.9 | - | **-** |
| | Elution | 0.26 | 76 | 19.5 | 93.1 | **78.5** |
| 1.5mg Gly-Tβ4 / Resin mL | Loading sample | 0.28 | 234 | 65.4 | - | |
| | Elution | 0.40 | 125 | 49.8 | 90.6 | **76.1** |

Purity and yield of AEXII with increased conductivity of loading sample

Due to the decrease in dilution factor of a loading sample, the conductivity was increased (1mg Gly-Tβ4 / resin mL Loading sample conductivity 1.16 -> 2.76 mS/cm, 1.5mg Gly-Tβ4 / resin mL Loading sample conductivity 1.13 -> 2.80 mS/cm), but there was no significant difference in yield and purity (Table 87).

**(7-3-4) 3rd process hold time study**

**(7-3-4-1) Purpose of experiment**

To ensure the storage conditions and period that can be applied in GMP production, in an AEXII process, eluted fraction samples were stored for 7 days in cold room (2 to 8 °C) and room temperature (18 to 22 °C) conditions, and then the change in purity was confirmed.

**(7-3-4-2) Experimental method**

In an experimental method, in a 5L scale process, a 3rd process eluted pool was subjected to sterile filtration, divided into microtubes by 300 uL, and stored for 7 days in cold room and room temperature conditions. Here, at the end of the storage period, samples were stored at -70 °C to completely freeze, and one day after the end of the storage period, all of the samples were simultaneously thawed, followed by RP-HPLC.

**(7-3-4-3) Result of experiment**

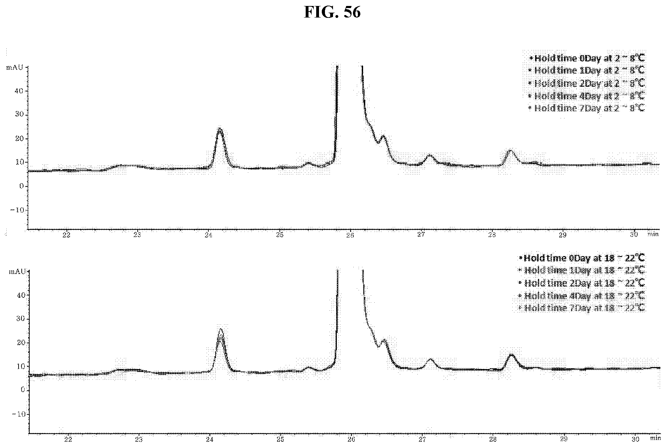

The chromatogram obtained by the test in each storage condition (room temperature or cold room) and RP-HPLC is shown in FIG. 56, and the table exhibiting a purity and an area for the main peak is shown in Table 88.
In terms of the stability criteria of a process sample, %CV of the area of the main peak with the lapse of time is confirmed to be 2.0 or less, compared with the system suitability criteria (%CV $\leq$ 2.0%) according to the RP-HPLC of (2-3), determining that there is no change. Therefore, it was confirmed that the 3rd process samples are stable for 7 days in the cold room (2 to 8 °C) or room temperature (18 to 22 °C) storage condition.

[Table 88]

| Sample | Hold time at 2~8°C | | Hold time at 18~22°C | |
|---|---|---|---|---|
| | Main peak Area | Purity (%) | Main peak Area | Purity (%) |
| 0day | 8,623 | 96.3 | 8,861 | 96.3 |
| 1day | 9,014 | 95.9 | 9,077 | 95.8 |

(continued)

| Sample | Hold time at 2~8°C | | Hold time at 18~22°C | |
|---|---|---|---|---|
| | Main peak Area | Purity (%) | Main peak Area | Purity (%) |
| 2day | 8,846 | 95.9 | 9,047 | 96.4 |
| 4day | 8,930 | 96.1 | 8,992 | 96.0 |
| 7day | 9,117 | 96.0 | 9,048 | 95.5 |
| %CV | 1.9 | 0.2 | 0.9 | 0.3 |

RP-HPLC analysis for AEXII hold time study

### (7-3-5) AEXII process summary

[Table 89]

| Items | Process conditions | |
|---|---|---|
| Resin | Fractogel EMD TMAE(M) | |
| Dynamic binding capacity | Not more than 1.8 mg/mL | |
| Equilibration buffer | 20 mM Tris-HCl,, pH 8.0 | |
| Elution buffer | 20 mM Tris-HCl, 80 mM NaCl, pH 8.0 | |
| Process temperature | 15-25 °C | |
| Linear flow rate | 200 cm/hr | |
| Loading sample condition | Thrombin digestion sample adjust pH and conductivity (pH 8.0, conductivity, 2~3mS/cm with DW) | |
| Process | Equilibration | 2 CV |
| | Sample loading | Sample |
| | Unbound wash (with Equilibration buffer) | 5 CV |
| | Elution | 5 CV |
| | Strip (2 M NaCl) | 4 CV |
| | CIP (0.5 M NaOH) | 3 CV |
| | Strip (2 M NaCl) | 2 CV |
| | Equilibration | 3 CV |
| | DW | 2 CV |
| | Storage (20 % Ethanol, 150 mM NaCl) | 3 CV |
| Elution pooling criteria | Start point: $UV_{21.4nm}$ Value $\geq$ 200 mAU<br>End point: $UV_{214nm}$ Value $\leq$ 200 mAU | |
| Hold time for AEXII eluted pool | Stable at 2 ~ 8°C and 18 ~ 22°C for up to 7 days | |

Summary of AEXII process

### (7-4) 4th column process study

### (7-4-1) Experiment of confirming condition reproducibility

Loading samples to be used in a 4th column reproducibility experiment were prepared in a B/E mode by applying

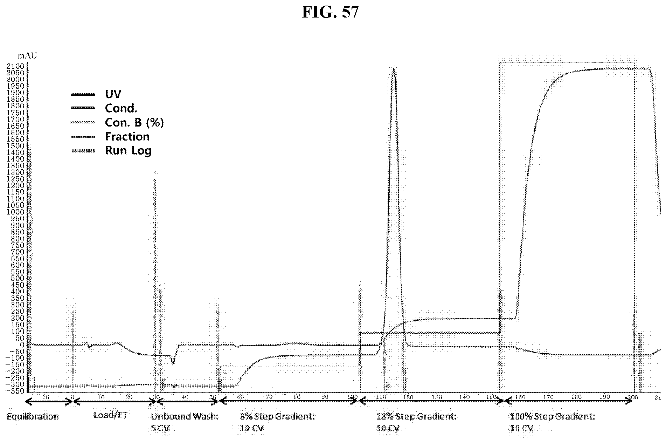

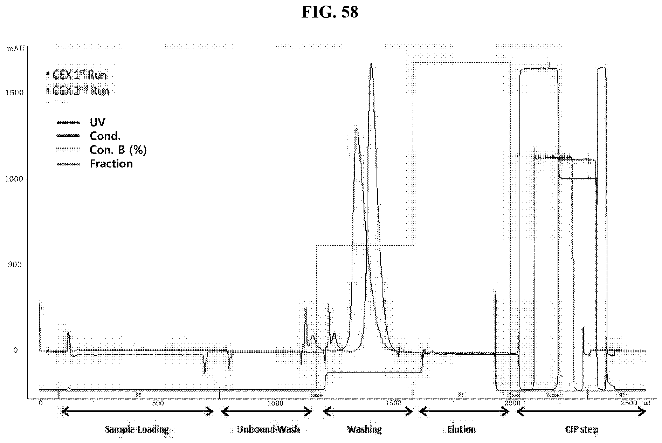

2<sup>nd</sup> 50L verification run criteria (50 mAU ~ 50 mAU) as elution criteria for AEXII, and other than that, all 4<sup>th</sup> column conditions are described in Table 90. The experiment was repeated twice (FIGS. 57 and 58).

[Table 90]

| Items | | Process conditions |
|---|---|---|
| Resin | | Nuvia HR-S |
| Column | | XK16 (ID 1.6 cm, Height 20 cm), Column volume (40 mL) |
| Flow rate | | 200 cm/hr (6.67 mL/min) |
| Sample preparation | | AEXII elution pool adjust pH and conductivity (pH 4.5, conductivity, 1-2 mS/cm - with DW) |
| Process | Equilibration | 20 mM Sodium Acetate anhydrous. pH 4.5 (2 CV) |
| | Sample loading | AEXII eluted pool adjusted pH and conductivity <Loading amounts 45 mg Gly-Tβ4> |
| | Unbound wash | 20 mM Sodium Acetate anhydrous. pH 4.5 (5 CV) |
| | Wash | 20 mM Sodium Acetate anhydrous. 80 mM NaCl, pH 4.5 (10 CV) |
| | Elution | 20 mM Sodium Acetate anhydrous, 180 mM NaCl, pH 8.0 (10 CV) |
| | Strip | 2 M NaCl (4 CV) |
| | CIP | 0.5 M NaOH (4 CV) |
| | Strip | 2 M NaCl (2 CV) |
| | Equilibration | 20 mM Sodium Acetate anhydrous. pH 4.5 (3 CV) |
| CEX process conditions | | |

[Table 91]

| Sample | | Conc. (mg/mL) | Volume (mL) | Load amount (mg/mL of resin) | Conductivity (mS/cm) | pH |
|---|---|---|---|---|---|---|
| CEX Run1 | Loading sample | 0.067 | 680 (17.0 CV) | 1.1 | 1.74 | 4.53 |
| CEX Run2 | Loading Sample | 0.069 | 580 (14.5 CV) | 1.0 | 1.68 | 4.50 |

Loading Sample preparation for CEX feasibility test

As a result of the experiment, a main peak elution position was slightly different from the conventional result (FIGS. 57 and 58). The difference in experiment conditions was examined, and it was confirmed that the conditions were the same. except a resin bed height, and the protein concentration and volume of a loading sample. In the conventional example, the Gly-Tβ4 concentration in the AEXII elution pool was 1 to 1.5 mg/mL, but in the in-house experiment performed with the elution criteria of 50 mAU ~ 50 mAU, the concentration of Gly-Tβ4 in the elution pool was confirmed as 0.4 mg/mL. Accordingly, a loading sample volume increased: for example, the conventional condition was 7.5 CV, and the in-house experiment repeated twice was conducted with 14.5 and 17.0 CV of the samples, respectively. To identify the exact cause, first, the effect of the difference in bed height was to be examined.

### (7-4-2) Effect caused by bed height difference

### (7-4-2-1) Purpose of experiment

It was to be confirmed whether there is a difference in elution position according to the difference in bed height.

### (7-4-2-2) Loading sample preparation

To prepare a sample as similar as possible to the conventionally used loading sample, an elution fraction was obtained by changing the AEXII elution criteria to 100 mAU ~ 172 mAU. The purity and step yield of the elution fraction prepared as described above were confirmed (Table 92). However, the protein concentration was 0.9 mg/mL, which was slightly lower than the conventional case (1 to 1.5 mg/mL).

[Table 92]

| Sample | | Conc. (mg/mL) | Volume (mL) | Amounts (mg) | Purity (%) RP-HPLC | Yield (%) SEC-HPLC |
|---|---|---|---|---|---|---|
| AEXII | Loading sample | 0.28 | 230 | 64.8 | - | - |
| | Elution | 0.90 | 57 | 51.1 | 94.8 | 78.3 |

Quality and quantity profile for AEXII process

### (7-4-2-3) Experimental method

An experimental method was conducted in the same manner as specified in Table 90, experiments were independently conducted using a manually packed XK16 (Bed height 20 cm, CV 40 mL) column and a prepacked column (Bed height 10 cm, CV 5 mL), and the loading for each process was performed according to the conditions shown in Table 93.

The same volume (5.5 CV) of loading sample was applied in both experiments. However, compared to the previous experiment, the volume was approximately 3-fold smaller. While a flow rate was constantly maintained at 200 cm/h, there was a twofold difference in resistance time between the experiments due to the difference in bed height. Compared with the previous experiment, the conductivity of the loading sample slightly increased from 1.7 mS/cm to 2.0 mS/cm. After the process was completed, the purity of the sample was analyzed by RP-HPLC.

[Table 93]

| Bed height | Conc. (mg/mL) | Volume (mL) | Load amount (mg/mL of resin) | Loading Sample Volume(CV) | Conductivity (mS/cm) | pH |
|---|---|---|---|---|---|---|
| Prepack column (10cm) | 0.18 | 27.5 | 1.1 | 5.5 | 2.01 | 4.58 |
| XK 16 columnn (20cm) | 0.18 | 220 | 1.0 | 5.5 | 2.02 | 4.45 |

Loading Sample Volume for bed height study

### (7-4-2-4) Result of experiment

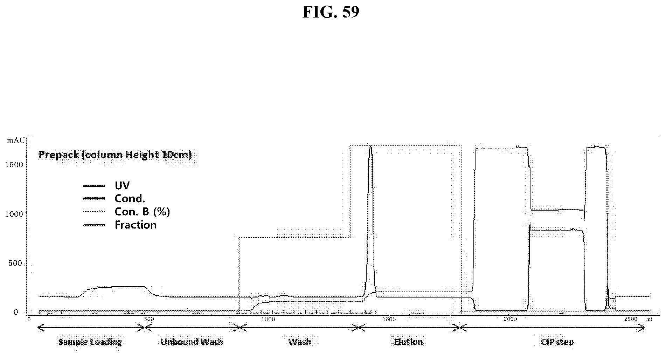

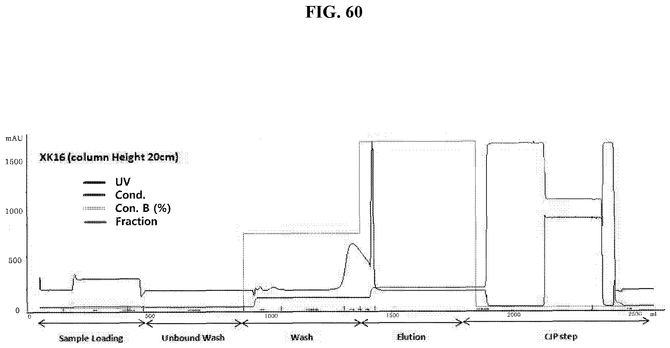

The chromatograms for the experiment processes are shown in FIGS. 59 and 60. From the result of the experiment conducted with a bed height of 10 cm, it was confirmed that a peak was found in the elution step, not the washing step. It was confirmed that this result is similar to the conventional chromatogram (FIG. 56).

The result of the experiment conducted with a bed height of 20 cm shows that elution was initiated at a time point of 8CV in the washing step, and completed in the elution step. However, according to the result of the previous experiment conducted with the same bed height condition, the main peak was fully eluted in the washing step. Such a difference is estimated to be associated with the difference in loading sample volume (14.5 vs 5.5 CV).

[Table 94]

| Sample | | Cone. (mg/mL) | Volume (mL) | Amounts (mg) | Purity (%) | Yield (%) |
|---|---|---|---|---|---|---|
| H 10cm | Loading sample | 0.18 | 27.5 | 5.4 | - | - |
| | Elution | 1.47 | 1.9 | 2.8 | 99.7 | N/A* |
| H 20cm | Loading sample | 0.18 | 220 | 40.9 | - | - |
| | Elution 1 | 0.25 | 93.1 | 23.1 | 99.1 | 56.4 |
| | Elution 2 | 0.69 | 16.2 | 11.2 | 99.3 | 27.3 |
| Sum | | | | | | 83.6 |

* not measured due to the loss of elution sample during process
Quality and quantity profile for CEX column length study

Consequently, to constantly maintain the bed height causing the difference in residence time, a 10 cm condition was selected, and it was confirmed that the same elution position as the conventional result was reproduced under conditions of a flow rate of 200 cm/hr and a loading sample volume of 5.5 CV.

### (7-5) 4th column process reproducibility experiment

### (7-5-1) Preparation of 4th column process sample

To confirm the reproducibility of a 4th column process, an AEXII process was conducted, and the 4th column process was conducted in the same manner as shown in Table 90, except that the pooling criteria was changed to 200 mAU ~ 200 mAU to reduce the volume of a 4th column eluted fraction sample. The 4th process-completed sample was subjected to quantification by SEC-HPLC and purity measurement by RP-HPLC, confirming that there was no difference from the previous result, and the result is shown in Table 95.

[Table 95]

| Sample | | Conc. (mg/mL) | Volume (mL) | Amounts (mg) | Purity (%) | Yield (%) |
|---|---|---|---|---|---|---|
| AEXII | Loading sample | 0.28 | 230 | 64.8 | - | - |
| | Elution | 1.19 | 39 | 46.5 | 94.1 | 71.8 |

Purity and step yield for preparation of loading sample for the study of CEX bed height

### (7-5-2) Purpose of experiment

It was to be confirmed whether the experiment result is reproduced using a manually packed column, rather than a prepacked column, under the 10-cm bed height (XK16 packing) condition.

### (7-5-3) Experimental method

While the experimental method was conducted as shown in Table 96, the volume of a loading sample was 7.0 CV, similar to the previous experiment result of 5.5 CV, and a loading amount was 1.65 mg Gly-T$\beta$4/mL of resin, which is slightly larger than the previous experiment result of 1.1 mg. The process-completed sample was quantified through SEC-HPLC, and its purity was confirmed through RP-HPLC.

[Table 96]

| Items | | Process conditions |
|---|---|---|
| Resin | | Nuvia HR-S |
| Column | | XK16 (ID 1.6 cm, Height 10 cm), Column volume (20 mL) |
| Flow rate | | 200 cm/hr (6.67 mL/min) |
| Sample preparation | | AEXII elution pool adjust pH and conductivity (pH 4.5, conductivity, 2 mS/cm - with DW) |
| Process | Equilibration | 20mM Sodium Acetate anhydrous. pH 4.5 (2 CV) |
| | Sample loading | AEXII eluted pool adjusted pH and conductivity <Loading amounts 32.9 mg Gly-Tβ4> |
| | Unbound wash | 20 mM Sodium Acetate anhydrous. pH 4.5 (5 CV) |
| | Wash | 20 mM Sodium Acetate anhydrous. 80 mM NaCl, pH 4.5 (5 CV) |
| | Elution | 20 mM Sodium Acetate anhydrous, 180 mM NaCl, pH 4.5 (5 CV) |
| | Strip | 2 M NaCl (4 CV) |
| | CIP | 0.5 M NaOH (4 CV) |
| | Strip | 2 M NaCl (2 CV) |
| | Equilibration | 20 mM Sodium Acetate anhydrous. pH 4.5 (3 CV) |
| CEX process conditions | | |

**(7-5-4) Result of experiment**

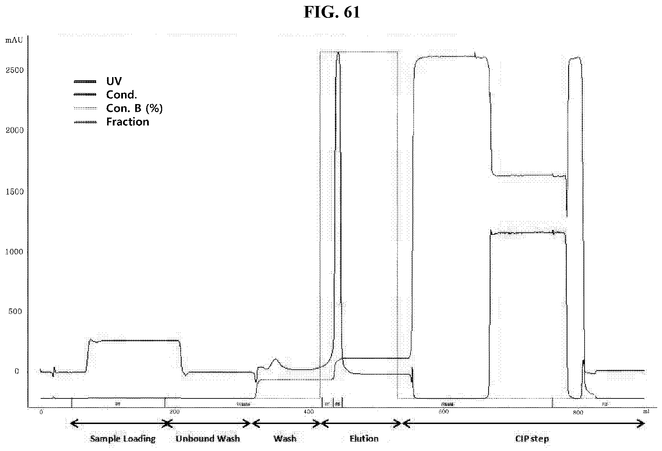

The chromatogram for the process is shown in FIG. 61, and unexpectedly, the main peak began to be eluted from the end of washing step before changing to an elution buffer. It is assumed that the reason that the elution start time was slightly faster than the previous experiment result is due to an increase in loading amount (1.1-->1.7 mg/mL of resin) and an increase in loading volume (5.5-->7.0 CV).

[Table 97]

| Sample | Conc. (mg/mL) | Volume (mL) | Load amount (mg/mL of resin) | Conductivity (mS/cm) | pH |
|---|---|---|---|---|---|
| Loading sample | 0.24 | 139 (7.0 CV) | 1.65 | 1.91 | 4.53 |

Loading Sample preparation for CEX feasibility test

[Table 98]

| Sample | | Conc. (mg/mL) | Volume (mL) | Amounts (mg) | Purity (%) | Yield (%) |
|---|---|---|---|---|---|---|
| H 10cm | Loading sample | 0.24 | 139 | 32.9 | - | - |
| | Elution | 2.21 | 13.5 | 29.9 | 98.8 | 90.9 |

Purity and yield of purified sample for CEX bed height study

As a result of a CEX process, it was confirmed that a step yield was 91%, and a purity was 98.8% (Table 98). The purity was slightly lower than the previous experiment result (99.7%), and the step yield could not be compared due to the absence of a previous result.

### (7-6) 4th column process hold time study

### (7-6-1) Purpose of experiment

To ensure the storage condition and period that can be applied in GMP production, 4th column eluted fraction samples were stored for 7 days in cold room (2 to 8 °C) and room temperature (18 to 22 °C) conditions, and then a change in purity was examined.

### (7-6-2) Experimental method

For the experiment, a 4th process eluted pool was subjected to sterile filtration in a 5L scale process, divided into microtubes by 300 uL, and stored for 7 days in cold room and room temperature conditions. Here, the sample was stored at -70 °C at the end of the storage period to completely freeze, and one day after the end of the storage period, all samples were simultaneously thawed and analyzed through RP-HPLC.

### (7-6-3) Result of experiment

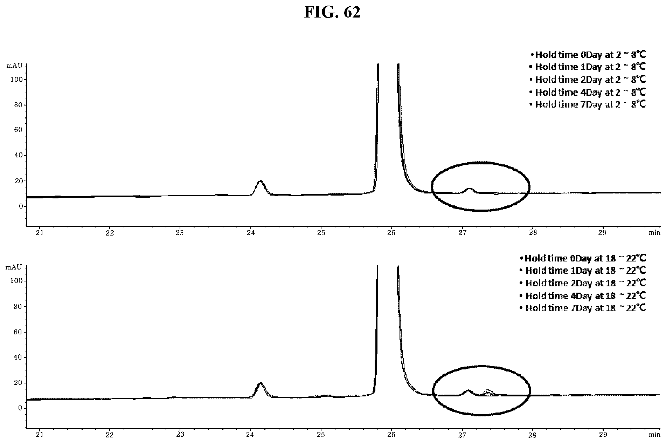

The chromatogram obtained by performing the experiment under each storage condition (room temperature or cold room) and then, RP-HPLC, is shown in FIG. 62, and purities and main peak areas are shown in Table 99. As stability criteria for process samples, the system suitability criteria (%CV ≤ 2.0%) according to RPC-HPLC (HU024-SOP-004) in (2-3) was applied, and even with the passage of time under the cold room and room temperature conditions, the %CV of the main peak area was 2.0 or less, confirming that there was no change. However, when the impurity peak of the part (retention time 27.4 min) indicated by a red circle in the sample stored in a cold room was compared with a day 0 sample, with the passage of time, it was confirmed that the peak area increased (Table 100). Accordingly, it was determined that the 4th process sample is stable in a cold room condition (2 to 8 °C) for 7 days, but is not appropriate for room temperature storage.

[Table 99]

| Sample | Hold time at 2~8°C | | Hold time at 18~22°C | |
|---|---|---|---|---|
| | Main peak Area | Purity (%) | Main peak Area | Purity (%) |
| 0day | 8,982 | 98.4 | 8,707 | 98.4 |
| 1day | 8,720 | 98.4 | 8,704 | 98.1 |
| 2day | 9,078 | 98.4 | 8,987 | 98.0 |
| 4day | 9,028 | 98.3 | 8,677 | 97.9 |
| 7day | 8,745 | 98.3 | 8,703 | 97.6 |
| %CV | 1.7 | 0.05 | 1.3 | 0.3 |

Purity in RP-HPLC analysis for CEX hold time study

[Table 100]

| Sample | Area (mAU) | | | | |
|---|---|---|---|---|---|
| | 24.1min | 25.1min | Main peak (25.9min) | 27.1min | 27.4min |
| 0day | 104.5 | N/D | 8,707.0 | 35.9 | N.D |
| 1day | 110.5 | 19.9 | 8,704.5 | 34.0 | 14.8 |
| 2day | 109.6 | 20.0 | 8,986.8 | 40.4 | 19.0 |
| 4day | 112.4 | 18.1 | 8,676.9 | 35.9 | 30.7 |
| 7day | 114.9 | 16.0 | 8,703.1 | 43.4 | 42.9 |

Impurity analysis in RP-HPLC analysis for CEX eluted pool hold time at room temperature (18 to 22 °C)

**(7-7) CEX process summary**

[Table 101]

| Items | Process conditions | |
|---|---|---|
| Resin | Nuvia HR-S | |
| Dynamic binding capacity | 1.65 mg Gly-T$\beta$4 / mL | |
| Equilibration buffer | 20 mM Sodium acetate anhydrate, pH 4.5 | |
| Wash buffer | 20 mM Sodium acetate anhydrate, 80 mM NaCl, pH 4.5 | |
| Elution buffer | 20 mM Sodium acetate anhydrate, 180 mM NaCl, pH 4.5 | |
| Process temperature | 15~25 °C | |
| Linear flow rate | 200 cm/hr (Bed height 10cm) | |
| Loading sample condition | AEXII eluted sample adjust pH and conductivity (pH 4.5 with acetic acid, conductivity, 2mS/cm with DW) | |
| Process | Equilibration | 2 CV |
| | Sample loading | Sample |
| | Unbound wash (with Equilibration buffer) | 5 CV |
| | Wash | 5 CV |
| | Elution | 5 CV |
| | Strip (2 M NaCl) | 4 CV |
| | CIP (0.5 M NaOH) | 3 CV |
| | Strip (2 M NaCl) | 2 CV |
| | Equilibration | 3 CV |
| | DW | 2 CV |
| | Storage (20 % Ethanol) | 3 CV |
| Elution pooling criteria | Start point : UV$_{214nm}$, Value $\geq$ 200 mAU End point : UV$_{214nm}$ Value $\leq$ 200 mAU | |

(continued)

| Items | Process conditions |
|---|---|
| Hold time for AEXII eluted pool | Stable at 2 ~ 8°C and 18 ~ 22°C for up to 7 days |
| Summary of CEX process | |

**Example (8) Purification of 5L scale culture broth**

**(8-1) Purpose of experiment**

It was to be confirmed whether the result performed at the XK16 size is reproduced with a BPG100 column size or more.

**(8-2) Experimental method**

Fourth column purification was conducted by the method specified in Table 101, and the other process conditions are shown in Table 102. For depth filtration and an AEXI process, process step yields were calculated using a GST-T$\beta$4 standard material (prepared in 1st 50L verification run) by the method described in (2-6), and for thrombin, AEXII and CEX processes, step yields were calculated through SEC-HPLC and RP-HPLC. In addition, each process sample was subjected to endotoxin and HCP analyses according to the methods described in (2-4) and (2-5).

[Table 102]

| Items | | Process conditions |
|---|---|---|
| AEXI | Resin | Fractogel EMD TMAE(M) |
| | Equilibration buffer | 20 mM Tris-HCl, pH 8.0 |
| | Elution buffer | 20 mM Tris-HCl, 300 mM NaCl, pH 8.0 |
| | Linear flow rate | 200 cm/hr (RT 6min) |
| | Loading sample condition | pH 8.0 $\pm$ 0.1 Conductivity 2.0 $\pm$ 0.2 mS/cm |
| | Elution pooling criteria | Start point : UV$_{280nm}$ Value $\geq$ 100 mAU<br>End point : UV$_{290nm}$ Value $\leq$ 100 mAU |
| Affinity | Same of above conditions | |
| Thrombin Reaction | Same of above conditions | |
| AEXII | Resin | Fractogel EMD TMAE(M) |
| | Mode | Bind / Elute mode |
| | Equilibration buffer | 20 mM Tris-HCl, pH 8.0 |
| | Elution buffer | 20 mM Tris-HCl, 80 mM NaCl, pH 8.0 |
| | Linear flow rate | 200 cm/hr (RT 6min) |
| | Loading sample condition | pH 8.0 $\pm$ 0.1<br>conductivity 2 $\pm$ 0.2 mS/cm <5 Dilution with DW> |
| | Elution pooling criteria | Start point : UV$_{214nm}$ Value $\geq$ 200 mAU<br>End point: UV$_{214nm}$ Value $\leq$ 200 mAU |

(continued)

| Items | | Process conditions |
|---|---|---|
| CEX | Resin | Nuvia HR-S |
| | Mode | Cation-Exchange chromatography |
| | Equilibration buffer | 20 mM Acetate, pH 4.5 |
| | Wash | 20 mM Acetate, 80 mM NaCl, pH 4.5 |
| | Elution buffer | 20 mM Acetate, 180 mM NaCl, pH 4.5 |
| | Linear flow rate | 200 cm/hr (RT 3min) |
| | Loading sample condition | pH 4.5 $\pm$ 0.1, Conductivity 2 $\pm$ 0.2 mS/cm (adjust with Acetic acid and DW) <5 Dilution with DW> |
| | Elution pooling criteria | Start point : $UV_{214nm}$ Value $\geq$ 200 mAU End point: $UV_{214nm}$ Value $\leq$ 200 mAU |

5L scale process buffer and sample preparation condition

[Table 103]

| Items | | Process conditions |
|---|---|---|
| Cell Lysis & Recovery | | Cell pellet 1080 g (batch No. 161115-B1, 161122-B2) Microfluidizer : 11,000 psi, 2 pass Continuous centrifugation (9,500 rpm, Pressure 250 kPa, Fee flow 100L/hr) |
| Depth filtration | | C0HC (0.054 m$^2$) + F0HC (0,054 m$^2$) + Opticap XL10 (0.45 $\mu$m) |
| Column controller | | AKTA Pilot |
| AEXI (2cycle) | Resin | Fractogel FMD TMAE(M) |
| | Column | BPG 100 (ID 10 cm, Height 20 $\pm$ 1 cm, column volume(CV) : 1570 mL) |
| | Flow rate | 262 mL/min |
| | Linear flow rate | 200 cm/hr (RT 6min) |
| Affinity (1cycle) | Resin | Glutathione Sepharose 4 Fast Flow |
| | Column | BPG140 (ID 14 cm, Height 15 $\pm$ 1 cm, column volume(CV) : 2308 mL) |
| | Flow rate | 205 mL/min (Sample loading, Unboundwash) 385 mL/min (Elution) |
| | Linear flow rate | 80 cm/hr (Sample loading, Unboundwash) 150 cm/hr (Elution) |
| Thrombin Digestion | Enzyme/Protein ratio | 1 mg GST-T$\beta$4 / 2 unit Thrombin |
| | Incubation time | 14~18 hrs |
| | Incubation temp | 13 ~ 27°C |
| AEXII (1cycle) | Resin | Fractogel EMD TMAE(M) |
| | Column | BPG 100 (ID 10 cm, Height 20 $\pm$ 1 cm, column volume(CV) : 1570 mL) |
| | Flow rate | 262 mL/min |
| | Linear flow rate | 200 cm/hr (RT 6min) |

(continued)

| Items | | Process conditions |
|---|---|---|
| CEX (1cycle) | Resin | Nuvia HR-S |
| | Column | BPG100 (ID 10 cm, Height 10 ± 1 cm, column volume(CV) : 785 mL) |
| | Flow rate | 262 mL/min |
| | Linear flow rate | 200 cm/hr (RT 3min) |

5L scale process conditions

[Table 104]

| Step | Conc. (mg/mL) | Volume (mL) | Load amount (mg/mL of resin) | Conductivity (mS/cm) | pH |
|---|---|---|---|---|---|
| 1st column (AEXI) | 4.34 | 3,650 | 8.15 | 2.02 | 8.09 |
| 2nd column (Affinity) | N/A | | | | |
| 3rd column (AEXII) | 0.21 | 11,536 | 1.48 | 1.79 | 8.05 |
| 4th column (CEX) | 0.33 | 6,925 (8.8 CV) | 2.79 | 2.08 | 4.48 |

Loading Sample preparation for 5L scale process

**(8-3) Result of experiment**

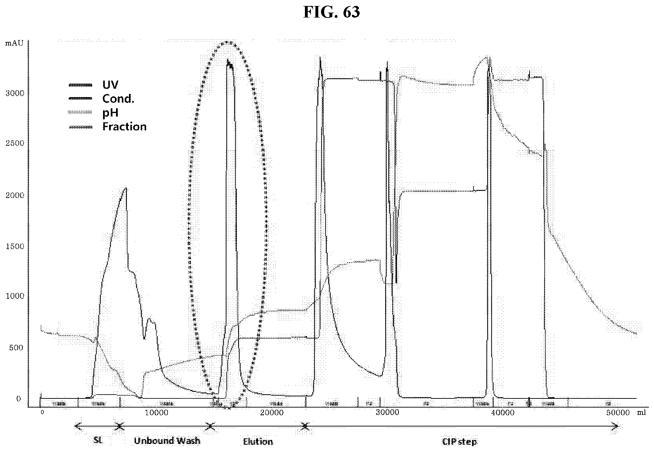

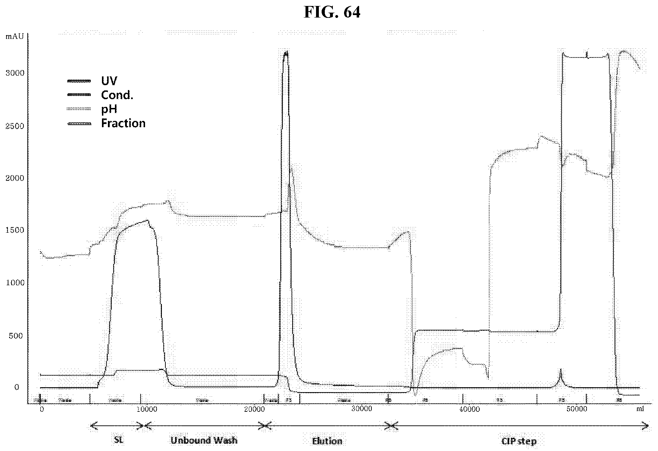

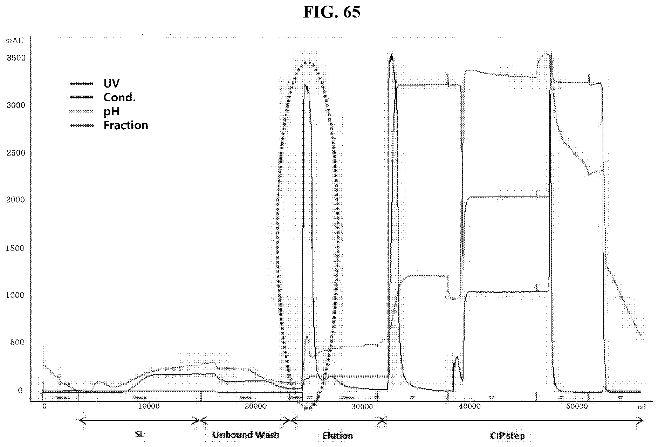

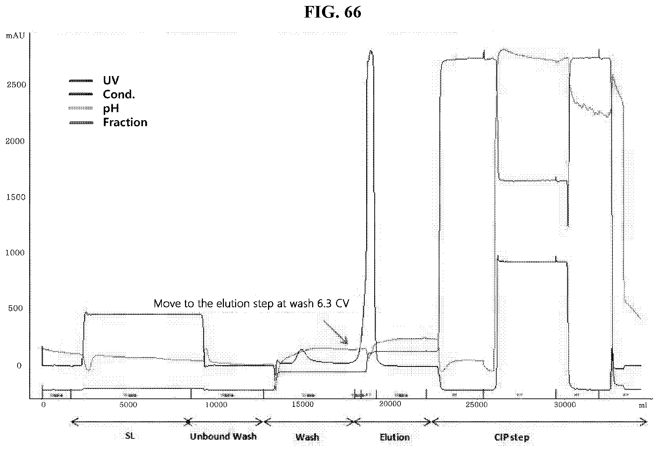

The chromatogram for each process was shown as follows, and it was confirmed that the process was performed without a significant difference from the process development (FIGS. 63, 64 and 65). However, in the previous CEX experiment performed under the XK16 column condition, the main peak eluted when washing progressed at approximately 9 CV or more began to elute from the 6 CV time point (FIG. 66). As described above, it may be reconfirmed that the reason that the elution start time of the main peak became faster is due to an increase in loading amount (1.7-->2.8 mg/mL of resin) and an increase in loading volume (7.0-->8.8 CV). Therefore, to keep a constant elution position, it was determined that 4th column loading sample data should be considered in future GMP production.

[Table 105]

| | | Loading Sample | | | Eluted Pool | | | Yield (%) | Purity (%) |
|---|---|---|---|---|---|---|---|---|---|
| | | Conc. (mg/mL) | Volume (mL) | Amount (mg) | Conc. (mg/mL) | Volume (mL) | Amount (mg) | | |
| Cell breakage* | | | 7,208 | | | 7,180 | | | |
| Recovery | | | 7,180 | | 5.34 | 6,480 | 34,603 | | |
| Depth Filtration/MF | | 5.34 | 6,480 | 34,603 | 4.14 | 8,795 | 36,411 | 105.2 | |
| AEX I | Cycle1 | 4.14 | 3,650 | 15,111 | 4.57 | 2,386 | 10,904 | | |
| | Cycle2 | 4.14 | 3,650 | 15,111 | 3.79 | 2,426 | 9,195 | | |
| Thrombin | | 7.69 | 1,979 | 15,221 | 1.11 | 2,240 | 2,475 | 16.3 | |
| AEX II | | 0.21 | 11,536 | 2,434 | 1.66 | 1,405 | 2,334 | 96.7 | 96.3 |
| CEX | | 0.33 | 6,925 | 2,299 | 2.61 | 875 | 2,286 | 99.4 | 98.4 |
| UF/DF | | 2.61 | 872 | 2,278 | 10.29 | 221 | 2,276 | 99.9 | 98.0 |
| Total Yield (%) | | | | | | | | 7.8 | |

\* Cell Batch No. 161115-B1, 161122-B2

Yield and purity in 5L scale process

[Table 106]

| | Eluted Pool | | | HCP | | | Endotoxin | |
|---|---|---|---|---|---|---|---|---|
| | Conc. (mg/mL) | Volume (mL) | Amount (mg) | Conc. (mg/mL) | HCP Conc. (ng/mL) | HCP (ppm) | Conc. (EU/mL) | Conc. (EU/mg) |
| AEX I | 4.35 | 4,840 | 21,054 | 4.35 | 14,339 | 3,296 | >500000 | |
| Affinity | 7.69 | 1,956 | 15,042 | 7.69 | 1,065 | 138 | 24,102 | 3,134 |
| AEX II | 1.66 | 1,416 | 2,354 | 1.66 | 16 | 10 | 0.065 | 0.04 |
| CEX | 2.61 | 880 | 2,299 | 2.61 | 4 | 1.5 | 0.005 | 0.002 |
| UF/DF | 10.29 | 221 | 2,273 | 10.29 | 21 | 2.0 | 4.55 | 0.44 |

Impurity analysis in 5L scale process

It was confirmed that final yield obtained by a 5L scale process was 7.8%, and the RP-HPLC purity of the 3rd column process eluted solution was 96.3%, which was equal to a small scale experiment result, and the RP-HPLC purity of the 4th column process eluted solution was 98.0%, which also satisfied the standard, 97% or more. However, compared to the previous small scale CEX purification results (99.6% and 98.8%), the purity was relatively low (98.4%), but the step yield was 99.4%, an increase of approximately 8% or more. It is determined that this is caused by the main peak elution time being faster than that of the previous experiment in the 4th column process. Purity was expected to decrease according to an increase in step yield (Table 105). The removal rates of process-derived impurities (HCP and endotoxin) were HCP 97.0% for HCP and 98.0% for endotoxin in the affinity process, compared to the AEXI process sample. After the affinity process, 99.9% or more of endotoxin was removed in the AEXII process, and the endotoxin removal rate was confirmed to be most excellent in the AEXII process. The endotoxin ($\leq$ 0.5 EU/mg) and HCP ($\leq$ 100 ppm) results in the final purification extract also met the criterion (Table 106). Based on the above results, a GMP technical transfer protocol was written.

**(9-1) Purpose of experiment**

Through the purification of a 50L scale culture broth, a sample for non-clinical testing was produced, and 100 vials of a standard product was to be prepared.

**(9-2) Experimental method**

The conditions for a buffer for a 50L verification run are shown in Table 107, and in 4th column process, unlike the washing condition used in the 5L scale process, and NaCl concentration was changed from 80 mM to 60 mM, and the other processes were the same as used in the conventional process. In addition, in the 1st, 2nd, and ,3rd column processes, the flow rate applied in the conventional 50L verification run was applied, and the 4th column process used a BPG200 column, so that the maximum flow rate that can be driven in an AKTA pilot was applied, but the conventional 200 cm/hr condition was not applied. Accordingly, the 150cm/hr condition was applied (manufacturer's instructions and records, batch No HU024-RD1603).

[Table 107]

| Items | | Process conditions |
|---|---|---|
| AEXI (3cycle) | Column scale | BPG 200 (i.d 20 cm, H 15.8 cm), CV 4961 mL |
| | Equilibration buffer | 20 mM Tris-HCl, pH 8.0 |
| | Elution buffer | 20 mM Tris-HCl, 300 mM NaCl, pH 8.0 |
| | Linear flow rate | 61 cm/hr (Flow rate 319 mL/min) |
| | Loading sample condition | pH 8.0 $\pm$ 0.1 Conductivity 2.0 $\pm$ 0.2 mS/cm |
| | Elution pooling criteria | Start point: $UV_{280nm}$ Value $\geq$ 100 mAU<br>End point: $UV_{280nm}$ Value $\leq$ 100 mAU |
| | Process | Equilibration buffer (2 CV)<br>Sample loading<br>Unbound wash (with EQ buffer) (5 CV)<br>Elution buffer (5 CV)<br>2 M NaCl (4 CV)<br>0.5 M NaOH (5 CV)<br>2 M NaCl (3 CV)<br>Equilibration buffer (3 CV) |
| Affinity (3cycle) | Column scale | BPG 200 (i.d 20 cm, h 10.5 cm), CV 3297 mL |
| | Equilibration buffer | 20 mM Tris-HCl, 150 mM NaCl, pH 8.0 |
| | Elution buffer | 50 mM Tris-HCl, 10 mM Glutathione (Reduced), pH 8.0 |
| | Acidic buffer | 100 mM Sodium Acetate, 500 mM NaCl, pH 4.5 |
| | Alkaline buffer | 100 mM Tris-HCl, 500 mM NaCl, pH 8.0 |
| | Linear flow rate | 61 cm/hr (Flow rate 319 mL/min) |
| | Loading sample condition | Direct Loading |
| | Elution pooling criteria | Start point: $UV_{280nm}$ Value $\geq$ 100 mAU<br>End point : $LN_{280nm}$ Value $\leq$ 100 mAU |

(continued)

| Items | | Process conditions |
|---|---|---|
| | Process | Equilibration buffer (2 CV)<br>Sample loading<br>Unbound wash (with EQ buffer) (5 CV)<br>Elution buffer (5 CV)<br>Acidic buffer (5 CV)<br>Alkaline buffer (5 CV)<br>6 M Guanidine hydrochloride (2 CV)<br>DW (2 CV)<br>Equilibration buffer (3 CV) |
| Thrombin Reaction & AEXII (2cycle) | Enzyme | Bovine Thrombin |
| | Enzyme ratio | 1mg GST-T$\beta$4 per 2 unit thrombin |
| | Reaction time | 16 + 2H |
| | Reaction temp | 22 + 2°C |
| | Agitation | 100 $\pm$ 10 ppm |
| | Column scale | BPG 200 (i.d 20cm, H 15.8 cm), CV 4961 mL |
| | Equilibration buffer | 20 mM Tris-HCl, pH 8.0 |
| | Elution buffer | 20 mM Tris-HCl, 80 mM NaCl, pH 8.0 |
| | Linear flow rate | 61 cm/hr (Flow rate 319 mL/min) |
| | Loading sample condition | pH 8.0 $\pm$ 0.1<br>Conductivity 2.0 $\pm$ 0.2 mS/cm |
| | Elution pooling criteria | Start point: $UV_{214nm}$ Value $\geq$ 200 mAU<br>End point: $UV_{214nm}$ Value $\leq$ 200 mAU |
| | Process | Equilibration buffer (2 CV)<br>Sample loading<br>Unbound wash (with EQ buffer) (5 CV)<br>Elution buffer (5 CV)<br>2 M NaCl (4 CV)<br>0.5 M NaOH (5 CV)<br>2 M NaCl (3 CV)<br>Equilibration buffer (3 CV) |

(continued)

| Items | | Process conditions |
|---|---|---|
| CEX (2cycle) | Column scale | BPG 140 (i.d 14 cm, H 9.0 cm), CV 1385 mL |
| | Equilibration buffer | 20 mM Sodium Acetate, pH 4.5 |
| | Wash buffer* | 20 mM Sodium Acetate, 60 mM NaCl, pH 4.5 |
| | Elution buffer | 20 mM Sodium Acetate, 180 mM NaCl, pH 4.5 |
| | Linear flow rate* | 150 cm/hr (Flow rate 385 mL/min) |
| | Loading sample condition | pH 4.5 $\pm$ 0.1<br>Conductivity 2.0 $\pm$ 0.2 mS/cm |
| | Elution pooling criteria | Start point: $UV_{214nm}$ Value $\geq$ 200 mAU<br>End point: $UV_{214nm}$ Value $\leq$ 200 mAU |
| | Process | Equilibration buffer (2 CV)<br>Sample loading<br>Wash buffer (10 CV)<br>Elution buffer (5 CV)<br>2 M NaCl (4 CV)<br>0.5 M NaOH (5 CV)<br>2 M NaCl (3 CV)<br>Equilibration buffer (3 CV) |

3rd 50L verification run process conditions

**(9-3)** Result of experiment

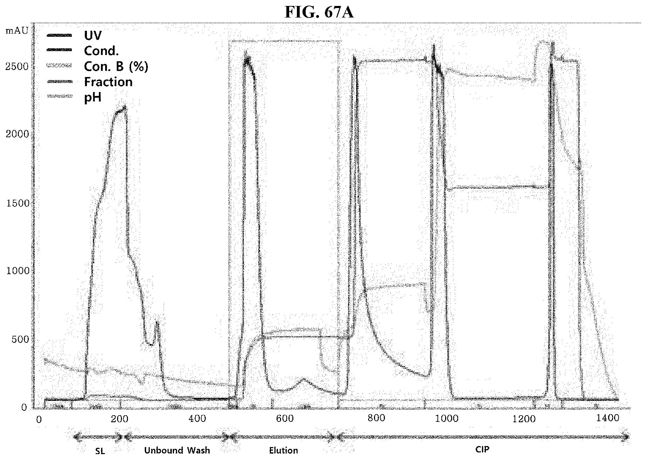

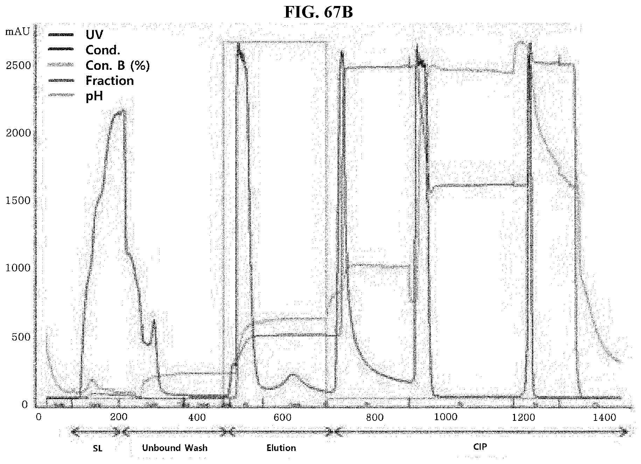

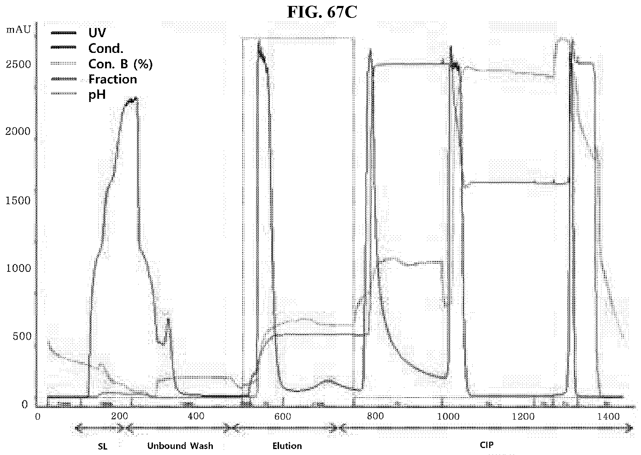

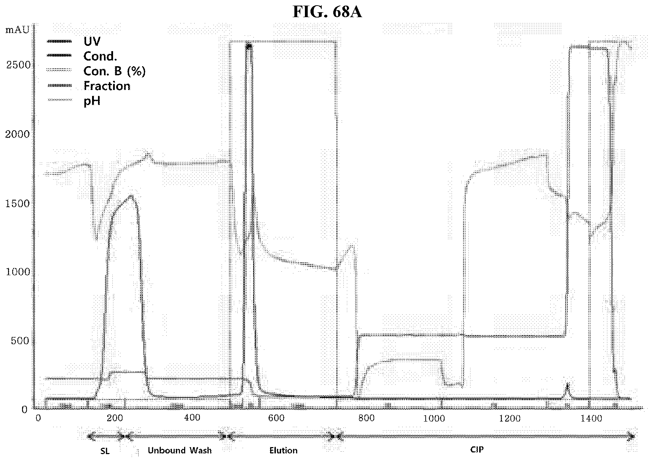

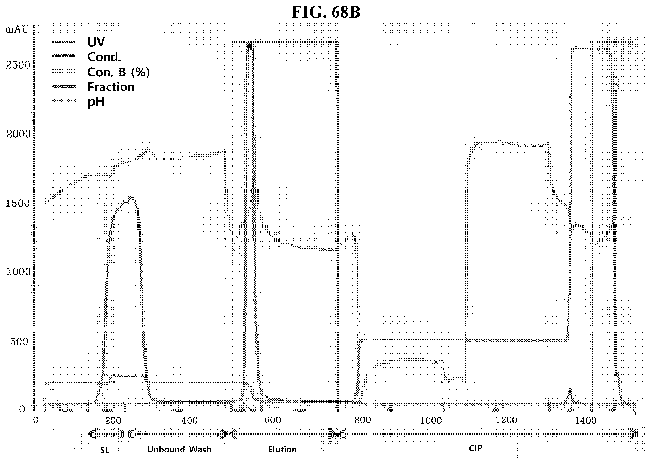

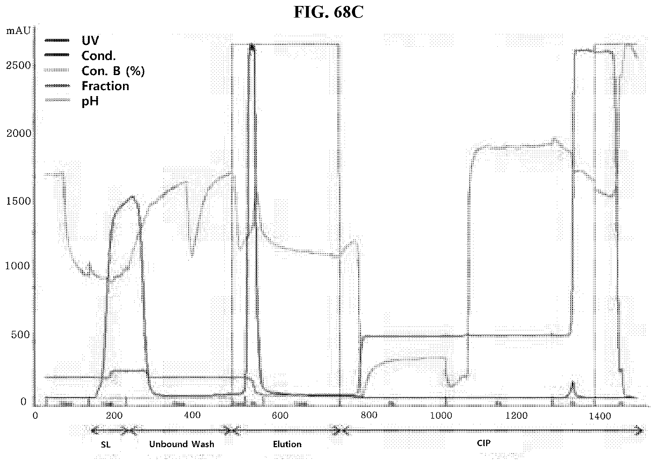

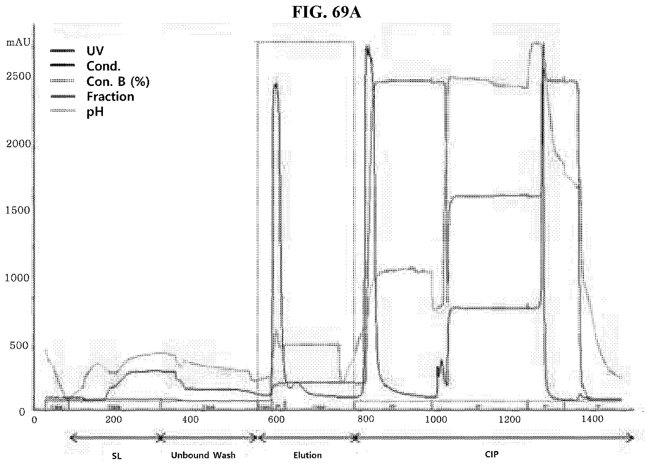

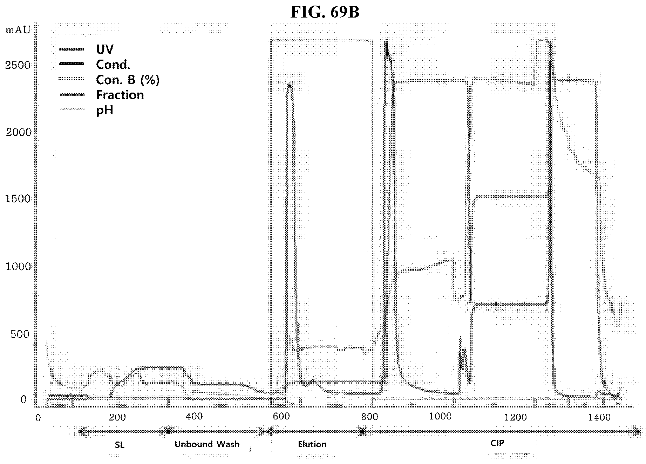

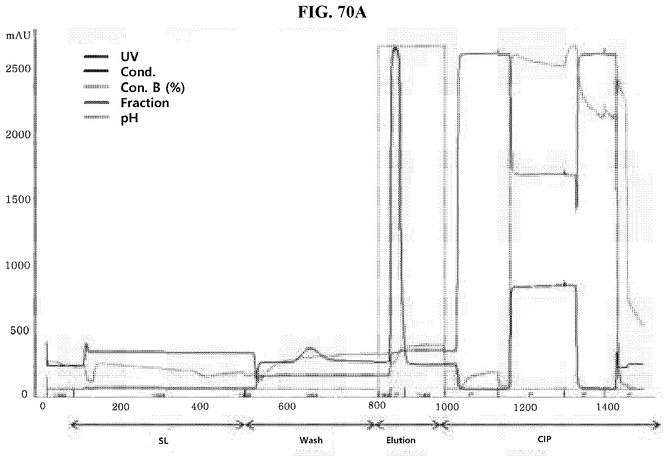

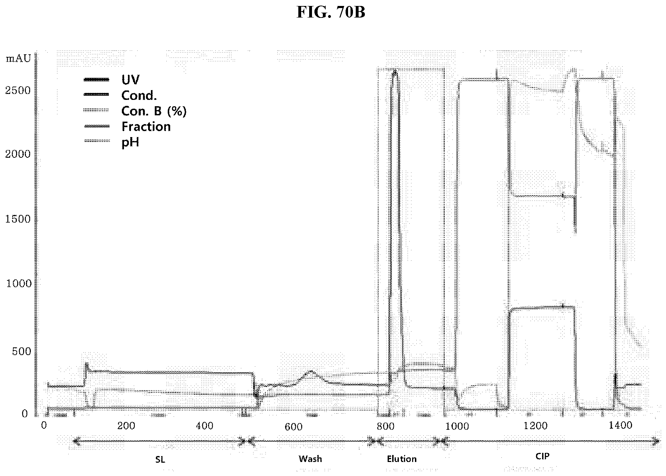

The chromatograms for the processes are shown in FIGS. 67 to 70, and it was confirmed that reproducible results are derived. The cumulative purification process yield was 12.4% (Table 108).

[Table 108]

| | Loading Sample | | | Eluted Pool | | | Yield (%) | Purity* (%) |
|---|---|---|---|---|---|---|---|---|
| | Conc. (mg/mL) | Volume (mL) | Amount (mg) | Conc. (mg/mL) | Volume (mL) | Amount (mg) | | |
| Cell breakage (170114.) | | 32,640 | | 5.62 | 24,805 | 139,404 | | |
| Depth Filtration&MF | 5.62 | 24,805 | 139,404 | 4.25 | 34,225 | 145,456 | 104.3 | |
| AEXI | 4.25 | 34,180 | 145,265 | 6.03 | 23,910 | 144,106 | 99.2 | |
| Glutathione S4FF | 6.03 | 17,930 | 108,064 | 10.47 | 8,370 | 87,634 | 81.1 | |
| Thrombin | 10.47 | 8,330 | 87,215 | 2.04 | 9,555 | 19,586 | 22.5 | |
| AEX II | 2.04 | 9,,545 | 19,565 | 1.97 | 7,215 | 14,206 | 72.6 | 96.3 |
| CEX | 1.97 | 7,175 | 14,128 | 4.61 | 2,901 | 13,371 | 94.6 | 98.1 |
| UF/DF | 4.61 | 2,891 | 13,323 | 10.23 | 1,299 | 13,283 | 99.7 | 98.1 |
| Total Yield (%) | | | | | | | 12.4 | |

*Purity was confirmed through RP-HPLC

Purity and Yield for 3rd 50L Verification run

The content of process-derived impurities for each process measured (Table 109).

[Table 109]

| | Eluted Pool | | | HCP | | | Endotoxin | |
|---|---|---|---|---|---|---|---|---|
| | Conc. (mg/mL) | Volume (mL) | Amount (mg) | Conc. (mg/mL) | HCP Conc. (ng/mL) | HCP (ppm) | Conc. (EU/mL) | Conc. (EU/mg) |
| AEX I | 6.03 | 23,910 | 144,106 | 6.03 | 11,554 | 1,916 | 173,276 | 28,735 |
| Affinity | 10.47 | 8,370 | 87,634 | 10.47 | 745.5 | 71.2 | 2,476 | 236 |
| AEX II | 1.97 | 7,215 | 14,206 | 1.97 | 13.3 | 6.7 | 0.065 | 0.033 |
| CEX I | 4.61 | 2,901 | 13,371 | 4.61 | 3.7 | 0.8 | <0.005 | 0.001 |
| UF/DF | 10.23 | 1,299 | 13,283 | 10.23 | 17.0 | 1.7 | 0.866 | 0.085 |

HCP and endotoxin analysis for 3rd 50L verification run

Approximately 13 g of the final purified extract was obtained, and the RP-HPLC purity was confirmed to be 98.1%, which meets the criterion (97% or more) (Table 108). HCP and endotoxin contents also met the standards (Table 109). It was confirmed that the unit process removal rates of the process-derived impurities were HCP 97.7% for HCP and 99.5% for endotoxin in the affinity process. After the affinity process, in the AEXII process, the endotoxin unit process removal rate was confirmed to be 99.9%. It can be seen that this result was equal to the result of the 5L scale culture broth purification, which is the previous experiment.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

[Industrial Applicability]

According to the present invention, high-purity Gly-Tβ4 can be stably prepared on a large scale, so that it is considered that the present invention has a great industrial value in that Gly-Tβ4 having an excellent industrial value can be obtained economically and with high efficiency.

**Claims**

1.   A method of preparing glycine-thymosin β4 (Gly-thymosin β4, Gly-Tβ4), comprising:

(S1) loading a sample in a primary anion exchange chromatography column equilibrated with an equilibration buffer, washing the column with a wash buffer, and eluting a fraction attached to the column with the elution buffer;
(S2) performing affinity chromatography on an eluate;
(S3) enzymatically cleaving the eluate by thrombin treatment;
(S4) loading the cleaved product in a secondary anion exchange chromatography column equilibrated with an equilibration buffer, washing the column with a washing buffer, and eluting a fraction attached to the column with an elution buffer at a dynamic binding capacity (DBC) of 3 mg/mL or less;
(S5) loading the eluate in a cation exchange chromatography column equilibrated with an equilibration buffer, washing the column with a wash buffer, and eluting a fraction attached to the column with an elution buffer at a DBC of 3 to 8 mg/mL; and
(S6) filtrating the eluate.

2.   The method of claim 1, wherein, in Step (S4), the cleaved product is loaded in the column at pH 8 to 9 and a conductivity of 4 mS/cm or less.

3.   The method of claim 1, wherein the fraction attached to the column in the anion exchange chromatography in Step (S4) is eluted with a buffer of pH 7 to 9, which contains 10 to 30 mM Tris-HCl and 30 to 130 mM NaCl, at a flow rate of 100 to 300 cm/hr.

4. The method of claim 1, wherein, in Step (S5), the eluted product is loaded in the column at pH 4.5 ± 0.1 and a conductivity of 2 ± 0.2 mS/cm or less.

5. The method of claim 1, wherein, in the cation exchange chromatography in Step (S5), the cleaved product is loaded in the column at pH 4 to 7 and a conductivity of 4 mS/cm or less.

6. The method of claim 1, wherein, in the cation exchange chromatography in Step (S5), the fraction attached to the column is eluted with a buffer of pH 4 to 5, which contains 10 to 30 mM acetate and 150 to 210 mM NaCl, at a flow rate of 100 to 300 cm/hr.

7. The method of claim 1, wherein, in step (S3), the ratio of GST-Tβ4 to thrombin is 5 to 0.5:1 (unit:mg).

8. The method of claim 1, wherein the reaction temperature in Step (S3) is 13 to 27 °C.

9. The method of claim 1, wherein the purity of the final purified extract according to the method is 97% or more.

10. The method of claim 1, wherein the sample in step (S1) is obtained by the following steps:

culturing host cells expressing a target protein;
lysing the host cells; and
washing and filtering the lysate.

11. The method of claim 10, wherein main culture of the host cells is fed-batch culture at an $OD_{600}$ of 40 to 65 and an induction temperature of 25 to 35 °C in the initiation of induction.

12. The method of claim 10, wherein cell productivity according to the method is 180 g pellet/L or more, and the productivity of the target protein is 54 mg/g pellet or more.

**FIG. 1**

| Time (hour) | 1st, 2nd | | 3rd – 1, 2 | | 4th | |
|---|---|---|---|---|---|---|
| | Feed-A (g/min) | Feed-B (mL/min) | Feed-A (g/min) | Feed-B (mL/min) | Feed-A (g/min) | Feed-B (mL/min) |
| 0 | Batch culture | | Batch culture | | Batch culture | |
| 1 | | | | | | |
| 2 | | | | | | |
| 3 | | | | | | |
| 4 | | | | | | |
| 5 | pH-stat | | | | 0.5 | |
| 6 | | | | | | |
| 7 | | | 0.8 | | | |
| 8 | | | | | Stop | |
| 9 | | | 0.9 | | | |
| 10 | | | | | | |
| 11 | 0.8 | | Stop | | 0.5 | |
| 12 | | | | | | |
| 13 | | 0.5 | | 0.3 | 0.8 | |
| 14 | | | 0.9 | | | 0.3 |
| 15 | 0.9 | | | | | |
| 16 | | | | | 0.9 | |
| 17 | | | | | | |
| 18 | 1.0 | | 0.8 | 1.0 | | 1.0 |
| 19 | | | | | | |
| 20 | | End | | | | |
| 21 | End | | | End | End | |
| 22 | | | | | | |

**FIG. 2**

| Time (hour) | NR process* | | 50 L verification run | |
|---|---|---|---|---|
| | Feed-A ( g/min) | Feed-B (mL/min) | Feed-A ( g/min) | Feed-B (mL/min) |
| 0 | | | | |
| 1 | Batch culture | | Batch culture | |
| 2 | | | | |
| 3 | | | | |
| 4 | | | | |
| 5 | 3.3 | | 1.7 | |
| 6 | 4.5→5 | | 2.3 | |
| 7 | 6.25 | | 3.0 | |
| 8 | 11.5 | | 3.3 | |
| 9 | | 1.7 | 4.3 | |
| 10 | | | 5.5 | |
| 11 | 6.25 | | 5.5 | |
| 12 | | | 6.7 | |
| 13 | | | 8.2 | 3 |
| 14 | | | 8.9 | |
| 15 | 5.42 | 4 | 10.7 | |
| 16 | | | 12.8 | |
| 17 | | | 15.4 | |
| 18 | | | 15.0 | |
| 19 | | | 15.0 | 10 |
| 20 | | | 15.7 | |
| 21 | | | 16.2 | |

*Reference 6.1

**FIG. 3**

Cell growth

Glucose & Acetate

<Appended reference> Batch record: 5 L verification run 1st ~ 4th

**FIG. 4**

SDS PAGE - Soluble protein

| Lane | Sample name |
|------|-------------|
| M | Marker |
| 1 | 4th (160512-B2) |
| 2 | 3rd-2 (160428-B2) |
| 3 | 3rd-1 (160428-B1) |
| 4 | 2nd (160407-B2) |
| 5 | 1st (160407-B1) |

Sample cell density : $OD_{600}$=5

Lysis buffer volume : 1,000 μL

Loading volume     : 20 μL

**FIG. 5**

<Appended reference 6~7> Batch record: 50 L verification run 1st, 2nd

**FIG. 6**

| Lane | Sample name |
|------|-------------|
| M | Marker |
| 1 | 50 L 1st (160629) |
| 2 | 50 L 2nd (160729) |

Sample cell density : $OD_{600}=5$

Lysis buffer volume : 1,000 μL

Loading volume    : 20 μL

FIG. 7

Flask 1 (w/ YE)

Flask 2 (w/o YE)

<Appended reference 8> Batch record: Flask culture test

**FIG. 8**

<Appended reference> Batch record: Fed-batch growth test

FIG. 9A

DoE 12 (160928-B1)

FIG. 9B

DoE 7 (161005-B1)

**FIG. 9C**

DoE 8 (161005-B2)

**FIG. 9D**

DoE 5 (161027-B1)

**FIG. 9E**

**FIG. 9F**

**FIG. 9G**

**FIG. 9H**

**FIG. 9I**

**FIG. 9J**

**FIG. 9K**

**FIG. 9L**

**FIG. 9M**

## FIG. 10

| Lane | Sample Name | Densitometric volume | Protein amount (μg) | GST-TB4 productivity per pellet* (mg/g pellet) | GST-TB4 productivity per culture volume (g/L) |
|---|---|---|---|---|---|
| 1 | DoE 12 (160928-B1) | 3.591678110 | 2.50 | 60.6 | 13.1 |
| 2 | DoE 7 (161005-B1) | 3.636301064 | 2.53 | 61.4 | 12.8 |
| 3 | DoE 8 (161005-B2) | 3.543084618 | 2.46 | 59.7 | 9.8 |
| 4 | DoE 5 (161027-B1) | 3.923724612 | 2.74 | 66.5 | 9.4 |
| 5 | DoE 10 (161011-B2) | 3.971714143 | 2.78 | 67.4 | 13.6 |
| 6 | DoE 2 (161101-B1) | 2.103865811 | 1.40 | 33.9 | 6.0 |
| 7 | DoE 13 (161018-B2) | 3.667646480 | 2.55 | 61.9 | 10.7 |
| 8 | **BSA (0.5) | 0.795285637 | 0.50 | N/A | N/A |
| 9 | BSA (1) | 1.578428809 | 1.00 | | |
| 10 | BSA (2) | 3.074617555 | 2.00 | | |
| 11 | BSA (4) | 5.553335527 | 4.00 | | |

\* $OD_{600}$ = 1 is converted into 1.1 g/L of cell pellet

\*\* Standard protein (BSA; bovine serum albumin)

## FIG. 11

| Lane | Sample Name | Densitometric volume | Protein amount (μg) | GST-TB4 roductivity* (mg/g pellet) | GST-TB4 productivity per culture volume (g/L) |
|---|---|---|---|---|---|
| 1 | DoE 11 (161018-B1) | 4.025547356 | 2.15 | **52.1** | **9.4** |
| 2 | DoE 3 (161103-B1) | 4.880852371 | 2.62 | **63.5** | **11.0** |
| 3 | DoE 9 (161011-B1) | 4.388502221 | 2.35 | **56.9** | **11.6** |
| 4 | DoE 1 (161025-B1) | 4.650105123 | 2.49 | **60.4** | **11.4** |
| 5 | DoE 6 (161103-B2) | 3.176320343 | 1.68 | **40.8** | **7.1** |
| 6 | DoE 4 (161101-B2) | 3.730516673 | 1.99 | **48.2** | **9.2** |
| 7 | **BSA (0.5) | 1.029281668 | 0.50 | | |
| 8 | BSA (1) | 1.857820684 | 1.00 | | |
| 9 | BSA (2) | 3.871906021 | 2.00 | N/A | N/A |
| 10 | BSA (4) | 7.354061890 | 4.00 | | |

* $OD_{600}$ = 1 is converted into 1.1 g/L of cell pellet

** Standard protein (BSA; bovine serum albumin)

**FIG. 12**

Residual diagram for GTS-TB4 productivity

Residual diagram for cell productivity

FIG. 13

**FIG. 14**

Contour plot GST-TB4 productivity, and cell productivity

**FIG. 15**

Cell growth — Cell density (OD600) vs Time (h)
- 161122-B1 (Springer YE)
- 161122-B2 (BD YE)
- 161115-B1 (BD YE)
- 161124-B1 (BD YE/DC Antiform/Air 0.5)
- 160629 (50 L 1st)
- 160729 (50 L 2nd)

Glucose & Acetate — Glucose & Acetate (g/L) vs Time (h)
- Glucose (161122-B1)
- Glucose (161122-B2)
- Glucose (161115-B1)
- Glucose (161124-B1)
- Acetate (161124-B1)
- Acetate (161122-B1)
- Acetate (161122-B2)
- Acetate (161115-B1)

<Appended reference 23~26> Batch record:
Confirmation of optimal condition reproducibility

**FIG. 16**

SDS PAGE - Soluble protein

Standard curve

y = 1.939x + 0.936
R² = 0.991

- Cell pellet sample $OD_{600}$ = 5
- Lysis buffer volume : 1,000 µL
- Loading volume : 15 µL
- Sample dilution 2 x (Lane 1 ~ 4)

| Lane | Sample Name | Densitometric volume | Protein amount (µg) | GST-TB4 productivity (mg/g pellet) | GST-TB4 productivity per culture volume (g/L) |
|---|---|---|---|---|---|
| 1 | 161115-B1 (Using BD YE) | 6.681783342 | 2.96 | 71.8 | 12.1 |
| 2 | 161122-B2 (Using BD YE) | 5.670222535 | 2.44 | 59.2 | 11.0 |
| 3 | 161122-B1 (Springer YE) | 6.238801451 | 2.73 | 66.3 | 11.7 |
| 4 | 161124-B1 (BD YE/DC Antiform/Air 0.5) | 6.412727897 | 2.82 | 68.5 | 12.3 |
| 5 | 160629 (50L 1st) | 4.962458949 | 2.08 | 25.2 | 2.3 |
| 6 | **Standard protein 0.5 µg | 1.715767325 | 0.50 | N/A | N/A |
| 7 | Standard protein 1.0 µg | 3.249055045 | 1.00 | | |
| 8 | Standard protein 2.0 µg | 4.587875254 | 2.00 | | |
| 9 | Standard protein 4.0 µg | 8.737791780 | 4.00 | | |

* $OD_{600}$ = 1 is converted into 1.1 g/L of cell pellet

** Standard protein (BSA; bovine serum albumin)

## FIG. 17

| Lane | Sample Name | Densitometric volume | 단백질량 (μg) | *GST-TB4 생산성 (mg/g pellet) | 배양부피당 GST-TB4 생산성 (g/L) |
|---|---|---|---|---|---|
| 1 | 161122-B1 (Biospringer YE) | 6.423098352 | 2.75 | 66.7 | 11.8 |
| 2 | 161115-B1 (BD YE 사용) | 5.667312004 | 2.30 | 55.8 | 10.3 |
| 3 | 161122-B2 (BD YE 사용) | 5.843964641 | 2.41 | 58.3 | 9.8 |
| 4 | 161124-B1 (BD YE/DC 안티폼/Air 0.5) | 6.293620618 | 2.67 | 64.8 | 11.6 |
| 5 | 160629 (50L 2 차) | 4.555698711 | 1.64 | 19.9 | 2.1 |
| 6 | **Standard protein 0.5 μg | 2.419153548 | 0.50 | N/A | N/A |
| 7 | Standard protein 1.0 μg | 3.495831866 | 1.00 | | |
| 8 | Standard protein 2.0 μg | 5.490469861 | 2.00 | | |
| 9 | Standard protein 4.0 μg | 8.386014374 | 4.00 | | |

\* $OD_{600}$ =1 을 cell pellet 1.1 g/L로 환산함.

\*\* Standard protein (BSA; bovine serum albumin)

**FIG. 18**

<Appended reference 27, 28> Batch record: Optimal condition 50 L scale-up

FIG. 19

**FIG. 20**

Lane 1 : Protein molecular weight standard marker
Lane 2 : 20mM Tris, pH 8.0
Lane 3 : 20mM Tris, pH 8.0 w/ 3mM DTT
Lane 4 : 20mM Tris, pH 8.0 w/ 10mM EDTA
Lane 5 : 20mM Tris, pH 8.0 w/ 20mM DETA
Lane 6 : 20mM Tris, pH 8.0 w/ 0.1% TX-100
Lane 7 : 20mM Tris, pH 8.0 w/ 0.5% TX-100
Lane 8 : 20mM Tris, pH 8.0 w/ 100mM Gdn-HCl
Lane 9 : 20mM Tris, pH 8.0 w/ 200mM Gdn-HCl

**FIG. 21**

Lane 1 : Protein molecular weight standard marker_5uL
Lane 2 : Ammonium sulfate 0%_2uL
Lane 3 : Ammonium sulfate 16.5%_2uL
Lane 4 : Ammonium sulfate 33%_2uL
Lane 5 : Ammonium sulfate 66%_2uL
Lane6 : BSA 1ug
Lane7 : BSA 2ug
Lane8 : BSA 4ug
Lane9 : BSA 6ug

**FIG. 22**

**FIG. 23**

**FIG. 24**

**FIG. 25A**

Lane M. Molecular weight size marker
Lane 1. GST-TB4 (3ug)_50L Verification Affinity Eluted pool
Lane 2. Cell lysate 0day (1uL)
Lane 3. Cell lysate 1day (1uL)
Lane 4. AEXI (61 cm/hr) Flow through (5uL)
Lane 5. AEXI (61 cm/hr) Eluted pool (3uL)
Lane 6. BSA 1ug
Lane 7. BSA 2ug
Lane 8. BSA 4ug
Lane 9. BSA 6ug

**FIG. 25B**

Lane M. Molecular weight size marker
Lane 1. GST-TB4 (3ug)_50L Verification Affinity Eluted pool
Lane 2. Cell lysate 0day (1uL)
Lane 3. Cell lysate 1day (1uL)
Lane 4. AEXI (200 cm/hr) Flow through (5uL)
Lane 5. AEXI (200 cm/hr) Eluted pool (3uL)
Lane 6. BSA 1ug
Lane 7. BSA 2ug
Lane 8. BSA 4ug
Lane 9. BSA 6ug

## FIG. 26

**FIG. 27**

**FIG. 28A**

Lane M. Molecular weight size marker
Lane 1. GST-TB4 (3ug)_50L Verification Affinity Eluted pool
Lane 2. Cell lysate 0day (1uL)
Lane 3. Cell lysate 1day (1uL)
Lane 4. AEXI (500mM NaCl) Flow through (5uL)
Lane 5. AEXI (500mM NaCl) Eluted pool (3uL)
Lane 6. BSA 1ug
Lane 7. BSA 2ug
Lane 8. BSA 4ug
Lane 9. BSA 6ug

**FIG. 28B**

Lane M. Molecular weight size marker
Lane 1. GST-TB4 (3ug)_50L Verification Affinity Eluted pool
Lane 2. Cell lysate (1uL)
Lane 3. AEXI (200mM NaCl) Flow through (5uL)
Lane 4. AEXI (200mM NaCl) Eluted pool (3uL)
Lane 5. AEXI Strip (3uL)
Lane 6. GST-TB4 (0.3ug)
Lane 7. GST-TB4 (0.6ug)
Lane 8. GST-TB4 (0.9ug)
Lane 9. GST-TB4 (1.2ug)

**FIG. 29**

**FIG. 30**

FIG. 31

**FIG. 32A**

Lane M. Molecular weight size marker
Lane 1. GST-TB4 (3ug)_50L Verification Affinity Eluted pool
Lane 2. Cell lysate (1uL)
Lane 3. AEXI (80mM NaCl) Flow through (5uL)
Lane 4. AEXI (80mM) Eluted pool (3uL)
Lane 5. AEXI Strip (3uL)
Lane 6. GST-TB4 (0.3ug)
Lane 7. GST-TB4 (0.6ug)
Lane 8. GST-TB4 (0.9ug)
Lane 9. GST-TB4 (1.2ug)

**FIG. 32B**

Lane M. Molecular weight size marker
Lane 1. GST-TB4 (3ug)_50L Verification Affinity Eluted pool
Lane 2. Cell lysate (1uL)
Lane 3. AEXI (50mM NaCl) Flow through (5uL)
Lane 4. AEXI (50mM NaCl) Eluted pool (3uL)_ peak 1
Lane 5. AEXI (50mM NaCl) Eluted pool (3uL)_ peak 2
Lane 6. AEXI Strip (3uL)
Lane 7. GST-TB4 (0.3ug)
Lane 8. GST-TB4 (0.6ug)
Lane 9. GST-TB4 (0.9ug)

**FIG. 33A**

Lane M. Molecular weight size marker
Lane 1. GST-TB4_50L Verification Affinity Eluted pool
Lane 2. Cell lysate (1uL)
Lane 3. AEXI Flow through (5uL)
Lane 4. AEXI Eluted pool (3uL)

**FIG. 33B**

Lane M. Molecular weight size marker
Lane 1. GST-TB4_50L Verification Affinity Eluted pool
Lane 2. Cell lysate (1uL)
Lane 3. AEXI Flow through (5uL)
Lane 4. AEXI Eluted pool (3uL)

**FIG. 33C**

Lane M. Molecular weight size marker
Lane 1. GST-TB4_SOL Verification Affinity Eluted pool
Lane 2. Cell lysate (1uL)
Lane 3. AEXI Flow through (5uL)
Lane 4. AEXI Eluted pool (3uL)

**FIG. 34A**

M : Protein molecular weight standard marker
Lane 1 : GST-TB4 _ 0.6mg/ml 5uL
Lane 2 : AEXI Eluted pool 0day (4°C)_5uL
Lane 3 : AEXI Eluted pool 1day(4°C)_5uL
Lane 4 : AEXI Eluted pool 2day(4°C)_5uL
Lane 5 : AEXI Eluted pool 3day (4°C)_5uL
Lane 6 : AEXI Eluted pool 4day (4°C)_5uL
Lane 7 : AEXI Eluted pool 5day (4°C)_5uL
Lane 8 : AEXI Eluted pool 6day (4°C)_5uL
Lane 9 : AEXI Eluted pool 7day (4°C)_5uL

**FIG. 34B**

M : Protein molecular weight standard marker
Lane 1 : GST-TB4 _ 0.6mg/ml 5uL
Lane 2 : AEXI Eluted pool 0day (20℃)_5uL
Lane 3 : AEXI Eluted pool 1day(20℃)_5uL
Lane 4 : AEXI Eluted pool 2day(20℃)_5uL
Lane 5 : AEXI Eluted pool 3day (20℃)_5uL
Lane 6 : AEXI Eluted pool 4day (20℃)_5uL
Lane 7 : AEXI Eluted pool 5day (20℃)_5uL
Lane 8 : AEXI Eluted pool 6day (20℃)_5uL
Lane 9 : AEXI Eluted pool 7day (20℃)_5uL

**FIG. 35**

**FIG. 36**

**FIG. 37A**

## FIG. 37B

FIG. 38A

FIG. 38B

**FIG. 39A**

M : Protein molecular weight standard marker
Lane 1 : GST-TB4 _ 0.6mg/ml 5uL
Lane 2 : GST affinity Eluted pool 0day (4°C)_5uL
Lane 3 : GST affinity Eluted pool 1day(4°C)_5uL
Lane 4 : GST affinity Eluted pool 2day(4°C)_5uL
Lane 5 : GST affinity Eluted pool 3day (4°C)_5uL
Lane 6 : GST affinity Eluted pool 4day (4°C)_5uL
Lane 7 : GST affinity Eluted pool 5day (4°C)_5uL
Lane 8 : GST affinity Eluted pool 6day (4°C)_5uL
Lane 9 : GST affinity Eluted pool 7day (4°C)_5uL

**FIG. 39B**

M : Protein molecular weight standard marker
Lane 1 : GST-TB4 _ 0.6mg/ml 5uL
Lane 2 : GST affinity Eluted pool 0day (20℃)_5uL
Lane 3 : GST affinity Eluted pool 1day(20℃)_5uL
Lane 4 : GST affinity Eluted pool 2day(20℃)_5uL
Lane 5 : GST affinity Eluted pool 3day (20℃)_5uL
Lane 6 : GST affinity Eluted pool 4day (20℃)_5uL
Lane 7 : GST affinity Eluted pool 5day (20℃)_5uL
Lane 8 : GST affinity Eluted pool 6day (20℃)_5uL
Lane 9 : GST affinity Eluted pool 7day (20℃)_5uL

FIG. 40

FIG. 41

**FIG. 42**

FIG. 43A

FIG. 43B

**FIG. 44**

**FIG. 45**

## FIG. 46

## FIG. 47

**FIG. 48**

**FIG. 49**

**FIG. 50**

<Binex DSP condition>  <Huons DSP condition>

```
                Cell Lysis & Recovery
                          |
                          v
                  Anion Exchange
          Chromatography (B/E)-AEXI
                          |
                          v
              Affinity Chromatography
                          |
                          v
                 Enzyme Digestion
                          |
            +-------------+-------------+
            v                           v
    Anion Exchange              Anion Exchange
Chromatography (F/T)-AEXII   Chromatography (B/E)-AEXII
            |                           |
            v                           v
 Mixed Mode Chromatography     Cation Exchange
         (MMC)              Chromatography (B/E) -CEX
            |                           |
            +-------------+-------------+
                          v
                       UF/DF
```

FIG. 51

**FIG. 52**

# FIG. 53

FIG. 54

**FIG. 55**

FIG. 56

**FIG. 57**

**FIG. 58**

**FIG. 59**

**FIG. 60**

# FIG. 61

**FIG. 62**

**FIG. 63**

**FIG. 64**

**FIG. 65**

**FIG. 66**

## FIG. 67A

## FIG. 67B

**FIG. 67C**

**FIG. 68A**

**FIG. 68B**

**FIG. 68C**

**FIG. 69A**

**FIG. 69B**

FIG. 70A

FIG. 70B

## INTERNATIONAL SEARCH REPORT

| International application No. |
|---|
| PCT/KR2019/009265 |

**A. CLASSIFICATION OF SUBJECT MATTER**

*C07K 14/575(2006.01)i, C07K 1/12(2006.01)i, C07K 1/18(2006.01)i, C07K 1/22(2006.01)i, C07K 1/34(2006.01)i, C07K 1/36(2006.01)i, C12P 21/00(2006.01)i*

According to International Patent Classification (IPC) or to both national classification and IPC

**B. FIELDS SEARCHED**

Minimum documentation searched (classification system followed by classification symbols)

C07K 14/575; A61K 31/728; A61K 38/07; A61K 38/08; A61K 38/18; A61K 38/22; A61K 8/64; C07K 19/00; C07K 7/00; C07K 1/12; C07K 1/18; C07K 1/22; C07K 1/34; C07K 1/36; C12P 21/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)

eKOMPASS (KIPO internal) & Keywords: thymosinβ4, equilibration buffer, anion Exchange Chromatography, affinity chromatography, thrombin, elution

**C. DOCUMENTS CONSIDERED TO BE RELEVANT**

| Category* | Citation of document, with indication, where appropriate, of the relevant passages | Relevant to claim No. |
|---|---|---|
| Y | KR 10-0984635 B1 (BIOTOXTECH CO., LTD. et al.) 01 October 2010 See abstract; claim 1; paragraphs [0008], [0015], [0081]-[0092]. | 1-12 |
| Y | KR 10-2016-0108260 A (UNION KOREA PHARM CO., LTD.) 19 September 2016 See abstract; paragraphs [0011], [0061], [0066]-[0088], [0158]. | 1-12 |
| Y | KR 10-2008-0002845 A (VIROMED LIMITED) 04 January 2008 See claims 1, 11-13, 62-74; paragraphs [79]-[88], [108]-[117], [126]-[129]. | 1-12 |
| A | KR 10-2014-0120018 A (IL YANG PHARM. CO., LTD.) 13 October 2014 See the entire document. | 1-12 |
| A | KR 10-2012-0115535 A (ORF LIFTAEKNI HF.) 18 October 2012 See the entire document. | 1-12 |

☐ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 NOVEMBER 2019 (21.11.2019) | **21 NOVEMBER 2019 (21.11.2019)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2019/009265**

| Patent document cited in search report | Publication date | Patent family member | Publication date |
|---|---|---|---|
| KR 10-0984635 B1 | 01/10/2010 | CN 101218249 A | 09/07/2008 |
| | | CN 1896096 A | 17/01/2007 |
| | | CN 1935838 A | 28/03/2007 |
| | | EP 1908779 A1 | 09/04/2008 |
| | | EP 1908779 B1 | 09/01/2013 |
| | | JP 2009-500045 A | 08/01/2009 |
| | | JP 5180074 B2 | 10/04/2013 |
| | | US 2009-0298758 A1 | 03/12/2009 |
| | | US 7816321 B2 | 19/10/2010 |
| | | WO 2007-009359 A1 | 25/01/2007 |
| KR 10-2016-0108260 A | 19/09/2016 | KR 10-1666934 B1 | 17/10/2016 |
| KR 10-2008-0002845 A | 04/01/2008 | AT 466868 T | 15/05/2010 |
| | | CN 101287748 A | 15/10/2008 |
| | | CN 101287748 B | 27/03/2013 |
| | | EP 1871785 A2 | 02/01/2008 |
| | | EP 1871785 B1 | 05/05/2010 |
| | | HK 1125387 A1 | 15/11/2013 |
| | | JP 2008-539696 A | 20/11/2008 |
| | | JP 4897792 B2 | 14/03/2012 |
| | | US 2006-0276625 A1 | 07/12/2006 |
| | | US 2010-0285526 A1 | 11/11/2010 |
| | | US 7585943 B2 | 08/09/2009 |
| | | US 8106158 B2 | 31/01/2012 |
| | | WO 2006-126102 A2 | 30/11/2006 |
| KR 10-2014-0120018 A | 13/10/2014 | None | |
| KR 10-2012-0115535 A | 18/10/2012 | AU 2011-204391 A1 | 16/08/2012 |
| | | BR 112012016575 A2 | 12/04/2016 |
| | | CA 2785631 A1 | 14/07/2011 |
| | | CN 102811704 A | 05/12/2012 |
| | | EP 2521532 A2 | 14/11/2012 |
| | | JP 2013-516460 A | 13/05/2013 |
| | | RU 2012133444 A | 20/02/2014 |
| | | SG 181969 A1 | 30/08/2012 |
| | | US 2013-0266536 A1 | 10/10/2013 |
| | | WO 2011-083500 A2 | 14/07/2011 |
| | | WO 2011-083500 A3 | 08/12/2011 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4767704 A
- US 4657866 A
- US 4927762 A
- US 4560655 A
- US 5122469 A
- US 9003430 B
- US 8700195 B
- US 30985 A

### Non-patent literature cited in the description

- Chemical Characterization of Thymosin beta. *The Journal of Biological Chemistry,* 1982, vol. 257 (2), 1000-1006
- Buffers. A Guide for the Preparation and Use of Buffers in Biological System. Calbiochem Corporation, 1975
- **HAM et al.** *Meth. Enz.,* 1979, vol. 58, 44
- **BARNS et al.** *Anal. Biochem.,* 1980, vol. 102, 255